# EUROPEAN PATENT APPLICATION

(11) **EP 4 094 726 A1**
(43) Date of publication of application: **30.11.2022**
(21) Application number: 20909876.3
(22) Date of filing: 15.12.2020
(51) Int. Cl.: A61F 2/30, A61F 2/34, A61F 2/36, A61F 2/40, A61F 2/44, A61F 2/28, B23K 11/00

(54) **POROUS COMPOSITE CONNECTION STRUCTURE CAPABLE OF SENSING DETECTION AND MEDICINE PREPARATION, METHOD, AND PROSTHESIS**

(30) Priority: 30.12.2019 CN 201911394644; 29.10.2020 CN 202011184579
(71) Applicant: JY MEDICAL DEVICES (SHANGHAI) CO. LTD., Zone Shanghai 200131 (CN); YBNX MEDICAL TECHNOLOGIES (SUZHOU) CO. LTD., Jiangsu 215558 (CN)
(72) Inventor: YAO, Jianqing, Shanghai 200131 (CN); SHI, Jinhu, Shanghai 200131 (CN)
(74) Representative: Yang, Shu
(86) International application number: PCT/CN2020/136523
(87) International publication number: WO 2021/135925

(57) **Abstract**

The present invention provides a porous composite the connected structure, method and prosthetic implant for sensor detection and drug delivery, where the porous structure is pre-connected or integrally formed with an intermediate to obtain a composite, which is connected to the substrate so that the porous structure covers the surface of the substrate. The present invention realizes the effective connection of a porous structure and a substrate, satisfying the connection requirements when the overall properties of the mechanical structure and its surface properties differ, and avoids the problem that the mechanical properties of the substrate could be greatly reduced by processes such as hot pressing. The connected structure of the present invention is provided with at least one holding space in which sensors can be placed to detect the status of the interior and exterior of the connected structure as needed, and in which drugs can also be placed and released according to the need for prevention or treatment, broadening the application scopes of the connected structure.

## Description

### Technology Field

The present invention relates to connection technology for composite materials, and in particular to applications in medical devices, providing a connected structure for porous structures and substrates and a method for making the same, which can be provided with sensors or drugs as desired; the invention also provides methods and prostheses for using said connected structure.

### Background

Engineering applications often have different requirements for the overall performance and surface properties of mechanical structures. For example, the overall performance (e.g., fatigue strength) of the acetabular cup and femoral stem of an artificial hip joint must meet the fatigue resistance requirements of the prosthetic implant under the dynamic loads that it will endure during an average of one to two million walking cycles per year for decades after implantation, and there are specific performance needs for the surface of the prosthetic implant to meet so that the surface of the prosthetic implant may be firmly bonded to the patient's bone structure to ensure that the prosthetic implant does not fail due to loosening; otherwise, the patient will have pain and the prosthetic implant must be removed, which means that the patient needs to undergo a revision surgery to implant a new prosthetic implant. Similar situations and needs exist for other orthopedic implants (e.g., spine). In fact, in other fields, there are situations where the substrate and the surface have different performance needs and a reliable and effective connection between the two is required.

Commonly used materials for joint prostheses are titanium alloy/ cobalt-chromium alloy/ stainless steel, etc., which do not form an effective biological or chemical bond with bone. The interface between the prosthetic implant and the bone is primarily through physical/mechanical interlocking. For example, a highly polished prosthetic surface and bone tissue cannot form an effective bond. Therefore, existing technologies can create a rough surface on the prosthetic surface by sandblasting, plasma spraying titanium, etc. to increase the interfacial friction between the prosthetic implant and bone tissue and to help enhance the physical/mechanical fixation of the prosthetic implant-bone interface. In the field of orthopedics, this is referred to as a "bone ongrowth" surface. In some cases, materials such as hydroxyapatite (or add-ons that can incorporate growth factors, stem cells, etc.) can be used as a coating over the rough surface of the prosthetic implant to increase osteoconductivity, osteoinduction, and bone regeneration to accelerate or enhance the connection of bone tissue to the surface of the prosthetic implant, further enhancing the "bone ongrowth" performance.

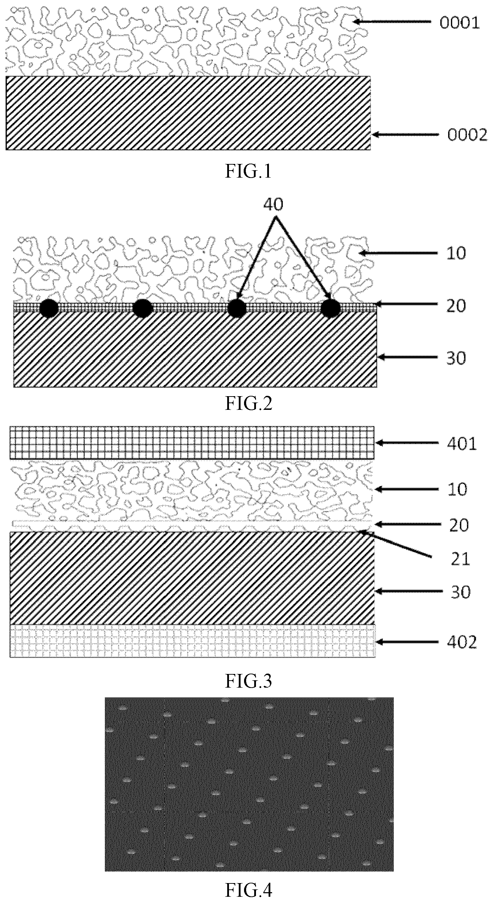

In addition, sometimes titanium wires or titanium beads, etc. can be used to form a porous coating on the surface of the prosthetic implant (e.g., acetabular cup/femoral stem) using methods such as sintering or diffusion bonding. Alternatively, a thin sheet 0001 with a porous structure is prefabricated using metal 3D printing additive manufacturing processes, vapor phase deposition processes, etc., and then the sheet 0001 is bonded to the solid substrate 0002 of the prosthetic implant through diffusion bonding, as shown in Figure 1. These methods provide a porous surface for the prosthetic implant, and the bone tissue in contact with the prosthetic implant is able to regenerate, filling the interconnected pores with new bone tissue and achieving the effect of "bone ingrowth" to the prosthetic implant. However, these processes have the inevitable consequence that the mechanical strength of the substrate is significantly reduced, thereby increasing the risk of fracture, especially when the prosthetic implant (e.g., femoral stem) is subjected to bending torque or tensile stress. Therefore, it is a design/process challenge to reliably and securely bond a porous structure to its substrate while ensuring that the mechanical properties of the substrate are not too significantly affected.

Relatively speaking, the laser welding process has a lower influence on the mechanical properties of the substrate. However, when the porosity of porous structures is high (>50%), the interconnected scaffolds are few in numbers and weak in strength; a large number of pores are formed between the scaffolds. Such a highly porous structure, whether achieved by 3D printing additive manufacturing process or other processes such as sintering, when the direct laser welding is used to connect the porous structure and the substrate, and the effective diameter of the laser beam is close to or even greater than the width of the scaffold, the laser energy may directly break the scaffolds, hence destroy the porous structure and can not achieve effective welding of the porous structure and the substrate. Alternatively, when diffusion bonding is used to connect the porous structure to the substrate, the strength of the substrate structure is significantly reduced due to the high temperature and elevated pressure of the process.

On the other hand, periprosthetic infection is a catastrophic complication of joint arthroplasty and a major cause of revision after joint arthroplasty. It is very difficult to diagnose and treat, often requiring multiple surgeries and long courses of anti-infective treatment, with high complications, long hospital stays, and expensive treatment. The occurrence of periprosthetic infection is the result of the interaction between pathogenic bacteria, the prosthetic implant and the human body. Based on the duration of symptoms, infection can be divided into: acute infection (less than 3 months), delayed infection (3 to 12 months), and chronic infection (more than 12 months). Acute and delayed infections are mostly caused by intraoperative contamination or postoperative wound complications resulting in pathogenic bacteria invasion, while chronic infections are mostly caused by the entry of pathogenic bacteria from distant initial foci of infection into the bloodstream causing bacteremia and dissemination to the affected joints via the blood circulation, making their prevention and treatment particularly difficult.

### SUMMARY

The present invention provides porous composite connected structures, methods and prostheses with sensor detection and drug placement. The said porous composite connected structure is an effective connected structure of a porous structure and a substrate, which can largely maintain the mechanical properties of the substrate and meet the overall requirements and the surface properties at the same time, and is suitable for prosthetic implants. The connected structure can be provided with sensors or drugs as needed. The present invention also provides prosthetic implant and method for applying the said connected structure.

A technical solution of the present invention is to provide a connected structure of a porous structure and a substrate; said connected structure, comprising: a composite, comprising a first porous structure pre-connected or integrally formed with an intermediate, said intermediate having a higher solid volume fraction than the first porous structure; a substrate, which is connected to the first porous structure and/or the intermediate of the composite; said connected structure is provided with at least one holding space for holding drugs and/or sensors.

Preferably, said intermediate comprises an insertion portion and/or an interlayer; said insertion portion having at least a portion of structure disposed within the first porous structure; said interlayer having at least a portion of structure disposed between the first porous structure and the substrate.

Preferably, said intermediate is a solid structure, or a second porous structure; said second porous structure having a higher solid volume fraction than said first porous structure.

Preferably, said substrate is a solid structure, or a third porous structure; said third porous structure having a higher solid volume fraction than said first porous structure.

Preferably, the solid volume fraction of said second porous structure is between that of said first porous structure and that of said third porous structure.

Preferably, said substrate is made by forging or casting or machining or powder metallurgy or metal powder injection molding.

Preferably, said first porous structure of the composite, together with the intermediate, is integrally formed by a 3D printing additive manufacturing process, or a vapor phase precipitation process, or a sintering process.

Preferably, said substrate is metallic; said first porous structure is metallic; and said intermediate is metallic.

Preferably, the channel or opening of the holding space, is directly or indirectly connected to an open space outside said connected structure; or, to other holding spaces of the connected structure.

Preferably, the channel or opening of the holding spaceis connected to the external open space through channels or openings of single or multiple components comprising a first porous structure, an intermediate, a substrate, an additional substructure of the connected structure.

Preferably, the channel or opening of the holding space is connected to other holding spaces through channels or openings of single or multiple components comprising a first porous structure, an intermediate, a substrate, an additional substructure of the connected structure.

Preferably, said channel or opening of said holding space, directly or indirectly connected to an exposed surface of said connected structure, said exposed surface being exposed to an external open space; with one or more of the following components having said exposed surface; and said components comprising a first porous structure, an intermediate, a substrate, an additional component of the connected structure.

Preferably, one or more of the following components contain channels or openings serving as or connecting with those of the said holding spaces; the said components comprising: a first porous structure, an insertion portion, an intermediate, a substrate, an additional component of the connected structure.

Preferably, said channel or opening of said holding space is open, or closed and to be subsequently opened, or, closed and then no longer to be opened.

Preferably, the holding space for holding the drug, is the same or a different holding space as that for holding the sensors..

Preferably, the channels or openings of the holding space for placing the drug are provided for the delivery of the drug.

Preferably, when the holding space for the drug is provided with openings/channels, the said openings/channels comprise an input port/channel and an output port/channel for the drug; said input and output ports/channels being different openings/channels or the same opening/channel;

Preferably, the said output port of the drug is an open orifice for controlled release, or an orifice closed by a controlled-release plug; and the said input port of the drug is an injection orifice closed by a plug for injection, or an open injection orifice.

Preferably, an external detection device can be used to detect and locate the input channel or input port for the drug, or the channel or opening connected with the input channel or input port.

Preferably, the external detection device uses radiation or ultrasound for detection.

Preferably, the plug for the channel or port is made of any of the following materials; a material that can self-trigger to open up in response to a change in the surrounding environment; a material that can trigger to open up in reaction with a set substance; a material that can degrade over a set period; a material that can dissolve; a material that can allow drugs to pass through; a material that can slowly release drugs.

Preferably, the drug form when placed into the holding space and that when released from the holding space are the same, or different.

Preferably, said drug is liquid, or solid object, or gas when placed into the holding space; and the said drug is liquid, or solid object, or gas when output from the holding space.

Preferably, said connected structure is subjected to a change in the state of the environment to which the drug is subjected, causing a transformation in the form of the drug.

Preferably, said drug reacts with the set substance to transform the form of the drug.

Preferably, the transformed drug is released from the holding space.

Preferably, said drug is encapsulated with any of the following materials; a material that can self-trigger to open up in response to a change in the surrounding environment; a material that can trigger to open up in reaction with a set substance; a material that can degrade over a set period; a material that can dissolve; a material that can allow drugs to pass through; a material that can slowly release drugs.

Preferably, said sensor detects at least one state inside or outside of the said connected structure; or, the said sensor detects a set substance.

Preferably, said sensor detects the temperature, or the pressure, or the humidity of the environment in which the connected structure is located.

Preferably, said sensor detects a set substance, including bacterial strains.

Preferably, said set substance is purposefully placed into the connected structure; or,spontaneously formed within the surrounding environment of the connected structure.

Preferably, said set substance is input from an open space outside the connected structure or from other holding spaces of the connected structure, directly or indirectly, to the holding space for placing the drug and/or to the sensor holding space.

Preferably, said set substance is input through an exposed surface of the connected structure and thus into a holding space directly or indirectly connected to said exposed surface.

Preferably, said set substance is input into the holding space for placing drugs and/or sensors, via channels or ports provided by one or more of the following components, comprising a first porous structure, an insertion section, an intermediate structure, a substrate, or an additional sub-structure of the connected structure.

Preferably, said set substance is input into the holding space prior to, or subsequent to, or simultaneously with the input of the drug.

Preferably, said set substance is water, or another drug.

Preferably, the sensor holding space is provided with a channel or opening for placing the sensor into the holding space.

Preferably, the sensor holding space is provided with a channel or opening, the sensor being provided with an antenna transmitting information or electrical energy in a wireless manner, said antenna being within the holding space and facing said channel or opening, or extending into the outside of the holding space through said channel or opening.

Preferably, the sensor holding space is provided with a channel or opening, the sensor is provided with a probe for detection, said probe being within the holding space and facing said channel or opening, or extending to the outside of the holding space through said channel or opening.

Preferably, said sensor is provided with detection probes or detection surfaces, which are exposed to the outside of the connected structure or are facing channels or openings connected to the exposed surface of the connected structure.

Preferably, devices charging the sensors are provided in the sensor holding space or other holding spaces within the connected structure or provided outside of the connected structure; said charging devices supplying power to the sensors by wired or wireless means.

Preferably, said sensor transmits electrical energy and/or information with other devices via wires; said other connected devices being located in the sensor holding space or other holding spaces of the connected structure,, or outside of the connected structure.

Preferably, the channels or openings of the sensor holding space are used for wiring; and the channels or openings of the following components are used for wiring, which are connected to the sensor holding space; the said components including a first porous structure, an intermediate, a substrate, and additional components of the connected structures.

Preferably, the receiving coil of the wireless charging module cooperates with the external feeding coil to obtain electrical energy wirelessly, and the electrical energy is converted, processed and supplied to the sensors or other electrical components or electrical energy storage elements.

Preferably, the device for charging the sensors or other electrical components of the connected structure, including electrical energy storage elements; said electrical energy storage elements obtaining electrical energy from an external source by wired or wireless means; said electrical energy storage elements provide electrical power by wired or wireless means.

Preferably, the electromagnetic signal trigger switch receives a magnetically induced signal corresponding to an external command and converts it to a corresponding electrical signal to control the sensor or other controlled components of the connected structure; the said electromagnetic signal trigger switch being provided in the holding space where the sensors or other controlled devices are located or in another holding space where there is no sensor or other controlled device; said electromagnetic signal trigger switch providing the converted electrical signal to the sensors or other controlled devices by wired or wireless means.

Preferably, said sensor is paired with a processor for signal transmission and analyses, with the said processor being used to analyze the detection data; said processor and sensor are integrated or both are independent and achieve signal transmission by wireless or wired means; said processor is provided in the sensor holding space or other holding spaces or outside the connected structure .

Preferably, said connected structure is provided with a trigger device, which acts upon the closure bodies plug, or the drug capsule, or opens the drug input or output channel/port by applying a set substance, or by changing the surrounding environment of the connected structure; said trigger device is engaged upon receiving, by wired or wireless means, instructions from sensors, processor, electromagnetic signal trigger switch, external devices, timer, or timing devices.

Preferably, said drug is timed for controlled release at scheduled time points or time intervals. Preferably, said triggering device is for single-use or multiple-uses.

Preferably, said sensor is driven to detect at set time schedule according to instructions from a timer or a timing device; said sensor performs detection at defined time points or time intervals.

Preferably, said holding space has no edge interface along external boundary of the connected structure; or, has edge interfaces in at least some directions.

Preferably, said edge interface of said holding space is either closed or not closed.

Preferably, , that said edge interface of the holding space is formed by a first porous structure or an intermediate or a part of the substrate adjacent to the holding space, or an additional sub-structure of the connected structure.

Preferably, the holding space contains one or more, or none of the following components: a first porous structure, or an intermediate, or a substrate, or an additional sub-structure of the connected structure.

Preferably, components on one or multiple sides around the holding space, individually or cooperatively, secure the solid object; the said components of the connected structure comprise of one or more of the following: a first porous structure, an intermediate, a substrate, an additional component of the connected structure; and said solid object comprises of one or more of the following: a solid object of the contained substance, an encapsulated object of the contained substance, an object formed by edge interfaces of the holding space.

Preferably, a solid object formed directly by the drug itself is directly secured with corresponding components of the connected structure; or, the encapsulated drug is placed into the holding space or secured by corresponding components of the connected structure; or, the solid object or non-solid object formed by the drug itself is placed within a holding space with solid object edge interfaces on at least one side.

Preferably, the sensors are directly secured by corresponding components of the connected structure; or, the sensors are enclosed in a solid object enclosure bodies, which is then placed into the holding space or secured by corresponding components of the connected structure; or, the sensors are placed in the holding space with a solid object edge interface on at least one side.

Preferably, securing the solid object includes loading the solid object: said components support the solid object from one or multiple directions, either individually or cooperatively; said components carry the load through structural parts adjacent to the holding space or those extended into the confinement of the holding space.

Preferably, securing the solid object includes a tight fit to the solid object: said components are in close contact with the solid object from at least two opposite directions; said components achieve tight fit through structural parts adjacent to the holding space or those extended into the confinement of the holding space; through structural parts adjacent to the holding space or those extended into the confinement of the holding space; and the tight fit to the solid object is achieved by different parts of the same component, or different components.

Preferably, securing the solid object includes position-limiting the solid object: said components individually or cooperatively form a position-limiting structure located in at least one direction of the solid object, preventing the solid object located on one side of the position-limiting structure from moving to the other side; said position-limiting structure includes position-limiting protrusions.

Preferably, securing the solid object includes position-limiting the solid object: said components individually or cooperatively form a position-limiting structure in close contact with the solid object from at least one direction; said position-limiting structure includes at least one position-limiting protrusion, formed at a location on the said component adjacent to the holding space and extending into the confinements of the holding space.

Preferably, the part of said solid object corresponding to the position-limiting protrusion is provided with a position-limiting recess matching the position-limiting protrusion so that said position-limiting protrusion can be set into the position-limiting recess; alternatively, the part of said solid object has no position-limiting recess corresponding to the position-limiting protrusion..

Preferably, when the component position-limiting the solid object comprises a first porous structure, said position-limiting protrusion comprises the ends of a portion of the struts of the first porous structure; which are adjacent to the holding space and their ends may extend into the confinements of the holding space.

Preferably, securing the solid object comprises position-limiting the solid object: said component individually or cooperatively forming a position-limiting structure in close contact with the solid object from at least one direction; said position-limiting structure comprising matching position-limiting protrusions and position-limiting recesss, said position-limiting recesss being formed at said parts adjacent to the holding space, said position-limiting protrusions being set on the surface of the solid object and extending into the confinements of the holding space, with the said position-limiting protrusions being set in the said position-limiting recesss.

Preferably, , when the component position-limiting the solid object comprises a first porous structure, said position-limiting recess comprises a portion of the pores of the first porous structure adjacent to the holding space; this portion of the pores retains the form it had when the first porous structure was made, or by machining this portion of the pores of the first porous structure to form said position-limiting recess.

Preferably, , after the composite is connected to the substrate, a part of the connected structure is removed to form at least a part of the holding space, or to form a channel or opening in the holding space; the removed part comprises one or more of the following: a part of the first porous structure, at least a part of the insertion, a part of the intermediate, a part of the substrate, at least a part of the additional components of the connected structure.

Preferably, the removed part is used as a closure bodies plug to subsequently close said channel or opening.

Preferably, the closure bodies that closes the channel or opening of the holding space is formed by the first porous structure or intermediate or part of the substrate adjacent to the holding space, or by an additional component of the connected structure.

Preferably, at least a portion of the intermediate, at a predetermined position of the connected structure, is adjacent to the holding space and serves as a channel or opening of the holding space; after the contained substance is placed into the holding space, that portion of the intermediate is mounted to the predetermined position to achieve closure bodies of the channel or opening.

Preferably, the additional components of the connected structure, includes a component which is used to form the holding space by acting as at least a part of the edge interface of the holding space or forms a closure bodies for closing the channel or opening of the holding space; said component being used to form the holding space by connecting with parts of the first porous structure or intermediate or substrate at locations adjacent to the holding space, or connected with other components of the connected structure.

Preferably, the additional component of the connected structure comprises a filling body which connects a portion of the struts of the first porous structure adjacent to the holding space to form a filling surface; said filling surface acting as at least a portion of the edge interface of the holding space, or forming a closure bodies for closing a channel or opening in the holding space; said filling surface being separately provided at the first porous structure or coupled to an intermediate or substrate or other components of the connected structure.

Preferably, a substance in molten form, after solidification at a designated part of the connected structure, serves as at least a portion of the edge interface of the holding space, or forms a closure bodies for closing a channel or opening in the holding space, or for coupling part a portion of the struts of the first porous structure; the substance is a polymeric material, or a material identical or similar in nature to the first porous structure or intermediate or substrate.

Preferably, the molten substance is injected into the designated part of the first porous structure and then waits for it to solidify; or, the raw material of the substance is placed in the designated position, at least the vicinity of the designated position is heated so that the raw material of the substance becomes molten and fills the pores of the first porous structure around the designated position.

Preferably, the closure bodies for closing the channel or opening is made of any of the following materials: a material identical to the first porous structure or intermediate or substrate; a material having properties similar to the first porous structure or intermediate or substrate.

Preferably, similar properties mean similar electrical conductivities, or similar solid volume fractions.

Preferably, the closure bodies for closing the channel or opening is a solid structure or a porous structure with the same or different solid volume fraction as the first porous structure.

Preferably, all or part of said holding space is formed within the first porous structure; at least part of the surface of said first porous structure is an exposed external surface of said connected structure.

Preferably, a portion of said holding space is formed at or within the first porous structure and other portions of the holding space are formed at or within one or more of the following components; said components comprising an intermediate, a substrate, an additional component of the connected structure.

Preferably, the recess formed at the first porous structure serves as at least a part of the holding space.

Preferably, said recess is formed on an exposed surface of the first porous structure.

Preferably, a specific pore or a plurality of interconnected pores within the first porous structure serves as at least a portion of the holding space.

Preferably, the first porous structure or intermediate or the substrate is formed with recessed or through structures at locations adjacent to the holding space such that a portion of the holding space extends into these structures.

Preferably, a portion of the poress of said first porous structure, serve as channels or openings for holding spaces, or are connected to channels or openings for holding spaces, or are connected to external open spaces, or are connected to channels or openings provided by one or more of the following components: an intermediate, a substrate, an additional component of the connected structure.

Preferably, said channel provided at the substrate, is connected to a channel or opening in the holding space, or to an external open space, or to a channel or opening provided by one or more of the following components: an intermediate, a substrate, an additional component of the connected structure; said channel provided at the substrate, being a duct formed inside the substrate; or, said channel provided at the substrate is a groove which is formed on the surface of the substrate near the side of the composite, and which is open at least partially in the direction in which the composite is located.

Preferably, the interlayer portion between the composite and the substrate, comprises raised structures and/or an intermediate body; said interlayer portion has the following positional relationship with the holding space (a1 and/or a2) :
a1, wherein there is no interlayer portion within the confinements of said holding space, or, wherein said confinements of said holding space contain raised structures and/or an intermediate body;
a2, wherein a part of the said interlayer is adjacent to the holding space and contains raised structures and/or an intermediate body; or, said interlayer is not adjacent to the holding space;
wherein the interlayer between the composite and the substrate contains raised structures without an intermediate body, said raised structures being provided on the side of the first porous structure proximal to the substrate and raised bumps facing toward the substrate;
said interlayer being in contact and connected with the substrate at said raised structures;
or, the interlayer between the composite and the substrate comprises an intermediate body without raised structures, said intermediate body being in contact with and connected to the substrate;
or, the interlayer between the composite and the substrate comprises an intermediate body and raised structures, said raised structure being provided on the side of said intermediate body proximal to the substrate and raised bumps facing towards the substrate; said interlayer being in contact with and connected with the substrate.

Preferably, when said part within or adjacent to the holding space contains raised structures without the intermediate body, said raised structures alone, or in conjunction with one or more of the following components located within or adjacent to the holding space to achieve fixation: a first porous structure, other raised structures, an insertion into the intermediate, an additional component of the connected structure, and a substrate.

Preferably, when said part within the holding space contains raised structures without an intermediate body, said composite body further comprises an extension part, a part of which is connected to raised structures and extends into the holding space; another part of the extension part is connected to one or more of the following parts adjacent to the holding space or outside the holding space: a first porous structure, other raised structures, an insertion portion of an intermediate, an additional component of the connected structure, a substrate.

Preferably, the gaps between adjacent raised structures serve as channels or openings for holding spaces, or are connected to channels or openings for holding spaces, or are connected to external open spaces, or are connected to channels or openings provided by one or more of the following components: the first porous structure, other interlayers of the intermediate, the insertion portions of the intermediate, the substrate, the additional component of the connected structure.

Preferably, said gaps between the raised structures and the substrate serve as channels or openings for the holding space, or are connected to channels or openings for the holding space, or connected to an external open space, or connected to channels or openings provided by one or more of the following components: a first porous structure, other interlayer portions of the intermediate, an inserted portion of the intermediate, the substrate, the additional component of the connected structure.

Preferably, said intermediate body is in the form of a layer, or a sheet, or a plate.

Preferably, the part of said intermediate body located within the holding space, alone, or in conjunction with one or more of the following components to secure the solid object within the holding space: the first porous structure, other parts of the intermediate body, other interlayer parts other than the intermediate body, the insertion portion of the intermediate, an additional component of the connected structure, the substrate.

Preferably, the part of said intermediate body located within the holding space acts as at least a part of the edge interface of the holding space.

Preferably, the part of said intermediate body located within the holding space contains openings to secure the solid object set in the openings.

Preferably, said the part of said intermediate body located within the holding space contains openings, acting as channels or openings of the holding space, or are connected to channels or openings of the holding space, or to an external open space, or to channels or openings provided by one or more of the following components: a first porous structure, other parts of the intermediate body, the interlayer other than the intermediate body, the insertion portion of the intermediate, the additional component of the connected structure, the substrate.

Preferably, the part of said intermediate body located within the holding space contains openings and an extension part is provided on the inside of the openings; the solid object being secured by the extension part.

Preferably, said intermediate body is closed; or, said intermediate body is provided with channels or openings.

Preferably, said channels provided to the intermediate body are formed in the interior of the intermediate body; or, said channel provided to the intermediate body are grooves formed on the surface of the intermediate body near the side of the substrate, and at least a portion of the groove are facing towards the substrate.

Preferably, said intermediate body is provided with a first groove as a channel for the intermediate body, and/or, said substrate is provided with a second groove at said substrate as a channel for the substrate;
wherein the first groove is formed on a surface of the intermediate body near the side of the substrate, and at least a portion of the first groove facing towards the substrate; the
second groove is formed on the surface of the substrate near the side of the composite body, and at least part of the second groove facing towards the composite body;
when the first groove and the second groove are provided at the same time, they are staggered, or they are interlocked to form a channel.

Preferably, when the composite is resistance welded to the substrate, the weld interface of the composite comprises a first porous structure and/or an intermediate body, without raised structures that projects toward the substrate; or, the weld interface of the composite comprises one or more of the first porous structure, the intermediate body, and raised structures that projects toward the substrate.

Preferably, when the composite body is resistance welded to the substrate, said weld interface of the substrate is provided with a substrate raised structure, which project towards the weld interface of the composite body; said substrate is connected to the weld interface of the composite body at least through the substrate raised structures; and, connected to said substrate raised structures, is a weld interface of the composite body on the first porous structure of the composite body, or an interlayer not containing raised structures, or a part of the interlayer at which no raised structure is provided.

Preferably, said connected structure comprises a support part, at least a portion of which is inserted into the first porous structure;
a first end of said support part is proximal to the first side of the first porous structure and a second end of said support part is proximal to the second side of the first porous structure; the second side of the first porous structure is the side proximal to the welding interface of the composite, the first side being the side opposite to the second side;
said support part alone or in conjunction with one or more of the following components to form the holding space, or, said support part alone or in conjunction with one or more of the following components to secure the solid object; said components, comprising: other support parts, the first porous structure, the insertion portion of the intermediate, the interlayer of the intermediate, the substrate, the additional component of the connected structure.

Preferably, said support part passes through the solid object in order to secure it;
the part of the solid object that is not secured by the support part is secured by one or more of the following components, or is not in contact with said components; said components, comprising: other support parts, a first porous structure, an insertion of the intermediate, an interlayer of the intermediate, a substrate, additional components of the connected structure.

Preferably, said holding space is formed, in whole or in part, within the support part.

Preferably, all or part of the surfaces of the support part serve as at least part of the edge interface of the holding space.

Preferably, gaps or openings existing or created in the support part serve as at least part of the holding space; or,is used for securing the solid object; or, is used to place the parts to secure the solid object.

Preferably, the gaps between adjacent support parts, and/or the channels or openings placed at the support parts, serve as channels or openings of the holding spaces, or are connected to channels or openings of the holding spaces, or are connected to external open spaces, or are connected to channels or openings of one or more of the following components: a first porous structure, an interlayer of intermediates, the insertion portion of the intermediate, the substrate, and additional component of the connected structure.

Preferably, after the composite body has been connected with the substrate, the gaps formed subsequent to the removal of all or parts of the support parts becomes at least a part of the holding space.

Preferably, the solid object is provided with an insertion structure, which is inserted to the gaps of the said porous structure to secure the fixation of the solid object.

Preferably, the solid object is provided with an insertion structure, said insertion structure being inserted to a gap formed within the first porous structure at a location corresponding to the insertion site of the support part; the gap is formed by removing all or part of the support part after the composite is connected to the substrate; alternatively, the gap is located between the support part and the first porous structure surrounding the insertion site thereof.

Preferably, the solid object is secured at the support part by means of a hoop structure provided.

Preferably, the solid object is provided with an integrated insertion and hoop structure; the insertion structure of the solid object is inserted at a gap formed within the first porous structure corresponding to the insertion site of the support part, and the hoop structure is fixed to the support part within the said gap.

Preferably, said hoop structure is a closed-hoop structure over the support part; alternatively, said hoop structure is an open-hoop structure in close contact with the support part.

Preferably, the solid object is provided with an insertion structure, said insertion structure being inserted at a gap set in the support part; or, the solid object is provided with an insertion structure, said insertion structure being inserted at a recess set in the support part.

Preferably, a recess is formed at said support part, at which the electrode segment is inserted for connecting the composite body with the substrate; after the composite body is connected to the substrate, the insertion structure of the solid object is inserted into said recess; or, said recess is used as at least part of the holding space.

Preferably, the position into which the insertion structure of the solid object is inserted corresponds to the first end of the insertion site of the support part; the solid object is in contact or not in contact with the surface of the first side of the first porous structure; or, the position into which the insertion structure of the solid object is inserted corresponds to locations other than the first end of the insertion site of the support part, with such locations being adjacent to the holding space; wherein, when the insertion structure is inserted, the first end or other parts of the support part are still present at the insertion area, or have been removed.

Preferably, the position at which the hoop structure of the solid object is fixed corresponds to the first end of the support part insertion site; the solid object is in contact or not in contact with the first side surface of the first porous structure; or, the position at which the hoop structure is fixed corresponds to a part of the support part insertion site other than the first end, which is adjacent to the holding space.

Preferably, the gap corresponding to the insertion site of the support part within the first porous structure, serves as a holding space, or as a channel or opening, or is used to provide other components for securing the solid object;
wherein the gap corresponding to the insertion site, is formed before the composite is connected to the substrate, said support part being within said gap;
or a gap corresponding to the insertion site, formed after the composite body is connected to the substrate, said support part being within said gap, or not located within said gap.

Preferably, prior to the connection of the composite body to the substrate, the support part is in direct contact with the first porous structure around its insertion site; after connecting the composite body with the substrate, all or parts of the support part are removed to form a gap corresponding to the insertion site.

Preferably, prior to the connection of the composite body to the substrate, the support part is not in direct contact with the first porous structure around its insertion site, and, said support part being inserted at the first gap formed within the first porous structure; the support part is separated from the surrounding first porous structure by the said first gap; after the connecting the composite body with the substrate, the space created by removing all or part of the support part is linked to the first gap, forming a gap corresponding to the insertion site.

Preferably, , prior to the connection of the composite body to the substrate, the support part is not in direct contact with the first porous structure around its insertion site, and, said support part being inserted at the first gap formed within the first porous structure; the support part is separated from the surrounding first porous structure by the said first gap; after the connecting the composite body with the substrate, keep the support part, and use the said first gap as the gap corresponding to the insertion site.

Preferably, at the time of connection of the composite body to the substrate, an insulator is provided in the interval of the first gap, separating the support part from the surrounding first porous structure; after connection of the composite body to the substrate, the insulator is removed in whole or in part to form a gap corresponding to the insertion site, or to form an interval connected to said gap.

Preferably, after the composite body is connected to the substrate, the first porous structure around the insertion site remains the same as before the connection of the composite body to the substrate, or is partially removed to form a gap corresponding to the insertion site, or is partially removed to form a space linked to said gap.

Preferably, after the composite body is connected to the substrate, the first end of the support part does not extend beyond the first side surface of the first porous structure, the gap corresponding to the insertion site comprises the gap located between the first end of the support part and the first side surface of the first porous structure; after the composite body is connected to the substrate, the first end of the support part extends beyond or is flush with the first side surface of the first porous structure, then the gap corresponding to the insertion site comprises the gap between the insertion site of the support part and the surrounding first porous structure.

Preferably, the component provided for securing the solid object at the gap corresponding to the insertion site comprises an insertion structure and/or a hoop structure of the solid object; said insertion structure is inserted in the gap and said hoop structure is secured to the support part.

Preferably, after the composite body is connected to the substrate, the first end of the support part extends beyond the first side surface of the first porous structure as an extended portion of the support part; the solid object insertion structure and/or the hoop structure are provided at the extended portion of the support part.

Preferably, the second end of the support part does not extend beyond or is flush with the second side surface of the first porous structure; when the second end of the support part is flush with said second side surface, the intermediate contains an interlayer portion that is or is not in contact with the second end of the support part; When the second end of the support part is flush with said second side surface and the intermediate does not contain an interlayer portion, the second end of the support part is or is not connected to the substrate.

Preferably, the support part is in contact with the side of the interlayer's intermediate body distal to the substrate, the side of the intermediate body proximal to the substrate being provided with or without raised structures;
or, the support part is in contact with the side of the interlayer's raised structures distal to the substrate, said raised structures being provided on the second side surface of the first porous structure, facing towards the substrate.

Preferably, the support part is in direct contact with the raised structures of the interlaye arranged in corresponding locations; or, alternatively, the support part is staggered with the raised structures of the interlayer and is not in direct contact with each other;
wherein said raised structures project towards the substrate and are provided on the second side surface of the first porous structure or on the side of the interlayer's intermediate body, facing the substrate.

Preferably, said support part is made of an insulating material; alternatively, said support part is made of a conductive material and is the insertion portion of the intermediate.

Preferably, said support part is made of a molten substance solidified after injection into the first porous structure; the substance being a polymeric material or a material of the same or similar nature as the first porous structure or intermediate or substrate.

Preferably, said support part is a solid structure; or, said support part is a porous structure with its solid volume fraction higher than that of the first porous structure.

Preferably, said support part is provided with a coupling part and/or an extension part, and serves as at least part of an edge interface, or as a closure bodies to close the channel or opening of the holding space, or for securing the solid object, individually or in conjunction with one or more of the following components: said components, comprising: a support part connected to the coupling part or extension part, other support parts, a first porous structure, an insertion portion of an intermediate, an interlayer of an intermediate, a substrate, other coupling part or extension parts, additional components of the connected structure; wherein two ends of the said coupling part are respectively connected to two support parts;
one end of said extension part is connected to the support part and the other end extends into the open space, or into the first porous structure, or the insertion portion of an intermediate, or the interlayer of the intermediate, or other parts provided to the component of the coupling part structure adjacent to the holding space.

Preferably, a coupling part connected to a pair of support parts at sites proximal to the first end of each of the paired support parts; openings are provided between the said pair of support parts at sites proximal to the second end of each of the support parts; a space is formed between said coupling part and the substrate surface exposed by the openings, and becomes at least a part of the holding space.

Preferably, a coupling part connected to a pair of support parts at sites proximal to the first end of each of the paired support parts; openings are provided between the said pair of support parts at sites proximal to the second end of each of the support parts; where extension parts connected to the support parts are provided; a space is formed between said coupling part and the extension parts and becomes at least a part of the holding space.

Preferably, a first coupling part connected to a pair of support parts at sites proximal to the first end of each of the paired support parts; a second coupling part connected to the same pair of support parts at sites proximal to the second end of each of the support parts; a space is formed between said first and second coupling parts and becomes at least a part of the holding space.

Preferably, said space is connected to an opening in a channel or pipeline located on the surface of the substrate; said channel or pipeline is used for placing wires to connect sensors or for delivering drugs.

Preferably, the portion of the said channel on the surface of the substrate not connected with said space is covered by the intermediate body of the interlayer which is connected to the substrate.

Preferably, the drug holding space is provided with a drug output channel or port in the first or second coupling part and a drug input channel or port in the first or second coupling part.

Preferably, the sensor holding space is provided with openings for the sensor tips and/or antenna to pass through the coupling part close to the first ends of the support parts.

Preferably, said coupling part or extension part bodies are secured by the support parts alone, or by the support parts in cooperation with one or more of the following components: the first porous structure, the insertion portion of the intermediate, the interlayer of the intermediate, the substrate, the additional component of the connected structure; the components that cooperate in securing the extension part are located at one end of the extension part or at locations of the extension part other than its ends; the components that cooperate in securing the coupling part are located at locations of the coupling part other than its ends.

Preferably, the two support parts connected to the ends of the said coupling part are located at the same edge interface of the holding space or at different edge interfaces adjacent or opposite to each other.

Preferably, said coupling part or extension part is made together with the support parts or subsequently provided for connection to the support parts.

Preferably, said gap or notch or recess at the support part is provided for connection with the coupling part or extension part.

Preferably, said coupling part or extension part is a formed part or filling surface provided to the support part; said filling surface comprises a filling body which fills up the pores of the struts of the first porous structure at locations adjacent to the support parts and the holding space; said filling body is formed after solidification of a molten substance at a designated site of the connected structure; the filling body substance is a polymeric material or a material identical or similar in nature to the first porous structure or intermediate or substrate.

Preferably, said coupling part or extension part is made of an electrical-conductive material or an electrical-insulating material.

Preferably, said coupling part or extension part is a solid structure or a porous structure having the same or different solid volume fraction as that of the first porous structure.

Preferably, said composite body is provided with anchor points; each anchor point comprises an anchor point body disposed on the second side of the first porous structure proximal to the substrate; said second side of said anchor point body is in contact with and connected to the substrate; a space formed above the first side of the anchor point body is used as a holding space, or for inserting the insertion structure of a solid object to secure the solid object, or as a channel or opening of the holding space, or connected to a channel or opening of the holding space, or connected to an external open space, or connected to a channel or opening provided by one or more of the following components: a first porous structure, a substrate, an additional component of the connected structure.

Preferably, said anchor point further comprises an anchor point periphery, with at least one anchor point periphery provided on the first side of each anchor point body, or, alternatively, with encircled anchor point periphery provided on the first side of the anchor point body; the anchor point periphery is at least partially inserted within the first porous structure, with the first end of the anchor point periphery proximal to the first side of the first porous structure and the second end of the anchor point periphery proximal to the second side of the first porous structure; wherein the anchor point periphery serves as at least a portion of the edge interface; or, the anchor point periphery is provided with a channel or opening for insertion of the insertion structure of the solid object to hold the solid object, or as a channel or opening for holding the space, or is connected to a channel or opening for holding the space, or is connected to an external open space, or is connected to a channel or opening provided by one or more of the following components: the first porous structure, an intermediate, a substrate, an additional component of the connected structure.

Preferably, said space enclosed by the peripherys of the encircled anchor points corresponds to the space on the first side of the anchor point body.

Preferably, said anchor point is connected to at least one other anchor point, by a coupling part; said coupling part comprises a coupling part body, connected to the anchor point body; said coupling part body is located on a second side of the first porous structure proximal to the substrate; said second side of said anchor point body is or is not in contact with the substrate; or, said coupling part comprises said coupling part body, further comprising at least one coupling part sidewall; said coupling part sidewall being at least partially inserted within the first porous structure, the first and second end of the coupling part sidewall being near respectively the first and second side of the first porous structure; said anchor point connected to the coupling part having no anchor point periphery, or said anchor point connected to the coupling part having an anchor point periphery connected to the coupling part sidewall; said coupling part serves as at least part of the edge interface, or, said coupling part is provided with a channel or opening, or for insertion of an insertion structure of the solid object to secure the solid object, or as a channel or opening of the holding space, or is connected to a channel or opening of the holding space, or is connected to an external open space, or is connected to a channel or opening provided by one or more of the following components: a first porous structure, an intermediate, a substrate, an additional component of the connected structure.

Preferably, said anchor point is a solid structure; or, said anchor point is a porous structure with higher solid volume fraction than that of said first porous structure; said coupling part is a solid structure; or, said anchor point is a porous structure with higher solid volume fraction than that of said first porous structure; the solid volume fraction of said coupling part is or is not consistent with that of said anchor point.

Preferably, anchor point body belongs to the interlayer of said intermediate; said anchor point periphery belongs to the support part; said coupling part body belongs to the interlayer of said intermediate and said coupling part sidewall belongs to the support part.

Preferably, there is a pair of contact surfaces between the composite body and the substrate, and they are connected; or, there is a plurality of pairs of contact surfaces between the composite body and the substrate, and at least one pair of contact surfaces are connected to each other; the holding space is provided at locations on the composite body away from the contact surfaces, or the holding space extends to the contact surfaces of the composite body, or the holding space extends through the contact surface of the composite body to the substrate.

Preferably, the contact surface of the substrate comprises a contact area that contacts and connects to one or a plurality of composite bodies, respectively; or that the contact surface of the substrate comprises a plurality of contact areas that contact and connect to the same composite body; or that the contact surface of the substrate comprises a plurality of contact areas, each of which contacts and connects to one or a plurality of corresponding composite bodies, respectively; where there is a plurality of composite bodies, the holding spaces of adjacent composite bodies are independent from or connected to each other.

Preferably, one contact area of said substrate, corresponding to a surface at that substrate in one same direction or in different directions; and a plurality of contact areas of said substrate, corresponding to a surface at that substrate in one same direction or in different directions.

Preferably, at least one pair of contact surfaces between the composite and the substrate form matching position-limiting openings and position-limiting protrusions; the position-limiting openings being formed on one of the paired contact surfaces, and the position-limiting protrusions being formed on the other paired contact surface while insertable into the position-limiting openings; the contact surface having the position-limiting openings and that having the position-limiting protrusions form an interconnected pair of contact surfaces, or a pair of non-connected contact surfaces; wherein the holding space or the channel or opening of the holding space is placed at locations away from the contact surfaces with position-limiting opening and protrusion; or, the holding space or the channel or opening of the holding space is placed on the contact surfaces with position-limiting opening and protrusion at locations avoiding these opening and protrusion; or the holding space or the channel or opening of the holding space is placed at locations of the position-limiting opening and/or position-limiting protrusion or such locations after further modifications, once the composite body has been connected to the substrate,

Preferably, at least one pair of contact surfaces of the composite and the substrate form matching surfaces thereby achieving positioning; the pair of contact surfaces are or are not connected to each other; wherein the holding space, or the channel or opening of the holding space, is provided away from or at the positioning contact surfaces.

Preferably, said substrate is provided with a recess, said composite body comprising a part inserted into the recess, between which and the recess of the substrate there are a pair of contact surfaces, where the composite and the substrate are connected; or, between the composite body insertion part and the recess of the substrate, there are multiple pairs of contact surfaces, where the composite and the substrate are connected at least at one pair of the contact surfaces.

Preferably, the part of the composite body at which it is inserted into the recess of the substrate comprises an interlayer portion of the intermediate; said recess of the substrate comprises an opening, a first surface opposite the opening, and a side surface disposed between the opening and the first surface; said interlayer portion of the composite body comprises a second surface in contact with the first surface of the recess, said side surface of the recess being in contact or not in contact with a first porous structure along the corresponding direction of the composite body; or, said interlayer comprises said second surface, and a third surface in contact with the side surfaces of the recess; wherein the second surface and/or the third surface of the interlayer is adjacent to a part of the holding space as at least a portion of the edge interface of the holding space; or, the second surface of the interlayer and/or the third surface adjacent to the holding space is removed such that the holding space extends to the first surface and/or side surfaces of the recess of the substrate; or, the second surface of the interlayer and/or the third surface adjacent to the holding space is used to secure the solid object, either alone or in conjunction with one or more of the following components; or, the second surface of the interlayer and/or the third surface adjacent to the holding space is provided with a channel or opening to be used as the channel or opening of the holding space, or the channel or opening connected to the holding space, or the channel or opening connected to an external open space, or the channel or opening connected to one or more of the following components; said components, comprising: a recess of the substrate, a first porous structure, other parts of the interlayer, other intermediates, additional components of the connected structure.

Preferably, at least one of the sides of said recess of the substrate is beveled, which forms a set angle with the first surface; a third surface in contact with this side is beveled, which forms a set angle with the second surface.

Preferably, said angle is acute.

Preferably, the composite body is connected to the substrate by laser welding or resistance welding; said holding space is formed before, or during, or after connecting the composite body to the substrate.

Preferably, the interlayer is welded to the substrate by laser welding, and after completion of the welding, the space formed in the first porous structure is used as a holding space, or for insertion of the insertion structure of the solid object to hold the solid object, or as a channel or opening of the holding space, or is connected to a channel or opening in the holding space, or is connected to an external open space, or is connected to a channel or opening provided by one or more of the following components: a first porous structure, an intermediate, a substrate, an additional component of the connected structure.

Preferably, said connected structure comprises an intermediate layer of polymeric material; said holding space is provided with or without using a polymeric material layer; The polymeric material layer is formed at chosen locations when a polymeric material is injected into any one of the porous structures of the connected structure, or between any one of the porous structures and the substrate, or between two adjacent porous structures, with the molten polymer material penetrating into the connected part.

Preferably, at least part of the surface of said intermediate layer of polymeric material is used as at least part of the edge interface of the holding space; or, all or part of the holding space is formed within the intermediate layer of polymeric material; or, at least part of the surface of said layer of polymeric material is used alone or in conjunction with one or more of the following components, for securing the solid object; a first porous structure, a substrate, a support part, an interlayer of the intermediate, an additional component of the connected structure.

Preferably, said holding space is provided in a component within the connected structure that does not have a polymeric material interlayer; or, the holding space is provided in an area away from the polymeric material interlayer within a component having a polymeric material interlayer.

Preferably, the intermediate layer of polymeric material is formed within the first porous structure and/or formed within the second porous structure as an interlayer.

Preferably, a polymeric material interlayer is formed between the first porous structure and the substrate; or, a polymeric material interlayer is formed between the second porous structure as the interlayer and the substrate.

Preferably, an intermediate layer of polymeric material is formed between the first side of the first porous structure and a fourth porous structure; the fourth porous structure is arranged separately and forms another composite body with pre-connected or integrally formed fifth porous structure, the fourth and fifth porous structures having different solid volume fraction; the first side of the first porous structure being its side distal from the substrate.

Preferably, a barrier layer is provided within the porous structure where the intermediate layer of polymeric material is located or adjacent thereto, and this barrier is provided for position-limiting the location where the polymeric material is injected and it penetrates; said barrier layer is integrally formed with the porous structure in which it is located or is separately provided to that porous structure.

Preferably, said barrier layer serves as at least part of the edge interface of the holding space, or is used to secure the solid object, either alone or in conjunction with one or more of the following components; a first porous structure, a substrate, a support part, an interlayer of the intermediate, an additional component of the connected structure, a polymeric material layer.

Preferably, said barrier layer is a solid structure or a porous structure, its solid volume fraction being higher than that of the porous structure into which the polymeric material is injected.

Preferably, said polymeric material layer or barrier layer is provided with channels or openings, as channels or openings of holding spaces, or channels or openings connecting to holding spaces, or channels or openings connecting to external open spaces, or channels or openings provided by one or more of the following components; said components, comprising: a first porous structure, a support part, an intermediate interlayer, a substrate, an additional component of the connected structure.

Preferably, said polymeric material layer or barrier layer acts as a closure bodies for the channel or opening.

Another technical solution of the present invention is to provide a prosthetic implant, said prosthetic implant provided with a connected structure of any of the above porous structures and substrates; said prosthetic implant, comprising: a composite, comprising a first porous structure pre-connected or integrally formed with an intermediate; said intermediate having a higher solid volume fraction than the first porous structure; a prosthetic implant body, serving as a substrate; at least part of the surface of said prosthetic implant body provided as a connection region connected to the first porous structure and/or the intermediate of the composite; wherein said connected structure is provided with at least one holding space for placing drugs and/or sensors.

Preferably, said prosthetic implant is an orthopedic prosthetic implant.

Preferably, said prosthetic implant is an joint prosthesis.

Preferably, said composite body is formed as a shell, wrapped around connection regions of said prosthetic body, in contact with and tapercted to the connection regions. The surfaces of the body of the prosthetic implant on which the connection regions are located correspond to the same orientation of the body of the prosthetic implant or different orientations.

Preferably, said composite shell is a single unit; or, said composite shell comprises a plurality of shell pieces; wherein the plurality of shell pieces are independent of each other or are connected to each other along at least one adjacent side between the adjacent shell pieces.

Preferably, said prosthetic implant is any of the following: femoral stem, acetabular cup, fusion cage, femoral condyle, tibial tray, spinal prosthetic implant, ankle joint, shoulder joint, elbow joint, finger joint, toe joint, facet joint, mandibular joint, wrist joint, tooth implant.

Preferably, said prosthetic implant is a femoral stem of the hip joint, said stem body of said femoral stem serving as a substrate; at least part of the surface of the proximal end of said stem body being provided with connection regions.

Preferably, said prosthetic implant is an acetabular cup of the hip joint, said cup body serving as a substrate; at least part of the outer surface of the cup body is provided with connection regions.

Preferably, said prosthetic implant is a fusion cage, the fusion cage of said fusion cage serving as a substrate; said connection regions are located at least on the upper and lower surfaces of said fusion cage.

Preferably, said prosthetic implant is a femoral condyle, the body of the femoral condyle serving as a substrate; said femoral condyle body having connection regions on at least a portion of its inner recess surface.

Preferably, said prosthetic implant is a tibial tray, the body of the tibial tray serving as a substrate, said connection regions being located on the inferior surface of the tibial tray.

Preferably, said porous surface structure is further added on its surface with any one or more of the following: an osteoconductive coating, an osteoinductive coating, an antimicrobial coating, a carrier of cells or growth factors.

Preferably, the open space outside the connected structure includes the body structure where the prosthetic implant is implanted.

Preferably, the drug is input to the holding space before, or during, or after implantation of the prosthetic implant.

Preferably, the set substance is input to the holding space before, or during, or after implantation of the prosthetic implant, wherein the set substance reacts with the closure bodies which closes the channel or opening of the holding space, thereby opening up the closure bodies; or, the set substance reacts with a drug in the holding space, causing a change in the form of the drug; or, the set substance reacts with the capsule of the drug, thereby opening up the capsule.

Preferably, said sensor is connected to one end of a wire and the other end of the wire is connected to an interface located on the body surface.

Another technical solution of the present invention is to provide a method of using the connected structure of a porous structure and a substrate, any of the connected structure as mentioned above, comprising: a composite comprising a pre-connected or integrally formed first porous structure with an intermediate, said intermediate having a higher solid volume fraction than that of the first porous structure; a substrate, which is connected to the first porous structure and/or intermediate of the composite; said connected structure provided with at least one holding space for placing drugs; said connected structure provided with sensors for detecting at least one state inside or outside the connected structure, or for detecting a set substance; wherein said set substance is manually input from external sources to the connected structure or is self-generated within the internal environment of the connected structure; the result of the sensor detection being used to determine whether to open the input or output channel or port of the drug holding space.

In the examples of the connected structure of the present invention, a composite body is manufactured by pre-connecting parts or with an integral-forming process (3D printing, etc.), the said composite body comprising a first porous structure and an intermediate (solid body or porous structure with lower porosity) with a higher solid volume fraction; the composite body is effectively bonded to the substrate using a welding method (laser welding or resistance welding); the laser welding beam acts on the intermediate to avoid the original laser energy directly acting on the first porous structure and breaking its struts, making the connection between the composite body and the substrate more reliable; projection resistance welding method uses contact resistance to generate a local heat source to achieve welding, greatly reducing or avoiding the significant decrease in mechanical properties of the substrate caused by hot pressing processes (such as diffusion welding process); the invention can also use the combined projection resistance welding and spot resistance welding to further enhance the welding strength between the intermediate body and the substrate and to reduce the surface damage of the porous surface structure.

The substrate of the connected structure in the examples of the present invention can be manufactured by various processes such as forging, casting or machining or powder metallurgy. Said substrate may be solid object or may be a porous structure with high solid volume fraction (the first porous structure has a lower solid volume fraction than the substrate), while the solid volume fraction of the intermediate may be in between them. The present invention achieve the effective combination of a porous surface and a solid substrate (high solid volume fraction), satisfies the overall requirements of the connected structure and the surface properties at the same time, and avoids the problem of substantial decrease of the mechanical properties of the substrate caused by hot pressing process, etc., so that the strength of the substrate is not substantially affected. The present invention also avoids 3D printing of the entire solid substrate (high solid volume fraction), which simplifies the manufacturing process, thereby reducing manufacturing costs and also saving time.

In the connected structure of the present invention, support parts are provided within the first porous structure for position-limiting the electrodes or indenter to ensure that the surface of the first porous structure is maintained at a predetermined position after the resistance welding process, in order to avoid excessive compression of the first porous structure; when the support parts are used for position-limiting, an insulating material or a conductive material can be used to make the support parts either as solid objects or porous structures with high solid volume fractions; when the support parts use good conductive materials, they can further guide the electrode output current to flow mostly and preferentially through the support parts to the welding interface of the composite body, thereby improving the current conduction efficiency, ensuring the welding strength between the composite body and the substrate, and reducing the damage to the surface of the first porous structure.

The connected structure of the example in the present invention provides an interlayer to the welding interface of the first porous structure, such as an intermediate body and/or raised structures , connecting the porous surface to the substrate through the interlayer; or, raised structures on the substrate can be provided to the welding interface and connects to non-raised parts such as the interlayer body or the first porous structure of the interlayer. The present invention uses the raised structure of the interlayer, or the raised structure of the substrate, to adjust the contact resistance of the welding interface, thereby improving the welding strength.

In the connected structure of the present invention, in order to adjust the contact resistance of the welding interface, change the path of current conduction, or improve the efficiency of current conduction, etc., conductive support parts placed within the first porous structure, the main body of the intermediate, raised structures on the intermediate etc. can be used. 3D printing and other integral-forming processes can be used conveniently, based on optimal designs, to make parts such as the support parts placed within the first porous structure, the main body of the intermediate, raised structures on the intermediate etc. Such technologies simply the manufacturing processes, cut manufacturing costs, and reduce manufacturing time.

The connected structure of the examples in the present invention is provided with a holding space, at least partially through the first porous structure. Preferably, the holding space is formed with various parts connecting the composite body to the substrate, such as the support part, the intermediate body, the raised structure, etc., so as to not only achieve a reliable connection between the composite body and the substrate, but also to make further use of these parts in the formation of the holding space so as ,to meet various needs for holding and utilizing the solid objects within the holding space.

The invention utilizes the connected structure of the porous structure and the substrate to form various prosthetic implants, especially various orthopedic prostheses including joint prostheses, such as femoral stem, acetabular cup, etc. It renders the prosthetic implant easy to process and to have high strengths, while optimizing the bone ingrowth properties through the porous surface and minimizing the cross section of the prosthetic implant (such as femoral stem). The connected structure can be equipped with sensors to detect various states around the prosthetic implant and detect infections in a timely manner; it can also be equipped with drugs that can be injected and released as needed to prevent or treat peri-prosthetic infections for a better user experience for the patients.

### Briefly Descriptions of Drawings

FIG. 1 is a schematic diagram of the direct connection of the first porous structure to the substrate in prior art;
FIG. 2 is a schematic diagram of the connection of the intermediate body of the composite body to the substrate as described in the present invention;
FIG. 3 is a schematic diagram of the connection of the intermediate body and the raised structure of the composite body to the substrate as described in the present invention;
FIG. 4 is a schematic diagram of the raised structure of the composite body described in the present invention.
FIG. 5 is a schematic diagram of the connection of a second porous structure of the composite body to the substrate as described in the present invention;
FIG. 6 is a schematic diagram of the connection of a second porous structure and raised structures of the composite body to the substrate as described in the present invention;
FIG. 7 is a schematic diagram of the present invention using flexible electrodes;
FIG. 8 is a schematic diagram of the present invention using a flexible conductive medium; FIG. 9 is a schematic diagram of another example of the present invention using a conductive medium;
FIGS. 10 to 14 are schematic diagrams of the first porous structure used to set up the holding space and the contained substance, as described in Embodiment I of the present invention;
FIG. 15 is a schematic diagram of the present invention in which the filler body is provided at the first porous structure;
FIG. 16 is a schematic diagram of two composite bodies connected to two opposing surfaces of the substrate as described in the present invention;
FIG. 17 is a schematic diagram of the connection of the raised structure to the substrate and the secured solid object of Embodiment II of the present invention;
FIG. 18 is a schematic diagram of the raised structure used to set up the holding space and the contained substance, as described in Embodiment II of the present invention;
FIG. 19 is a schematic diagram of the connection of the raised structure of the substrate to the intermediate body, as described in the present invention;
FIGS. 20 and 21 are two examples of the heat fusion state of the intermediate body with the first porous structure, as described in the present invention;
FIGS. 22 and 23 are schematic diagrams of the polymer material before and after filling between the first porous structure and the substrate, as described in Embodiment III of the present invention;
FIG. 24 is a schematic diagram of the connected structure based on FIG. 23 to set up the holding space and the contained substance;
FIGS. 25 and 26 are schematic diagrams of the polymer material before and after filling between the second porous structure and the substrate, as described in Embodiment III of the present invention;
FIG. 27 is a schematic diagram of the connected structure based on FIG. 26 to set up the holding space and the contained substance;
FIGS. 28 and 29 are schematic diagrams of the polymer material before and after filling between the first porous structure with a barrier layer and the substrate, as described in Embodiment III of the present invention;
FIG. 30 is a schematic diagram of the connected structure based on FIG. 29 to set up the holding space and the contained substance;
FIG. 31 is a schematic diagram of placing the polymer material between the a fourth porous structure and a first porous structure, and setting up the holding space and contained substance, as described in Embodiment III of the present invention ;
FIG. 32 is a schematic diagram of placing the polymer material between the a fifth porous structure and a first porous structure, and setting up the holding space and the contained substance, as described in Embodiment III of the present invention ;
FIG. 33 is a schematic diagram of placing the polymer material between the a fourth porous structure with a barrier layer and a first porous structure, and setting up the holding space and contained substance, as described in Embodiment III of the present invention ;
FIGS. 34 to 42 are schematic diagrams of the connected structure with support parts to set up the holding space and the contained substance, as described in the present invention; wherein,

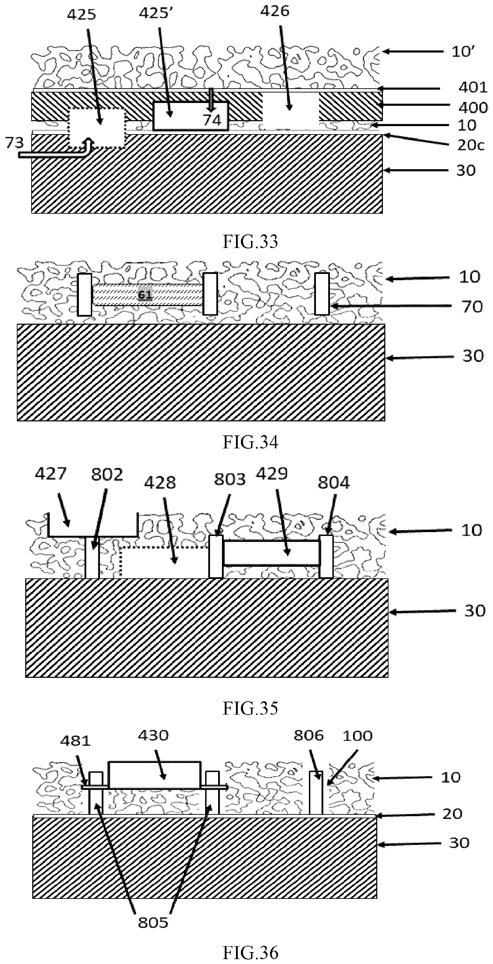

The top of the support part of FIG. 34 is below the top surface of the first porous structure and its does not contact the substrate.

The top of the support part of FIG. 35 is below the top surface of the first porous structure and its bottom contacts the substrate;

The top of the support part of FIG. 36 is below the top surface of the first porous structure and its bottom contacts the intermediate body;

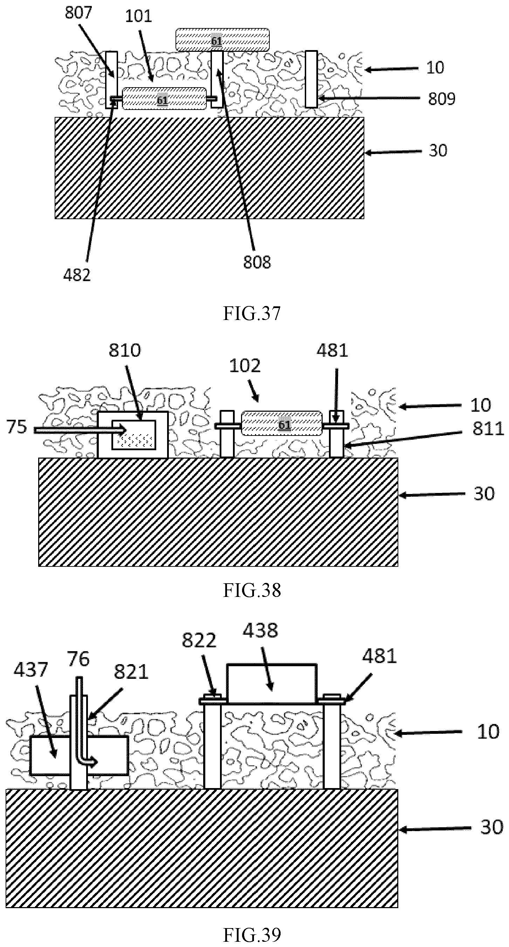

The top of the support part of FIG. 37 is flush with the top surface of the first porous structure and its bottom does not contact the substrate;

The top of the support part of FIG. 38 is below the top surface of the first porous structure and the bottom contacts the substrate;

The top of the support part of FIG. 39 is above the top surface of the first porous structure and the bottom contacts the substrate;

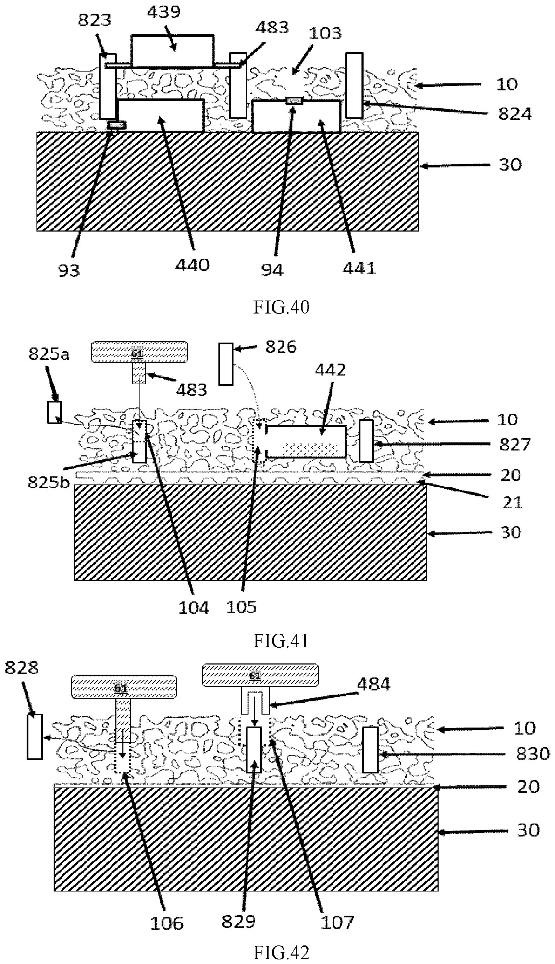

The top of the support part of FIG. 40 is above the top surface of the first porous structure and the bottom does not contact the substrate;

The top of the support part of FIG. 41 is below the top surface of the first porous structure top surface and its bottom does not contact the intermediate body and the raised structure;

The top of the support part of FIG. 42 is below the top surface of the first porous structure and its bottom does not contact the intermediate body.

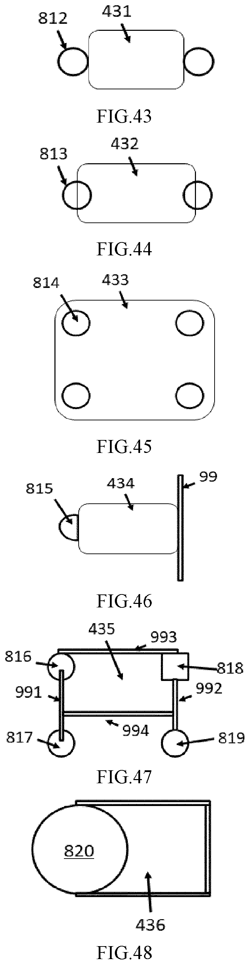

FIGS. 43 to 51 are schematic diagrams in the top view direction; where FIGS. 43 to 48 are schematic diagrams of support parts used to set up the holding space and the contained substance;

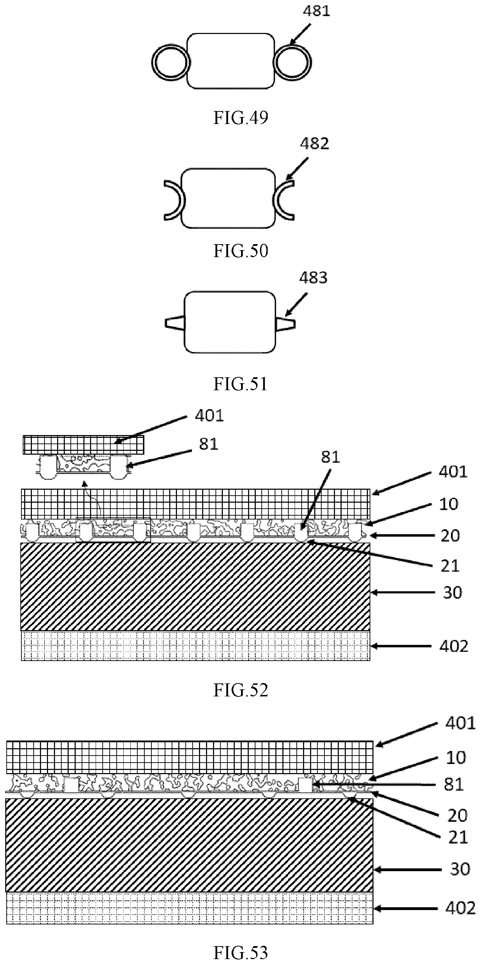

FIG. 49 is a schematic diagram of the closed hoop structure;

FIG. 50 is a schematic diagram of the open hoop structure;

FIG. 51 is a schematic diagram of the lateral insertion structure;

FIGS. 52 to 61 are schematic diagrams of a composite body with support parts connected to a substrate, as described in the present invention; wherein

The top of the support parts of FIGS. 52 to 55 are below the top surface of the first porous structure prior to welding.

The intermediate of FIG. 52 comprising the intermediate body, corresponding support parts and raised structures;

The intermediate of FIG. 53 comprising the body of the intermediate, staggered support parts and raised structures.

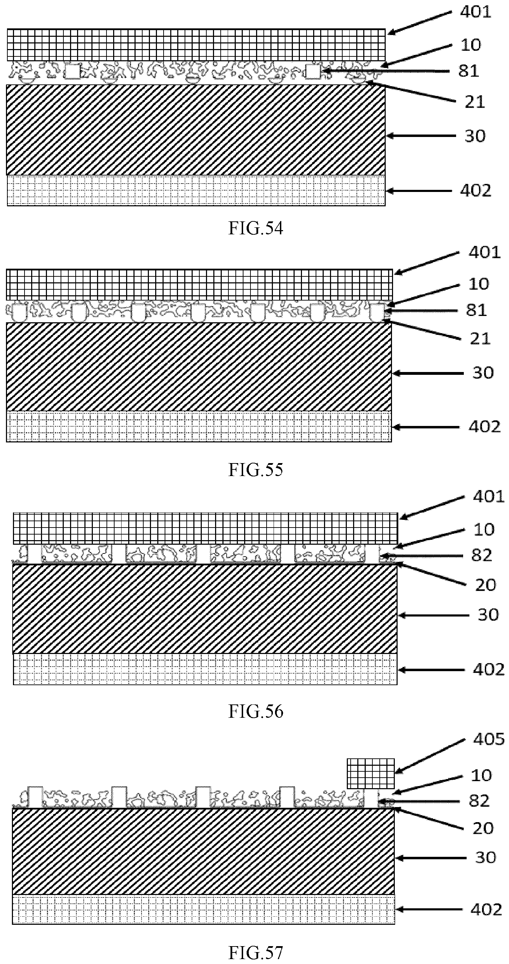

The intermediate of FIG. 54 containing staggered support parts and raised structures;

The intermediate of FIG. 55 containing corresponding support parts and raised structures;

The top of the support part of Figures 56 to 57 is flush with the top surface of the first porous structure before welding, the intermediate containing the intermediate body and the support parts, and the first polar electrode using a planar electrode and an electrode segment, respectively.

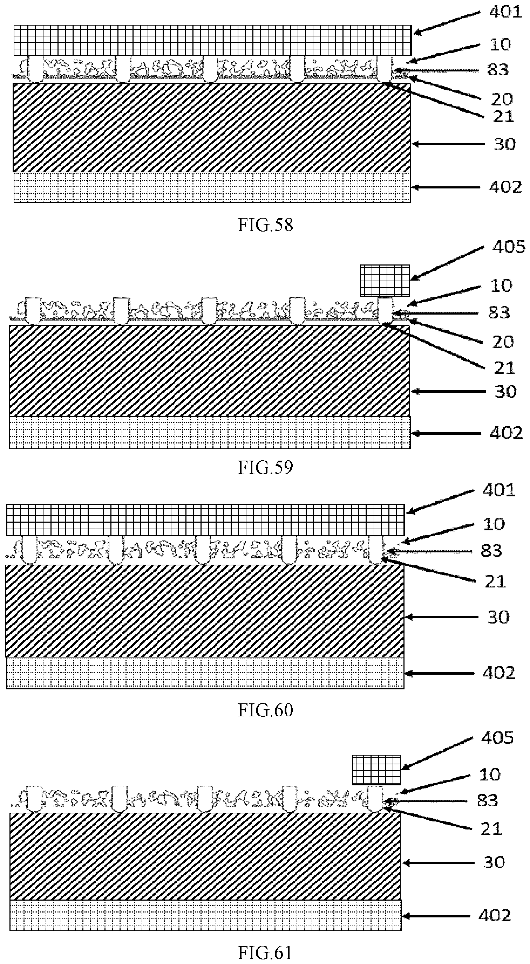

The top of the support part of FIGS. 58 to 61 is above the top surface of the first porous structure prior to welding;

The intermediates of FIGS. 58 and 59 comprise an intermediate body, corresponding support parts and raised structures, a first polar electrode using a planar electrode and an electrode segment, respectively.

The intermediates of FIGS. 60 and 61 contain corresponding support parts and raised structures, with the first polar electrode using a planar electrode and an electrode segment, respectively.

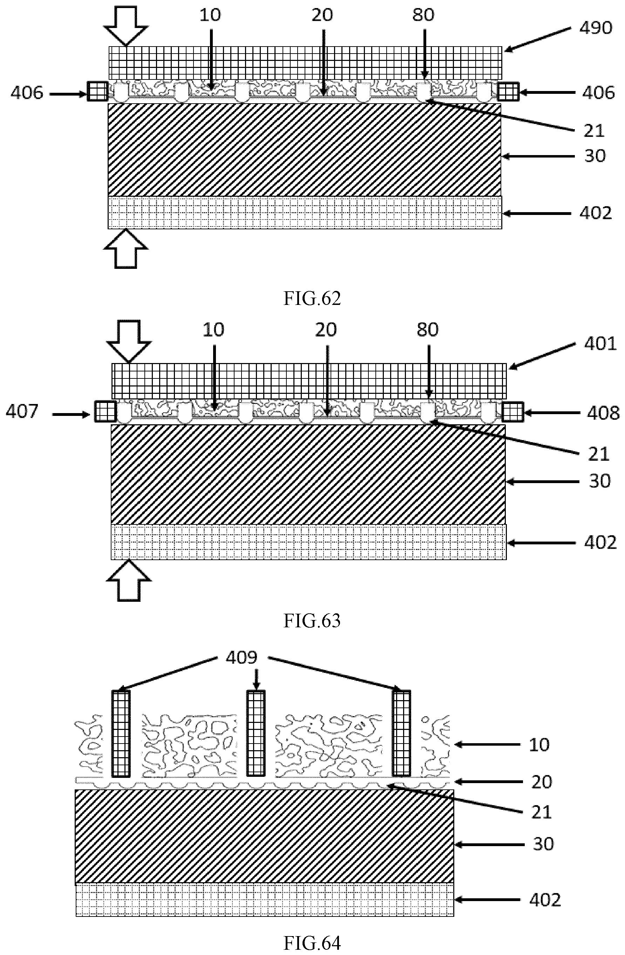

FIG. 62 is a schematic diagram of the first polar electrode using the indenter and the side;

FIG. 63 is a schematic diagram of the first polar electrode using both the top and the side; FIG. 64 is a schematic diagram of the first polar electrode segment inserted into the gap of the first porous structure in contact with the intermediate body;

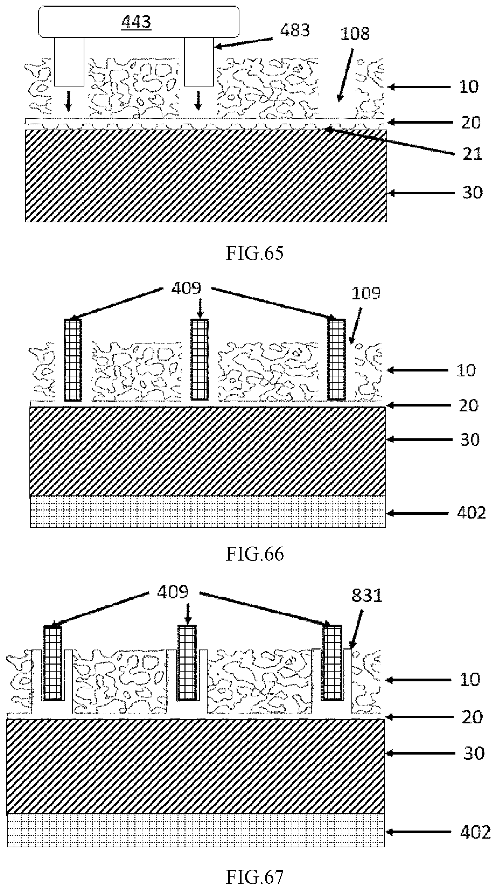

FIG. 65 is a schematic diagram of the use of the gap left after welding of FIG. 64 to form the holding space and placing the contained substance;

FIG. 66 is another example of a first polar electrode segment inserted into the gap of the first porous structure in contact with the body of the intermediate.

FIG. 67 is a schematic diagram of the first polar electrode segment inserted into the gap of the support part in contact with the intermediate body;

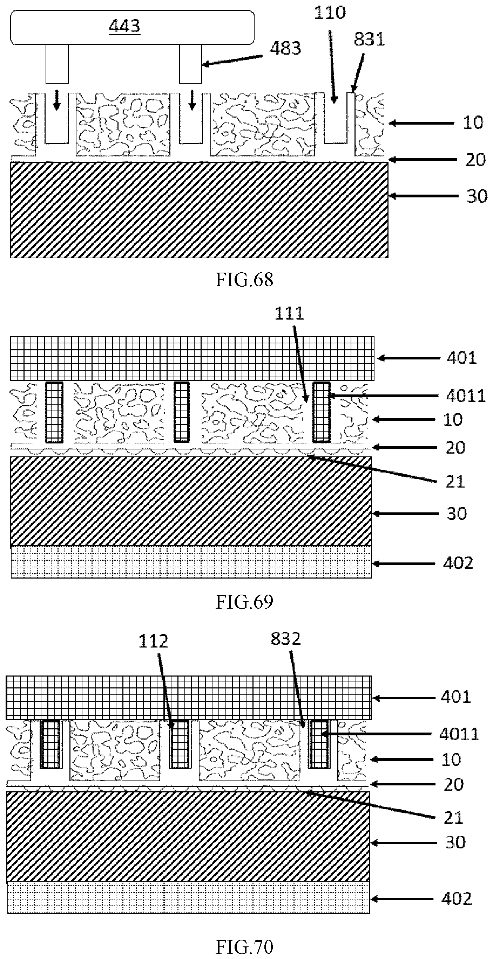

FIG. 68 is a schematic diagram of using the gap left after the welding of FIG. 67 to set up the holding space and the contained substance;

FIG. 69 is a schematic diagram of the first polar electrode in contact with the intermediate body through the conductive medium inserted into the gap of the first porous structure

FIG. 70 is a schematic diagram of the first polar electrode in contact with the intermediate body through the conductive medium inserted into the gap of the support part a schematic diagram of contact with the body of the intermediate.

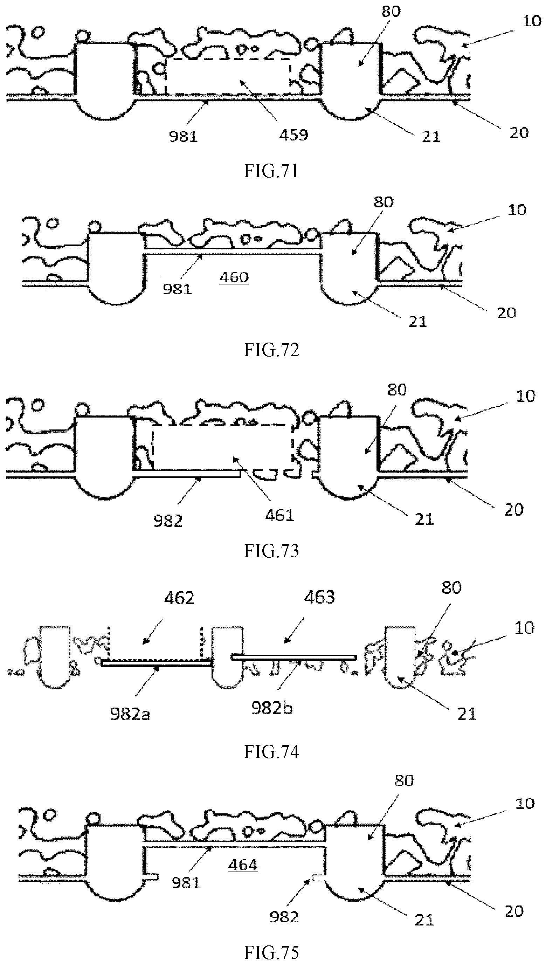

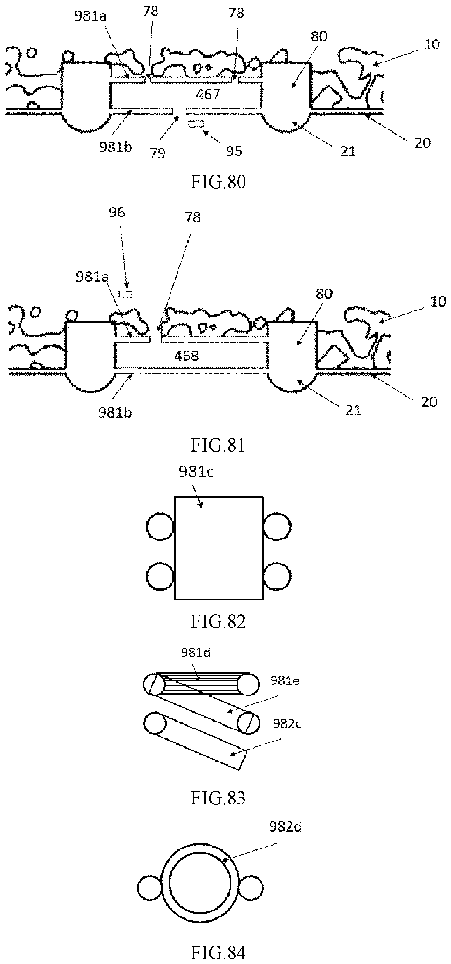

FIGS. 71 to 85 are schematic diagrams of the support part described in Embodiment VI of the present invention provided with a coupling part and/or an extension part, and provided with a holding space and contained substance; wherein FIGS. 82 to 85 are schematic diagrams in the top view direction.

The coupling part of Figure 71 is flush with the body of the intermediate;

The coupling part of Figure 72 is higher than the body of the intermediate, forming the first pocket structure of the holding space;.

The extension part of FIG. 73 is flush with the body of the intermediate;

The support parts of FIG. 74 have two extension parts of different heights connected.

The coupling part, the support part, the intermediate body with openings and extension parts of FIG. 75, forming the second pocket structure of the holding space;

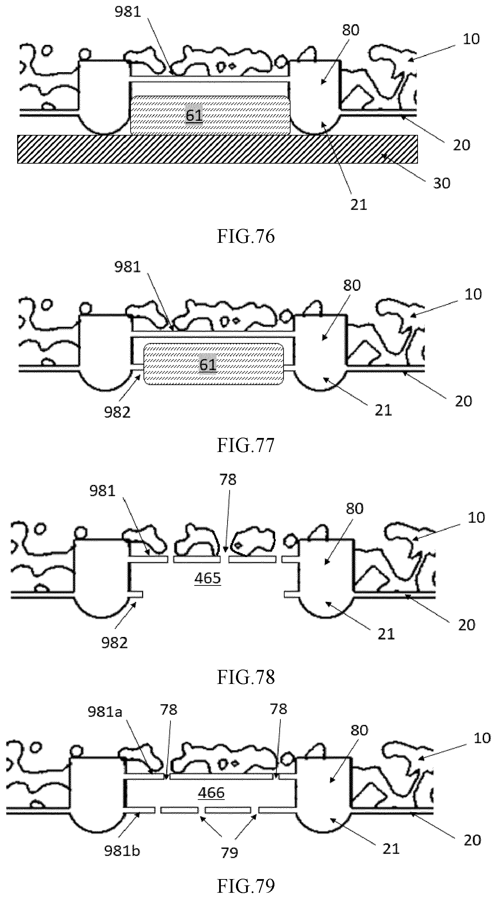

FIG. 76 is an example of setting contained substances based on the holding space of FIG. 72;
FIG. 77 is an example of setting contained substances based on the holding space of FIG. 75;
FIG. 78 is an example of opening through-holes in the coupling part based on FIG. 75;
FIGS. 79 to 81 consist of two sets of coupling parts with support parts, forming the third, fourth, and fifth pocket structures, respectively, of the holding space;
Both sets of coupling parts of Figure 79 are provided with through holes.

Both sets of coupling parts of FIG. 80 are provided with through-holes, and the through-holes of the lower coupling part are closed.

The upper coupling part of FIG. 81 has a through hole and is closed.

FIG. 82 is a schematic diagram of a coupling part corresponding to a plurality of support parts;

FIG. 83 is a schematic diagram of supports parts connected with coupling parts at different heights and orientations and connected with extension parts;

FIG. 84 is a ring-shaped extension part;

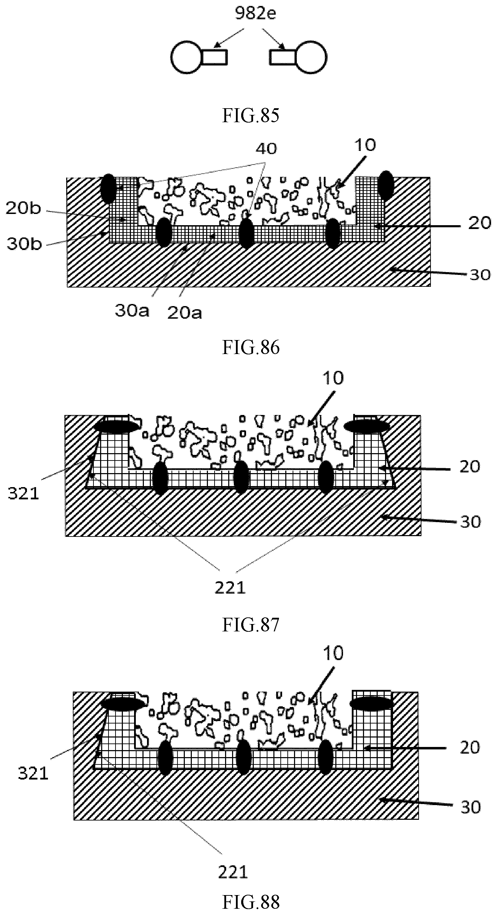

FIG. 85 is a segmented extension part.

FIGS. 86 to 93 correspond to Embodiment VII, wherein:
FIG. 86 is a schematic diagram of the composite body connected to a substrate having a recess, as described in the present invention ;
FIG. 87 is a schematic diagram of the composite body and the recess of the substrate having corresponding beveled edges on both sides, as described in the present invention ;
FIG. 88 is a schematic diagram of the composite body and the recess of the substrate having corresponding beveled edges on one side, as described in the present invention;

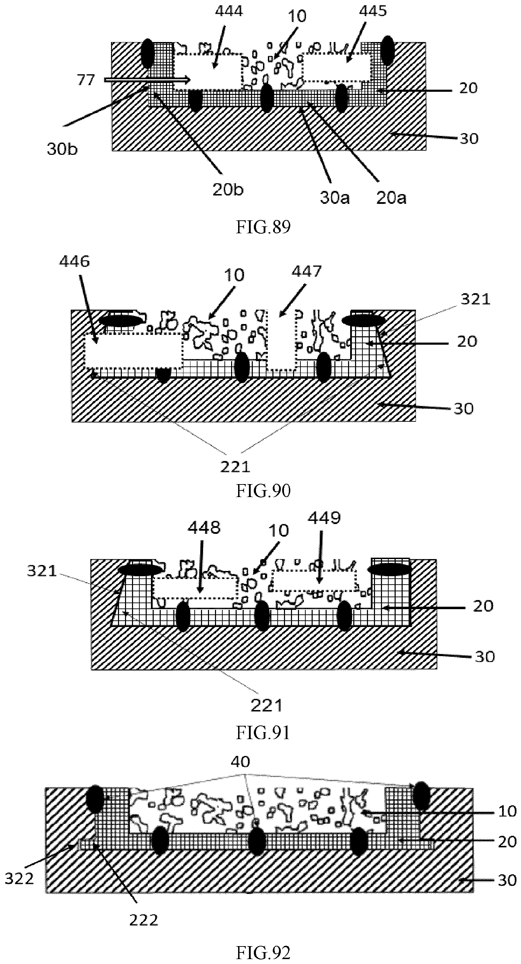

FIGS. 89 to 91 are schematic diagrams of setting up a holding space and contained substance based on the connected structure of FIGS. 86 to 88, respectively.
FIG. 92 is a schematic diagram of the composite body and the recess of the substrate having a snap structure on both sides, as described in the present invention;

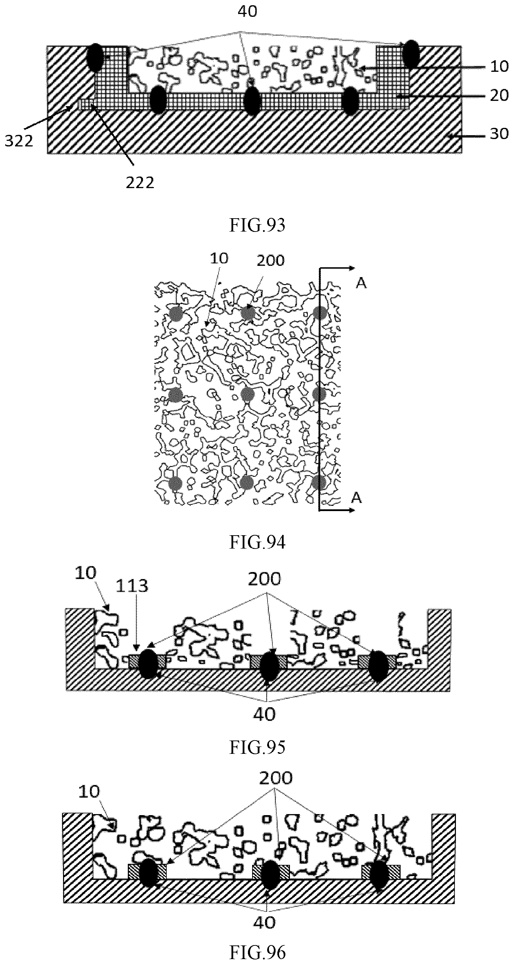

FIG. 93 is a schematic diagram of the composite body and the recess of the substrate having a snap structure on one side, as described in the present invention;
FIGS. 94 to 113 correspond to Embodiment VIII, wherein,
FIG. 94 is a schematic diagram of the freestanding anchor point structure of the composite of the present invention in a top view direction;
FIG. 95 is a schematic diagram of the freestanding anchor point structure with a gap above the anchor point, as described in the present invention;
FIG. 96 is a schematic diagram of the freestanding anchor point structure with the anchor point above filled by a porous structure, as described in the present invention;

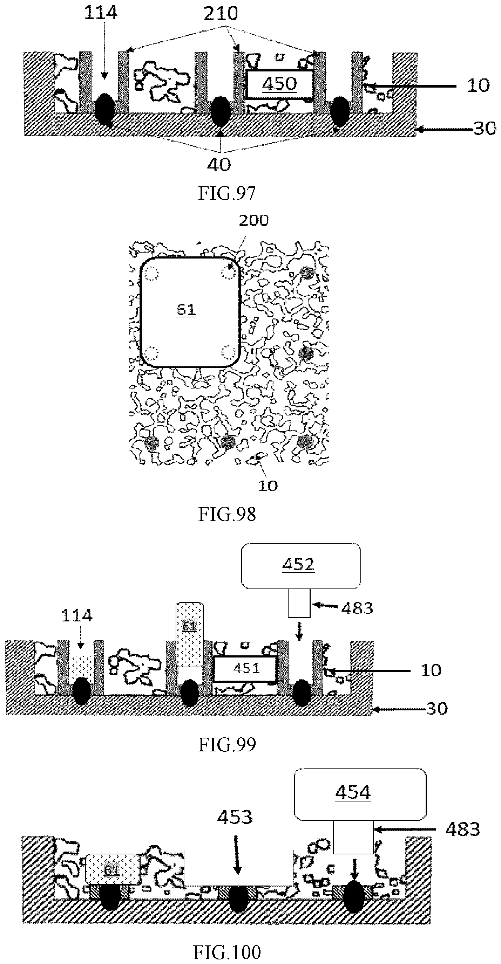

FIG. 97 is a schematic diagram of the freestanding anchor point structure with the anchor point containing the bottom and the periphery, as described in the present invention;
FIGS. 98 to 101 are schematic diagrams of setting up the holding space and the contained substance based on the connected structure of FIGS. 94 to 97, respectively.

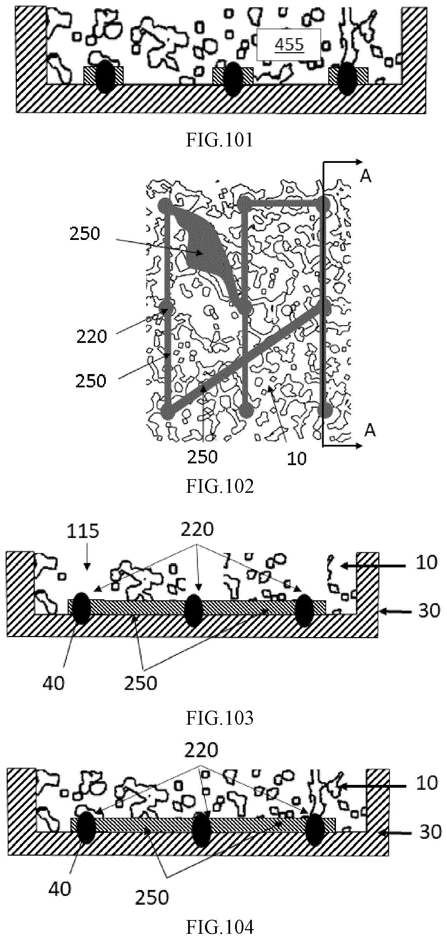

Fig. 102 is a schematic diagram of the top view direction of the jointed anchor point structure of the composite of the present invention;
Fig. 103 is a schematic diagram of the gap above the anchor points of the jointed anchor point structure of the present invention;
Fig. 104 is a schematic diagram of the jointed anchor point structure with the anchor point above being filled with a porous structure, as described of the present invention;

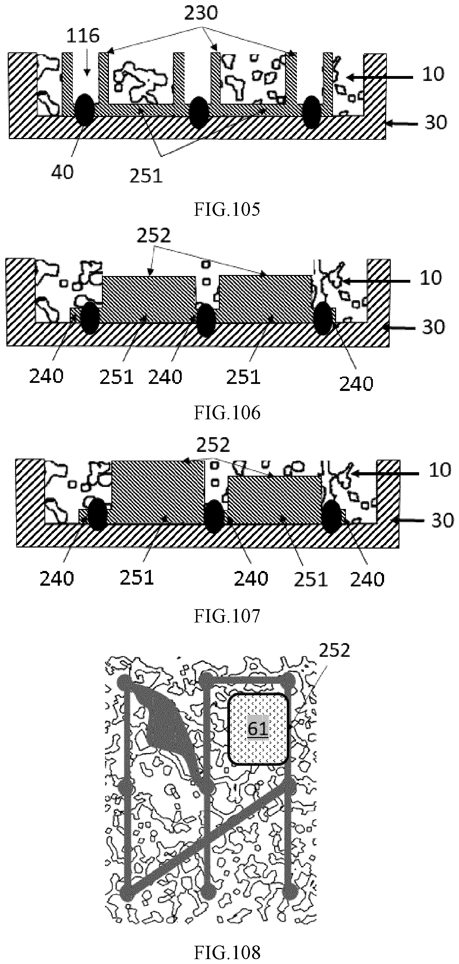

Fig. 105 is a schematic diagram of the jointed anchor point structure of the present invention, with anchor points containing the bottoms and the periphery;
Fig. 106 is a schematic diagram of the jointed anchor point structure of the present invention with the same height of the sidewalls of each coupling part;
Figure 107 is a schematic diagram of the coupling part anchor point structure of the present invention with different heights of the sidewalls of each coupling part.
FIGS. 108 to 113 are schematic diagrams of setting up the holding space and the contained substance based on the connected structure of FIGS. 102 to 107, respectively.

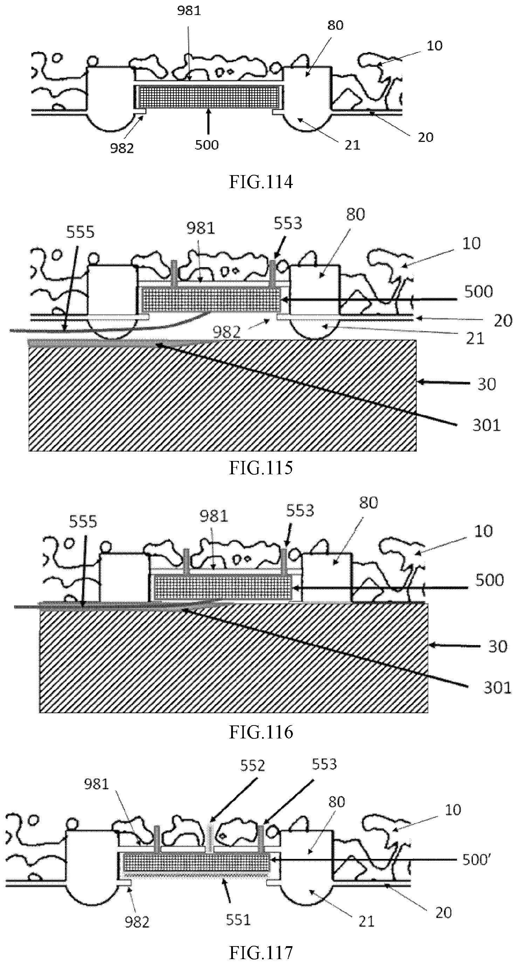

FIGS. 114 to 120 correspond to Embodiment IX, where.
FIG. 114 is a schematic diagram of the sensor set in the second pocket structure;
FIGS. 115 and 116 are two schematic diagrams of the arrangement of the sensor wire;
FIG. 117 is a schematic diagram of the sensor arranged with the wireless charging module;

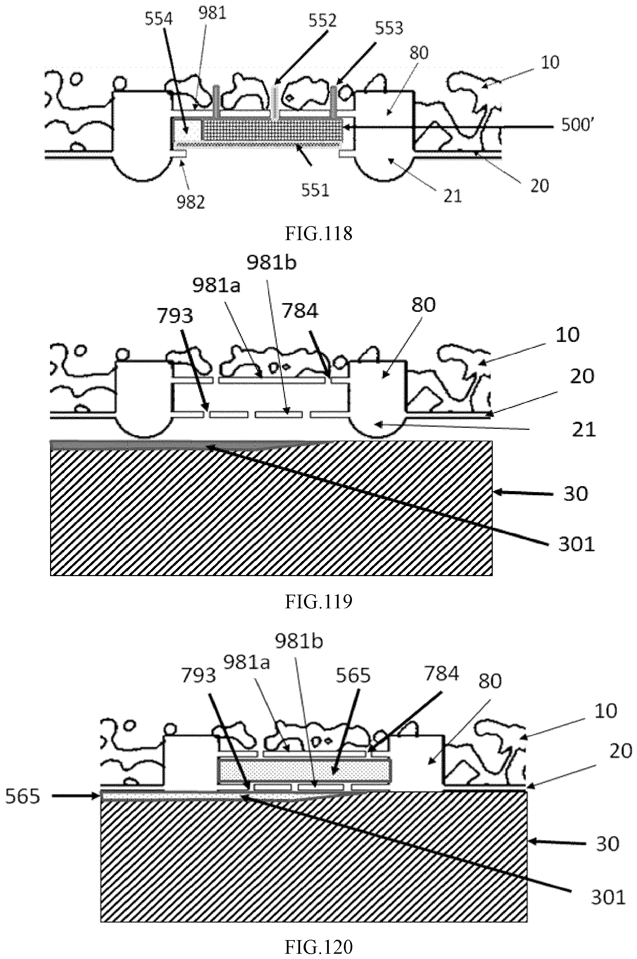

FIG. 118 is a schematic diagram of the sensor arranged with the wireless charging module and the electromagnetic signal trigger switch.
FIGS. 119 to 124 correspond to Embodiment X, where,
FIGS. 119 and 120 are schematic diagrams of the substrate opening grooves connected to the through holes opened by the coupling part below, with a gap between the substrate and the body of the intermediate in FIG. 119 and without a gap between the substrate and the body of the intermediate in FIG. 120.

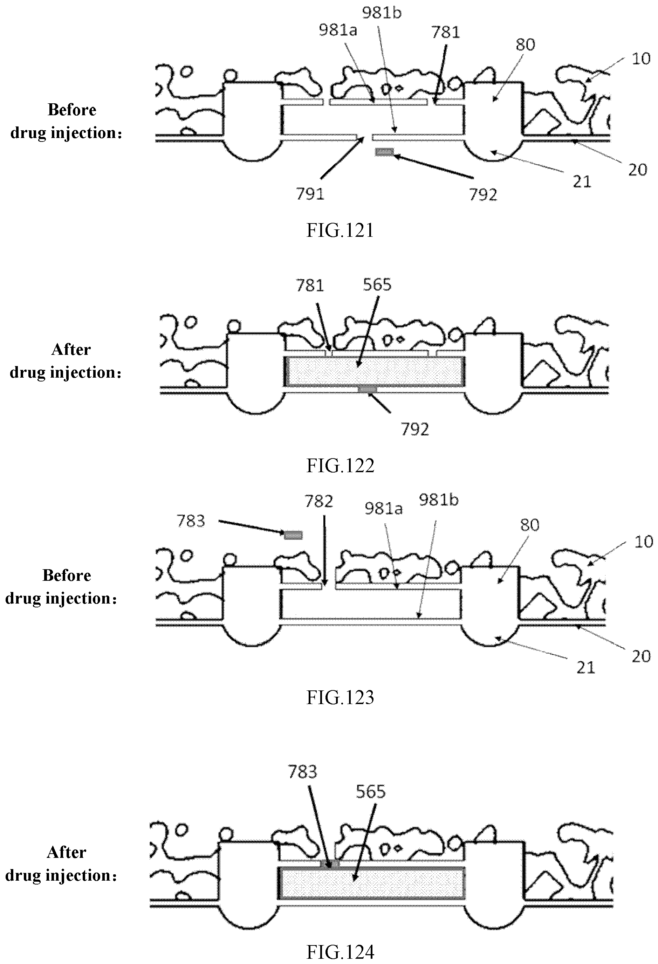

FIGS. 121 and 122 are schematic diagrams before and after drug injection based on a fourth pocket structure;
FIGS. 123 and 124 are schematic diagrams before and after drug injection based on a fifth pocket structure.

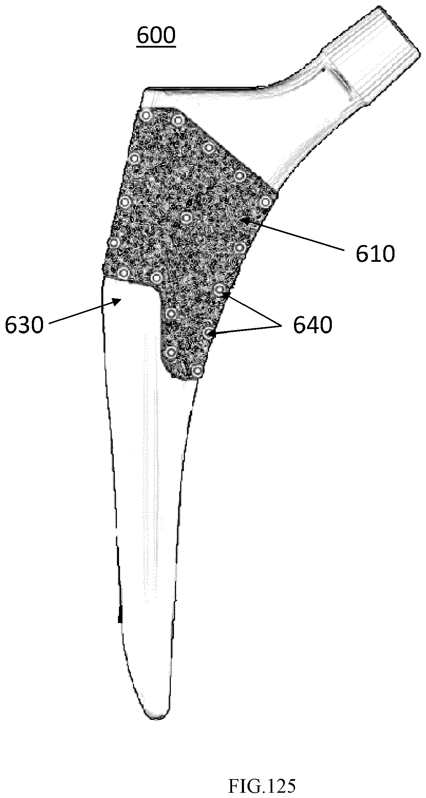

Figure 125 to Figure 155 correspond to Embodiment XI, where.
FIG. 125 is a front view of the femoral stem of the present invention, applying the first porous structure to the substrate;

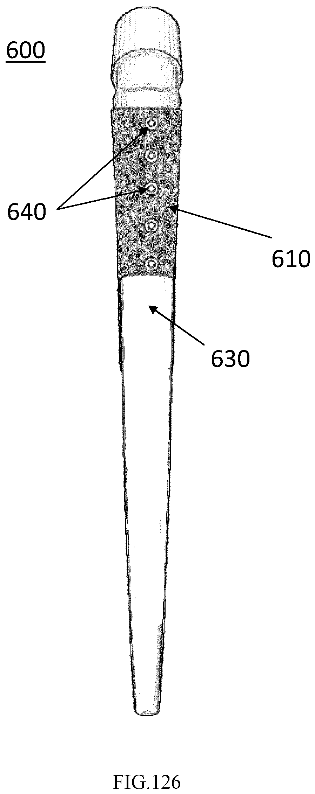

FIG. 126 is a side view of the femoral stem of the present invention applying the first porous structure to the substrate.

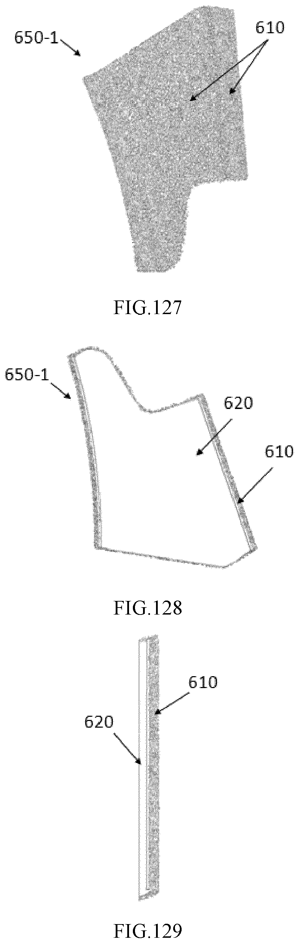

FIG. 127 is a front schematic view of one housing body for the femoral stem of the present invention;
FIG. 128 is a back schematic view of one housing body for the femoral stem of the present invention;
FIG. 129 is a partial cross-sectional schematic view of one housing body for the femoral stem of the present invention;

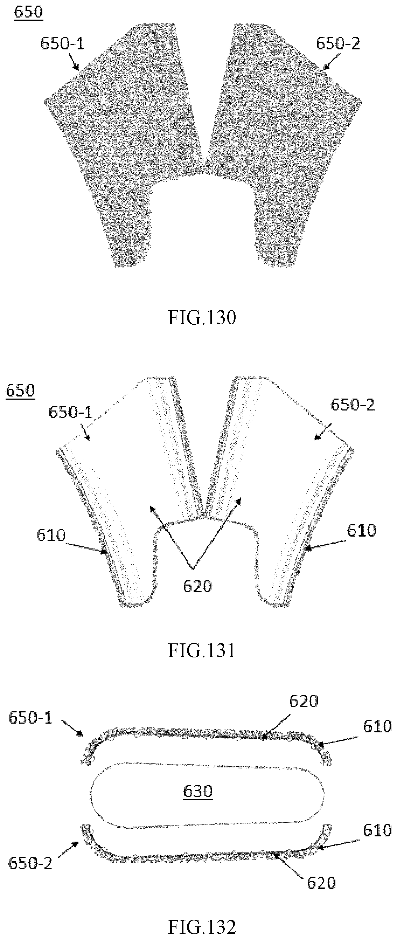

FIG. 130 is a front schematic view of body shells for the femoral stem of the present invention when they are not closed;
FIG. 131 is a back schematic view of Fig 130.
Fig. 132 is a schematic diagram of a transverse section of the present invention with two shell pieces wrapped around a femoral stem;

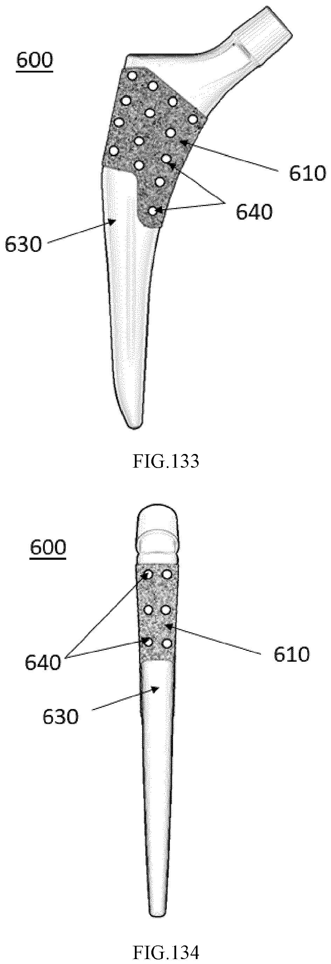

Fig. 133 is a front view of the stem body of the femoral stem of the present invention without a skirt line and with the composite body connected;
Fig. 134 is a side view of the stem body of the femoral stem of the present invention without a skirt line and with the composite body connected;

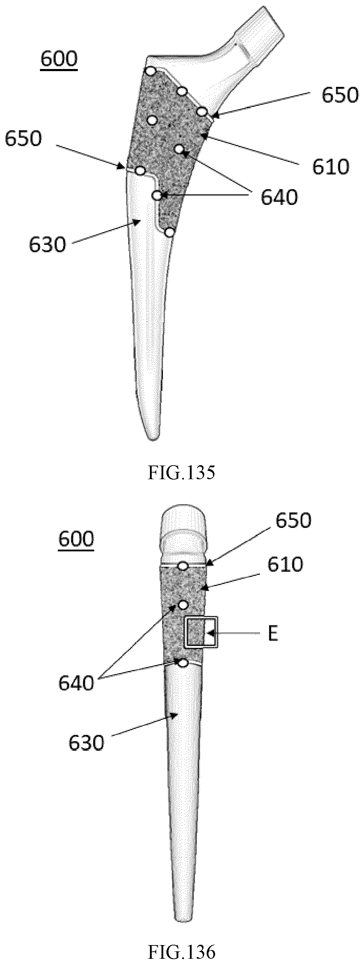

FIG. 135 is a front view of the stem body of the femoral stem of the present invention having a skirt line and with the composite body connected.
FIG. 136 is a side view of the stem body of the femoral stem of the present invention having a skirt line with and with the composite body connected;

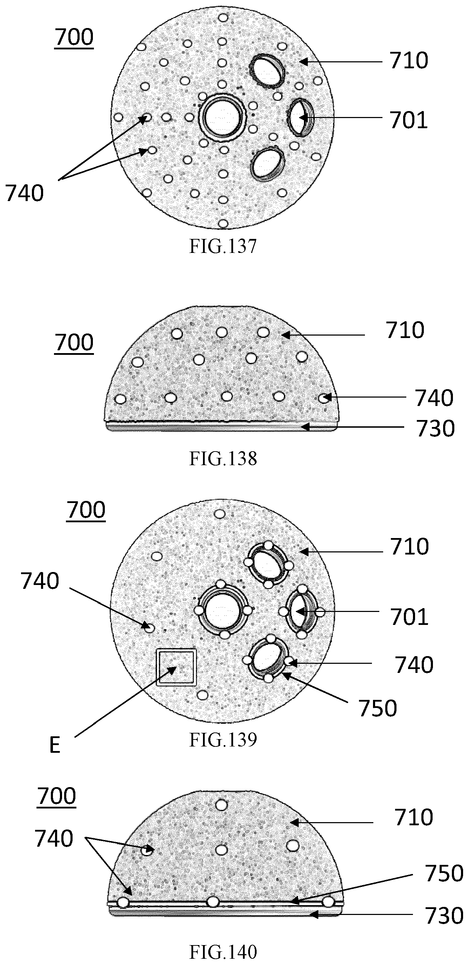

FIG. 137 is a front view of the cup body of the acetabular cup of the present invention without a skirt line and with non-divided composite body connected;
FIG. 138 is a side view of the cup body of the acetabular cup of the present invention without a skirt line and with non-divided composite body connected;
FIG. 139 is a front view of the cup body of the acetabular cup of the present invention having a skirt line and with non-divided composite body connected;
FIG. 140 is a side view of the cup body of the acetabular cup of the present invention having a skirt line and non-divided composite body connected;

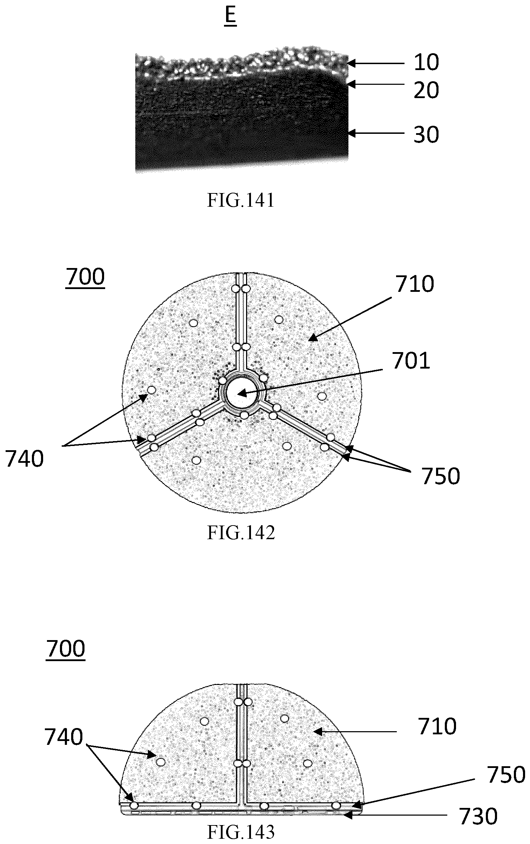

FIG. 141 is a schematic diagram of the physical substrate and composite body the connected structure appliwire to the prosthetic implant shown in FIGS. 136, 139 and 149;
FIG. 142 is a front view of the cup body of the acetabular cup of the present invention having a skirt line and with zonally connected composite body;
FIG. 143 is a side view of the cup body of the acetabular cup of the present invention with a skirt line and with zonally connected composite body;

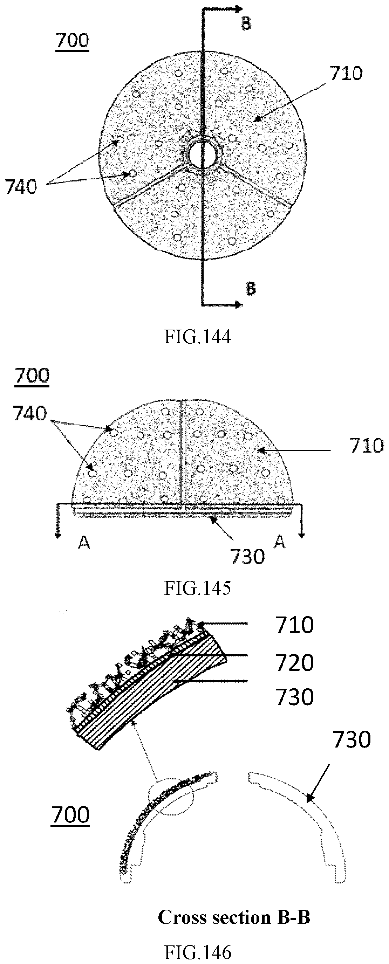

FIG. 144 is a front view of the cup body of the acetabular cup of the present invention without a skirt line and with zonally connected composite body;
FIG. 145 is a side view of the cup body of the acetabular cup of the present invention without a skirt line and with zonally connected composite body;
FIG. 146 is a B-B cross section of the acetabular cup prosthetic implant of the present invention in FIG. 144 and an enlarged schematic view thereof;

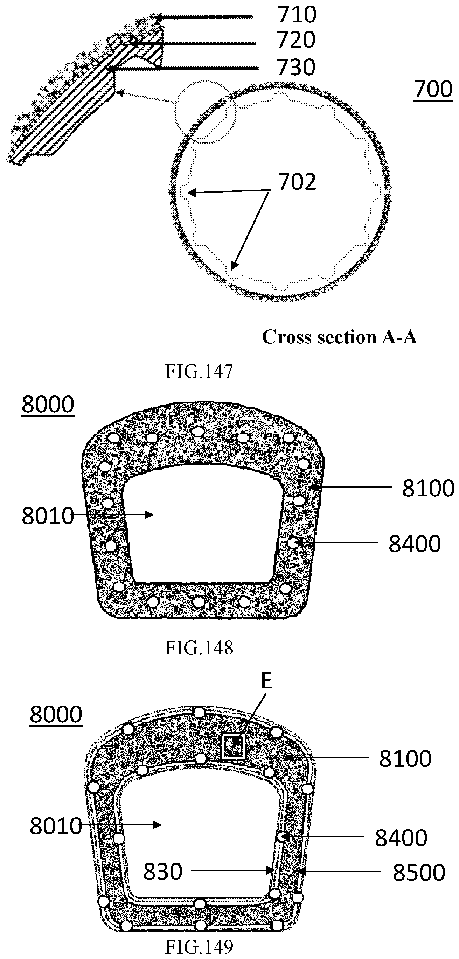

FIG. 147 is a schematic diagram of the acetabular cup prosthetic implant of the present invention in section A-A in FIG. 145 and an enlarged schematic view thereof;
FIG. 148 is a schematic diagram of the fusion cage of the present invention without skirt line and with the composite connected;
FIG. 149 is a schematic diagram of the fusion cage of the present invention with skirt line and with the composite connected;

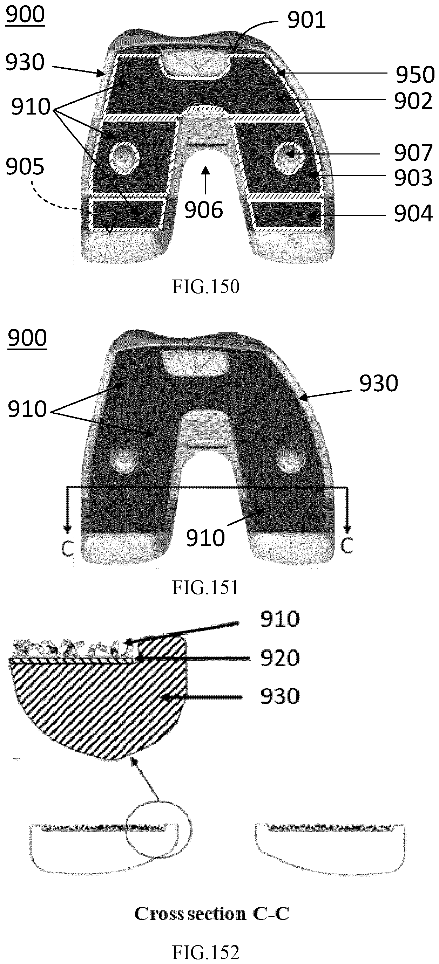

FIG. 150 is a schematic diagram of the femoral condyle body of the present invention with skirt line and with zonally connected composite body;
FIG. 151 is a schematic view of the femoral condyle body of the present invention without a skirt line and with a zonally connected composite.
FIG. 152 is a schematic view of the femoral condylar prosthetic implant of the present invention in cross section C-C in FIG. 151 and an enlarged schematic view thereof;

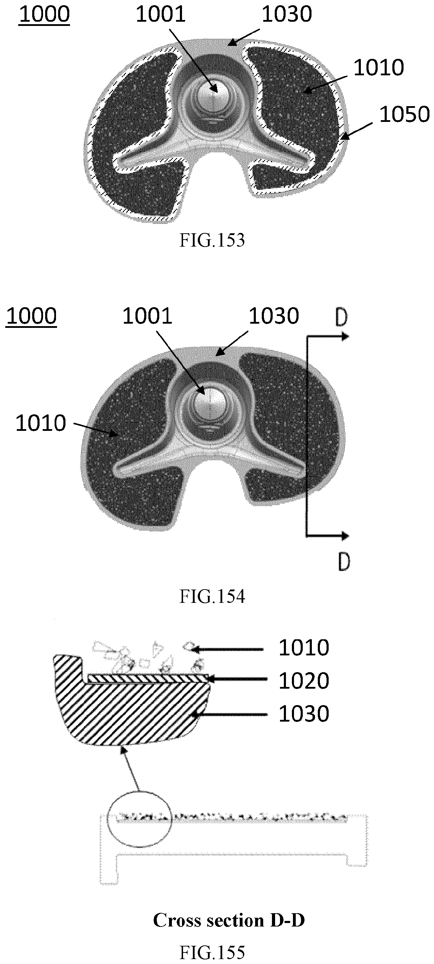

FIG. 153 is a schematic diagram of the tibial tray body of the present invention with a skirt line and zonally connected composite;
FIG. 154 is a schematic diagram of the tibial tray body of the present invention without a skirt line and zonally connected composite;
FIG. 155 is a schematic diagram of the tibial tray prosthetic implant of the present invention in D-D section in FIG. 154 and an enlarged schematic view thereof;

### Detailed Descriptions

The present invention provides a connected structure comprising a substrate, an intermediate, and a first porous structure. Said intermediate comprises: an insertion portion and/or an interlayer; at least a portion of the structure of the insertion portion, disposed within the first porous structure; and at least a portion of the structure of the interlayer, disposed between the first porous structure and the substrate.

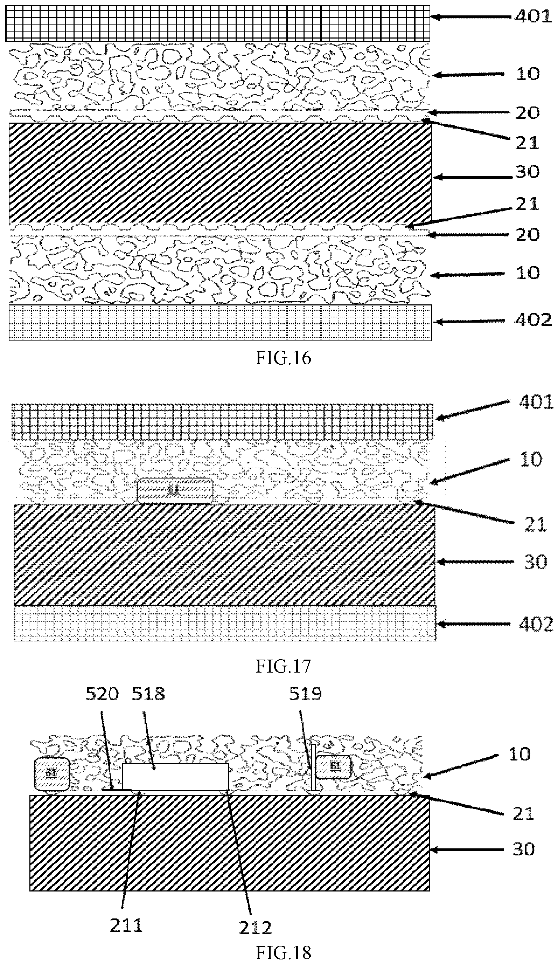

Taking Figures 2, 3, 17, 34, 52, 54 and 58 as examples, Figures 2 and 3 show a structure with an interlayer between the first porous structure 10 and the substrate 30; the interlayer of Figure 2 is the intermediate body 20; the interlayer of Figure 3 contains the intermediate body 20 and the raised structure 21 (Figure 4 is a schematic arrangement of the raised structure); the interlayer of Figure 17 contains the raised structure 21; FIG. 34 shows an example of a support part 801 as an insertion portion; FIG. 52 shows an intermediate containing both a support part 81, an intermediate body 20, and raised structures 21, FIG. 54 shows an intermediate containing both a support part 81 and raised structures 21, and FIG. 58 shows an intermediate containing both a support part 82 and an intermediate body 20, the description herein is not intended as a limitation on the respective state of the insertion and/or interlayers or their fit. The description herein is not intended as a limitation on the respective states of the insertion and/or interlayer portions or their fit with each other, and other examples will be specified below.

In the connected structure of the present invention, at least a portion of the first porous structure 10 and the intermediate are pre-connected to form a composite. Exemplarily, the composite body is connected to the substrate 30 by a connection between the intermediate and/or the first porous structure 10 and the substrate 30. In some products formed based on the above connected structure, at least a portion of the surface of the composite, such as at least a portion of the surface of the first porous structure 10, may be exposed to become the outer surface of the product (as shown in FIG. 2 where the top surface of the first porous structure 10 is exposed; however, what may be exposed is not limited to the surface in that direction, nor is it limited to the first porous structure 10).
wherein the first porous structure 10 is pre-connected to at least a portion of the intermediate to form a composite; or, wherein the first porous structure 10 is a one-step formed structure with at least a portion of the intermediate, such as achieved by a 3D printing additive manufacturing process, or a vapor phase precipitation process, or sintering, etc. Wherein, the timing or manner in which the different portions contained in the intermediate (not limited to the described interlayer and insertion portions), are pre-connected to the first porous structure 10 as a composite, may be the same or different.

In the example where the timing or manner of pre-bonding is different, the interlayer is integrally formed with the first porous structure 10; and the insertion portion is later provided into the first porous structure 10 to connect or contact with the first porous structure 10 and/or the interlayer; thus forming a composite body that is then connected to the substrate 30. For example, the insertion portion is integrally formed with the first porous structure 10, and the interlayer is later fixedly connected to the first porous structure 10 by other means, thereby forming a composite body that is then connected to the substrate 30.

In some examples, after the composite is securely connected to the substrate 30, a portion of the first porous structure 10, and/or a portion of the intermediate can be removed, thereby extending the scenario for subsequent applications.

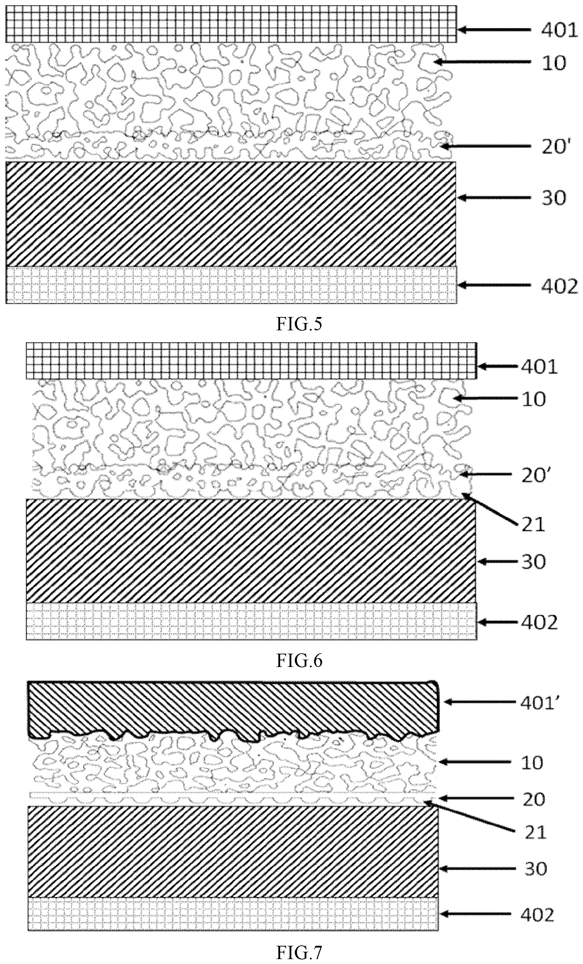

Preferably, the solid volume fractions of the intermediates are higher than the solid volume fraction of the first porous structure 10. For example, the intermediate may be solid object or may be a porous structure (referred to as the second porous structure 20', as shown in FIGS. 5 and 6). The first porous structure 10 and the second porous structure 20' may be clearly demarcated, e.g., combined in separate parts; or, alternatively, they may not be clearly demarcated and formed in some connected state, e.g., the solid volume fraction are layered or graded in the same porous structure, where the exposed, less dense portion subsequently corresponds to the first porous structure 10 and the more dense part connected to the substrate 30 corresponds to the second porous structure 20'.

For example, the intermediate body 20 shown in FIGS. 2 and 3 is solid object and has a relatively distinct interface with the first porous structure 10; the intermediate body shown in FIGS. 5 and 6 is formed using a second porous structure 20', which is at least somewhat connected to the first porous structure 10 at the interface between the two, or the two are originally formed in one piece; FIGS. 3 and 6 show examples of a solid object intermediate body 20 and a second porous structure 20' connected to raised structures 21, respectively, where the raised structure 21 and the second porous structure 20' of FIG. 6 are also connected to each other. The intermediate body 20 can be replaced with the intermediate body using the second porous structure 20', and will not be described below.

The porous structure, as described above, comprises a multitude of staggered struts, between which a number of multi-directional interconnected, regularly or irregularly shaped pores are formed. When the intermediate body is composed using a second porous structure 20', said second porous structure 20' has a higher solid volume fraction than the first porous structure 10, as shown by the thicker and/or less porous struts in the second porous structure 20'.

In different examples, the solid volume fraction of the insertion and the interlayer of the intermediate may be the same or different, however, the solid volume fraction of both parts are higher than the solid volume fraction of the first porous structure 10. In the preferred example, the first porous structure 10 and the intermediate are both made of metallic materials.

The solid volume fraction of the substrate 30 is higher than the solid volume fraction of the first porous structure 10. Preferably, the substrate 30 is solid object, which facilitates the overall strength of the connected structure. The example substrate 30 is made of a metallic material, formed by various means such as forging, casting, powder metallurgy or metal powder injection molding, and can be subjected to various mechanical processes.

In some examples, a porous structure (referred to as a third porous structure) may be used for the substrate 30, for instance, the substrate 30 being made wholly of a third porous structure, or having certain surfaces pre-made or pre-set with a third porous structure to be in contact with the first porous structure 10 and/or the intermediate. The third porous structure used in each of the above examples for substrate 30 has a higher solid volume fraction than the solid volume fraction of the first porous structure 10, as evidenced by thicker struts and/or lower porosity of the third porous structure. The solid volume fraction of the third porous structure can be the same as or different from the solid volume fraction of the second porous structure 20' used for the intermediate, preferably the solid volume fraction of the third porous structure being higher.

Exemplarily, both the composite and the substrate 30 are made of conductive materials, and the two are effectively bonded to each other by welding (e.g., laser welding, resistance welding, etc.). Preferably, the substrate 30 and the composite body (e.g., the part of the composite body corresponding to the welding interface) are made of materials with the same or similar melting points. The symbol 40 schematically indicates a welded connection between the two (as in Figure 2), with the position of the weld freely chosen or adjusted according to the needs of the application. Without limitation, in some examples, certain parts between the composite body and the substrate 30 are connected individually by adhesive bonding, by using connectors (screws, etc.), or by various other means of connection, or as an adjunct to a welded connection in these ways, which are not described herein.

The application of laser welding in the present invention is to cause a focused laser beam to pass through the first porous structure 10 and weld the interlayer of the intermediate to the substrate 30 at each welding point location, respectively. The application of resistance welding in the present invention is to press two metal workpieces to be welded between two electrodes, form a current circuit between these two electrodes of different polarity so that the current flows through the two workpieces, generating resistance heat in the contact areas and adjacent areas of both surfaces, while leaving said contact areas and adjacent areas in a molten or plastic state, and thus effectively bonding the two workpieces. Wherein, the resistance heat Q is proportional to IR2, R is the contact resistance, I is the current through the workpiece; contact resistance is the resistance generated by the current between two separate workpieces when they are in contact; it can be seen that the greater the current, the greater the value of the resistance heat; the greater the contact resistance, the greater the value of the resistance heat; this allows the contact areas and adjacent areas of the two workpieces to reach a molten or plastic state more quickly and to bond with each other more firmly.

When the present invention uses resistance welding, the electrodes can be rigid, with a fixed form, in any shape, such as plate (flat or curved, etc.), block, column, etc.; for example, the electrodes 401, 402 illustrated in Figure 3 are large flat electrodes that can cover most of the top surface of the first porous structure 10 at the same time; Figure 59 illustrates an electrode segment 405 in contact with a conductive support part. Alternatively, the electrode is flexible, a flexible body that can be deformed to some extent (Figure 7 shows an electrode 401' made of a flexible body), the shape of the flexible body and the area covered are not limited (e.g., in the form of a thin film). The electrode may be in conductive contact with the first porous structure 10 and/or the exposed surfaces of the intermediate, which may correspond to one or more directions. Exemplarily, electrode 401 in FIG. 3 is in direct contact with the top surface of first porous structure 10; electrode 406 as in FIG. 62 is in direct contact with the sides of first porous structure 10 and intermediate body 20; electrode 401 as in FIG. 63 is in direct contact with the top surface of first porous structure 10, while electrodes 407 and 408 are in direct contact with the sides of first porous structure 10 and intermediate body 20.

In this regard, the flexible electrode 401', as shown in Figure 7, may, in addition to covering the exposed surface of the first porous structure 10, have a portion that has some extension part towards the interior of the first porous structure 10 through the pores of the surface, thus increasing the conductive contact area of the electrode 401' with the first porous structure 10, or allowing the electrode further contacts the intermediate (not shown in the figures).

In some examples, the electrode segment 409 may be inserted into the first porous structure 10 to be in conductive contact with the first porous structure 10 and/or with the intermediate (as in FIG. 64, FIG. 66 in direct contact with the intermediate body 20); alternatively, the electrode segment 409 may also be inserted into the intermediate to be in direct conductive contact with the intermediate (as in FIG. 67 in which the electrode segment 409 is inserted directly into the recess on the support parts 931).. For example, a plurality of electrode segments connected to the same electrode of the same polarity are set up to weld at multiple positions simultaneously; with the plurality of electrode segments powered independently, or they can be connected in parallel with each other; or, only one electrode segment corresponds to an electrode of the same polarity, and the electrode segment is moved to each welding position separately.

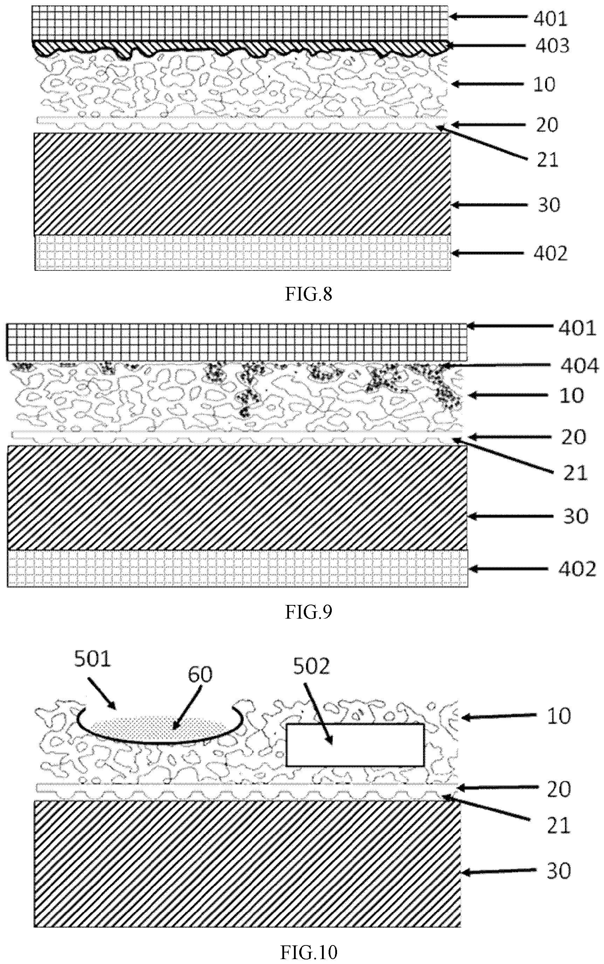

In addition, between the electrode 401 and the first porous structure 10, a variety of conductive media may be further provided. For example, the conductive medium is in various regular or irregular forms such as powder, foil, filaments, microspheres, particles, etc. (indicated schematically by the symbol 404 of FIG. 9), or in a thin film-like flexible body (indicated schematically by the symbol 403 of FIG. 9), or in various rigid structures (e.g., a mesh, which can be covered on the top surface of the first porous structure 10; e.g., a conductive column, with one end touching the large flat electrode and The other end is inserted into the first porous structure 10 or a conductive support part, interlayer, etc.); or, the conductive medium is a molten substance with conductive properties and injectable, and may also be a sprayable conductive medium, etc.

Compared with the original form in which the electrode 401 is in direct contact with the struts protruding from the surface of the first porous structure 10 (Fig. 3), the conductive medium, exemplified by Figs. 8 and 9, prevents the electrode 401 from coming into direct contact with the first porous structure 10, prevents the pressure or excessive resistance heat applied to the electrode 401 from acting directly on the first porous structure 10 and causing damage (e.g., denting, darkening, pore space reduction, etc.), effectively protecting the surface of the first porous structure 10. The conductive medium 403 or 404 can also compensate for the irregular shape of the surface of the first porous structure 10 in order to make the surface of the first porous structure 10 receive a more balanced current from the electrodes 404; wherein the conductive medium 403 or 404 dispersed on the surface of the first porous structure 10 makes the distribution of pressure or heat more uniform, increasing the conductive contact area on this side (illustrated as the top surface) and reducing the contact resistance and the resulting resistive heat on this side, protecting the surface of the first porous structure 10.

In some examples, see Figure 9, the conductive medium can also temporarily increase the solid volume fraction of the first porous structure 10 (filling site) by filling a portion of the pores (or further touching the intermediate) inward from the surface of the first porous structure 10, thus allowing the slightly weaker conductivity at the pores to be improved, increasing the current conduction in the overall circuit and improving the welding bonding efficiency.

Given that the solid volume fraction of the intermediate is higher than that of the first porous structure 10, the electrical conductivity is better, and that the intermediate may not be on the surface of the connected structure, the effect of surface damage on the intermediate due to electrode pressure or resistive heat is relatively small, thus, the above-mentioned conductive medium can be similarly provided between the electrode and the intermediate, or the two can be in direct contact without the conductive medium.

When selecting the material of the conductive medium, it is possible to consider whether the conductive properties are good, such as the use of copper, tin, etc., or to consider the use of materials with the same or similar properties as the first porous structure 10 or intermediate, such as the material used in the connected structure of a prosthetic implant, such as titanium, etc.; or, the similar properties refer to the similar solid volume fraction or conductive properties, etc. Alternatively, the material of the conductive medium can be considered in light of whether it is easy to remove after the current conduction is completed or whether it will affect subsequent use. It is necessary to remove at least a large part of the conductive medium after the completion of welding to leave the pores of the first porous structure 10 open, for example, by emptying the conductive medium out of the pores or by removing the conductive medium with physical or chemical means based on the different states of the conductive medium and the first porous structure 10 (e.g., the melting point of the molten-like substance of the conductive medium can be lower than the melting point of the first porous structure 10); or allowing a small amount of conductive medium to adhere to the first porous structure 10 or intermediate, if doing so does not affect the subsequent use of the connected structure, such as when the conductive medium and the first porous structure 10 or intermediate material is made of the same or similar materials, and it is not prone to peel off in subsequent use.

With resistance welding, two composite bodies can be welded onto two opposite sides of the substrate 30 simultaneously or successively (as in Figure 16). If welded simultaneously, a current circuit is formed between the two polarities of electrodes 401, 402, and the composite body on the first side, the substrate 30, and the composite body on the second side are connected into this current circuit (the welding interface between the substrate and both composite bodies are in close contact), and the electrodes of each polarity can be used in one or more of the above-mentioned ways to make conductive contact with the composite body on the corresponding side (the composite body of this FIG. 16, comprising the first porous structure 10, intermediate body 20 and raised structure 21, for example, can be replaced with any other examples of composite body of the present invention; in this case, electrodes 403, 404 contact the first porous structure 10 of the two composites, respectively).

If two composites are connected successively, when the first composite is connected on the first side of the substrate 30, a current circuit is formed between the electrodes of the two polarities, into which the first composite is connected to the substrate 30 (the welded interface between the two is in close contact), one polar electrode may be in conductive contact with the first composite using one or more of the above, and the other polar electrode may similarly be in one or more of the above conductive contact with the substrate 30. Then, when the second composite is connected to the second side of the substrate 30, a current circuit is formed between the two polar electrodes, into which the substrate 30, the second composite, which has been connected to the first composite, is connected (the second side of the substrate 30 is in close contact with the weld interface of the second composite). The electrodes of each polarity may be in conductive contact with the corresponding side of the composite using one or more of the above; alternatively, one polarity may be in conductive contact with the second composite using one or more of the above, and the other polarity may be similarly in conductive contact with the substrate 30 using one or more of the above (across the first side of the composite).

The said connected structure of the present invention is provided with one or more holding spaces in which to place the contained substance as desired. In different examples, the first porous structure 10, the intermediate, and the substrate 30, may be provided with holding spaces individually; or, the first porous structure 10 and the intermediate and/or the substrate 30, or the intermediate and the substrate 30, may be provided with holding spaces cooperatively.

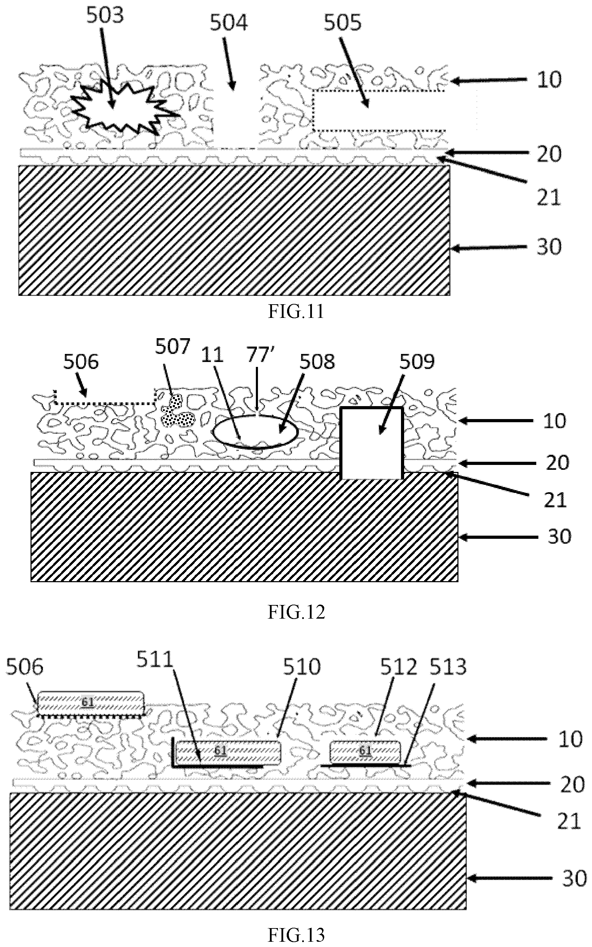

The present invention does not make specific limitations on the contained substances and can be decided according to the actual application. The shape size quantity, etc. of the holding space is not limited and is generally decided based on the actual use of the contained substance, the composite body, the substrate 30, the connected structure or its holding space and the contained substance, etc., such as considering the shape, type, size, etc. of the contained substance, considering the structure, connection part, overall strength, etc. of the composite body and/or the substrate 30, but not limited to these factors. The holding space may be formed before, during, or after the connection of the composite body to the substrate 30. The contained substance may be solid object, liquid, or gaseous. The contained substance may be in a more discrete state resembling a particle, powder; or it may be a solid object with a more stable form and structure (the volume of the solid object may be less than, equal to, or greater than the volume of the holding space; or, the solid object may be less than, equal to, or greater than the edge interface to which the holding space is fixed in a certain direction). As shown in Figure 13, the solid object 61 on the left side extends beyond the holding space 506 in which it is located; the solid object 61 in the middle extends beyond the edge interface 511 of the holding space 510 on its bottom side; and the other solid object 61 on the right side does not extend beyond the bottom edge interface 513 of the holding space 512.

The holding space may be relatively closed (e.g., holding spaces 502, 503 of FIGS. 10, 11), may not be closed, such as where the edge interfaces are interrupted, or may be provided with one or more channels or openings connecting to the outside of the holding space (e.g., holding spaces 501, 504, 505, 506, 508, etc. of FIGS. 10, 11, 12); said channels or openings that may be used to place or release the contained substances, and may also be used for information and/or material transfer through contained substances. Depending on the states and usages of the contained substances, etc., some channels or openings may be open at all times, and some channels or openings may be closed after use; the channels or openings may be permanently closed and may generally remain closed for subsequent use of the connected structure; or, the channels or openings may be temporarily closed until the next use.

And depending on differences in the structure of the holding space itself, its location, the state and usage of the contained substances, etc., the said holding space, or its channel/opening, may connect to the first porous structure 10, intermediate, substrate 30, etc., or to the external open space through these components, without any specific limitation in this regard herein. For example, the opening 77' of the holding space 508 shown in FIG. 12 connects to the pores of the first porous structure 10, and thus to the outside of the exposed top surface of the composite body.

The parts used to close the channels or openings are referred to as closure bodies(e.g., closure bodies93, 94 of FIG. 40, closing the openings of holding spaces 440, 441). The closure bodies can be made additionally from the first porous structure 10 or intermediate or substrate 30 of the same material. Alternatively, the first porous structure 10 or intermediate body 20 or substrate 30 is cut at a certain place to form a channel or opening, and then the cut-off part is used as a closure bodies. Alternatively, depending on the location and function of the channel or opening, the form of the contained substances, etc., a suitable material and form can be selected and the closure bodies can be designed and manufactured. Alternatively, the pre-determined location of a part of the connected structure can be used as a channel or opening, and the channel or opening can be closed by installing the part in place after placing the contained substances (e.g., the gap 105 in FIG. 41 is connected to the opening of the holding space 442, and the support part 826 is installed in a position corresponding to the gap 105, and after placing the contained substances in the holding space 442, the support part 826 is installed in place and the opening is closed).

There may be no first porous structure 10, or intermediate, or substrate 30 in the holding space, or, in some examples, a portion of the first porous structure 10 or intermediate or substrate 30 may be allowed to remain in the holding space if it does not interfere with the placement or use of the contained substances (e.g., a portion of the first porous structure 10 is left in the holding space 508 of FIG. 12)

In some examples, the holding space is formed with edge interfaces that clearly delineate the boundaries of the holding space in all or at least some of its directions. Alternatively, the holding space may not have a clearly delineated edge interface. Said edge interface may be used to enclose the holding space, or may be used to carry or attach the contained substance, or may be used to provide other components for securing the contained substance, or may be used to attach the holding space to the first porous structure 10 or intermediate or substrate 30, or may provide access or openings to the outside at the edge interface, and is not limited to these examples.

In some examples, certain portions of the first porous structure 10 or intermediate or substrate 30 may also serve as the edge interface of the holding space in a certain direction (e.g.,

Figure 48 with a side of the support part 820 as the edge interface). Sometimes these parts of the first porous structure 10 or intermediate or substrate 30, in the as-made state, can serve directly as the edge interface of the holding space, and sometimes additional processings of these parts are required. In some examples, the edge interface of the contained substances in some directions coincides with the edge interface of the holding space in some directions.

Alternatively, in some examples, the edge interfaces of the holding space along all or some directions are component structures additionally provided either before the composite is connected to the substrate 30, such as by pre-connection or one-step forming, with the first porous structure 10 or intermediate or substrate 30; or during or after the connection of the composite to the substrate 30.

A plurality of Embodiments of the present invention are described hereinafter in connection with the attached drawings. The orientation described below (e.g., up, down, left, right, etc.) is illustrated corresponding to the orientation of each component in the attached drawings and is not intended as a limitation on the orientation of the connected structure when it is actually used.

### Embodiment I

In this Embodiment, the first porous structure 10 is used to design the holding space and place the contained substance.

In conjunction with FIGS. 10 to 14, the holding space is formed at the first porous structure 10. The shape of the holding space may be regular or irregular. The edge interface of the holding space can be regular or irregular; the state of the edge interface in different directions of the same holding space can be the same or different; the state of the edge interface, for example, refers to whether it is closed, the shape or nature of the surface, for example, whether it contacts the contained substance, whether other additional component structurest are provided and so on, but is not limited to these examples.

One or more directions around the periphery of the holding space is adjacent to the first porous structure 10 or other components of the connected structure, or connected to the external open space. The holding space may contain a portion of the first porous structure 10 and may just contain spaces without containing first porous structure 10.

The holding space may be formed during the process of manufacturing the first porous structure 10, such as one-step forming processes, in which the first porous structure 10 is pre-formed; or, the holding space may be formed at a later stage by removing a portion of the already manufactured first porous structure 10, such as by various machining means such as cutting, gouging, and the like. Alternatively, for example, the first porous structure 10 is not pre-formed into the holding space, but is compressed to a certain extent by an external force at the area where the contained substances will be placed, so that a recess is formed wherein to function as the holding space.

Exemplarily, the holding spaces 501, 502, 503, 505 shown in FIGS. 10 and 11 have edge interfaces, and the holding space 501 is open near the top of the first porous structure 10 with openings connecting to the exposed top surface; schematically, a contained substance 60 is placed within this holding space 501. The holding space 502 has edge interfaces in all directions; the holding space 503 schematically represents an irregular edge interface whose respective openings or enclosure bodiesare not shown in the Figure. The holding space 505 has openings on the sides of the first porous structure 10, and the edge interfaces in the other directions of the holding space 505 may be open (indicated by dashed lines in the Figures). The holding space 504 is a gap opened at the first porous structure 10, extending from the top surface of the first porous structure 10 to the top surface of the interlayer intermediate body 20; the lateral direction of this holding space 505 is surrounded by the first porous structure 10 located next to the gap and no additional edge interface is formed.

Exemplarily, the holding space 506 shown in FIG. 12 is a recess formed on the surface of the first porous structure 10 for holding the solid object 61, with a portion of the solid object 61 set in the recess and the other portion remaining outside the recess. The holding space 508 itself is opened within the first porous structure 10, with an opening 77' connecting to the pores of the first porous structure 10 and thus to the exterior from the top surface of the first porous structure 10; a portion 11 of the first porous structure 10, such as the ends of the struts, remains within the confinements of this holding space 508.

Alternatively, one of the larger pores within the first porous structure 10 is used directly as a holding space (see holding space 507 of FIG. 12, which is schematically filled with granular material); or, some of the smaller pores within the first porous structure 10 are connected to each other (the struts between the pores can be retained or removed) to form a larger pores that are used as a holding space (not shown in FIG. shown). This can be achieved by pre-designing the shape and size of one or some of the pores, or by re-processing of one or some of the pores at a later stage.

The first porous structure 10 around the holding space may apply loads on or be connected to the contained substance. Loading on the contained substance means bearing the contained substance from at least one direction, for example, for example carrying the bottom of the contained substance from below, but not limited thereto. Connection to the contained substance, depending on the form of the contained substance, may refer to connection to the contained substance itself or to the external capsule of the contained substance (e.g., container, shell, etc.); said connection, for example, is adhesive bonding, welding, use of matching connectors, position-limiting structures, etc., without limitation. The following involves other parts of the structure (intermediates, substrate 30, etc.) to the contained substance and its loading or connecting to the contained substance have similar meanings , and we will not repeat herein.

Some of the contained substances are relatively stable entities 61 in structure, form, etc., when placed and/or used, and can be tightly fitted with the first porous structure 10 on its periphery (at least one direction on both sides) so that the whole substance is set in the resulting holding space. In this example, the width of the contained substance is equal to or slightly larger than the gap between the left and right sides of the first porous structure 10 and the contained substance is fixed in close contact with at least the left and right sides the first porous structure 10 after insertion. If the outer capsule of the contained substance is a stable solid object as described above, it can be tightly fitted to the first porous structure 10 around it in a similar manner.

The holding space at the first porous structure 10 can be provided with channels or openings along some directions (e.g., top or side) that lead to external open spaces (e.g., the holding spaces 501, 504, 505 of FIGS. 10 and 11 all have openings to the external space); the holding space can also be provided with channels or openings along some directions that lead to the intermediate and/or substrate 30, examples of which will be described below . The first porous structure 10 near the channel or opening, or the first porous structure 10 in other directions, or the channel or opening that is closed, may load, or be connected to the contained substance.

The top surface of the intermediate body 20 shown in FIG. 11 serves as the edge interface at the bottom of the holding space 504; the holding space 509 shown in FIG. 12 is formed mostly within the first porous structure 10, and the bottom runs through the intermediate body 20 and the raised structure 21 of the interlayer and extends to a recess at the top surface of the substrate 30, and the bottom part of the substrate 30 within the holding space 509 becomes the edge interface of the bottom part of the holding space 509, and the holding space 509 forms additional edge interfaces in other directions (indicated by thick solid lines).

Exemplarily, the location of the first porous structure 10 adjacent to the holding space may form a position-limiting protrusion extending into the holding space in contact with the surface of the solid contained substances (or its enclosure bodies bodies) for position-limiting; the position-limiting protrusion may be arranged in one, two or more directions towards the solid object respectively. Alternatively, a number of position-limiting protrusions adjacent to one of the directions of the holding space may be simultaneously located on the same side of the solid object to restrict it; the position-limiting protrusions may be the ends of some struts of the first porous structure 10 itself located at the edge interface of the holding space, or they may be separately formed components (e.g., bumps, pins,, etc., with the same or different solid volume fraction as the first porous structure 10). Alternatively, the corresponding position on the surface of the holding space (or its enclosure bodies) is further formed with a position-limiting recess matching the position-limiting protrusion, said position-limiting protrusion being able to be set in the position-limiting recess accordingly. Another example is the formation of outwardly facing position-limiting protrusions on the surface of the contained substances (or its enclosure bodies), capable of being set in the pores of the first porous structure 10 and imposing position-limiting on the contained substances; the pores corresponding to the position-limiting protrusions may be kept in the same form as when the first porous structure 10 was made, or may be made to better match the shape and size of the position-limiting protrusions by later processing.

As shown in FIG. 13, the holding space 506 is formed using a recess at the first porous structure 10 (see FIG. 12) so that the solid object 61 is at least partially inserted; the holding spaces 510, 512 are constructed using two gaps formed within the first porous structure 10, with additional edge interfaces formed in both directions of the holding space 510, and the bottom edge interface 511 does not cover all of the bottom surface of the holding space 510. The edge interface 511 at the bottom does not cover the entire bottom surface of the holding space 510, and the edge interface 511 is smaller than the solid object 61 it carries, and the part of the solid object 61 that is not carried by the edge interface 511 is overhung and extends over the rest of the holding space 510. The holding space 512 has an edge interface 513 on one side that loads the solid object 61 placed into the holding space 512, and the solid object 61 does not extend beyond the edge interface 513 (no channel or opening through which to place the solid object 61 into its corresponding holding space 510 or 512 is shown in the Figures).

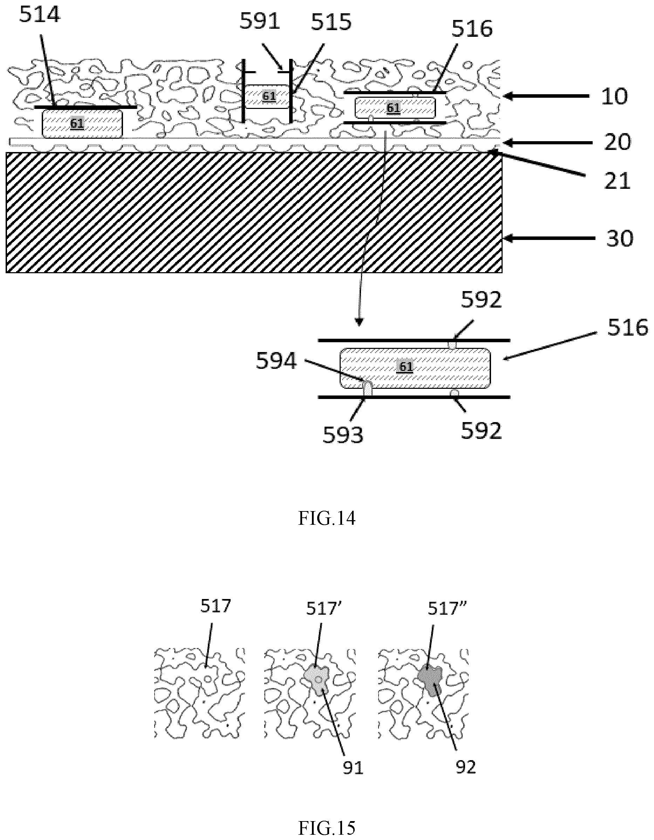

As shown in FIG. 14, holding spaces 514, 515, and 516 each contains a gap formed within the first porous structure 10; the top of the gap corresponding to holding space 514 forms an edge interface that can be used to attach the contained substance or to tightly fit solid object 61 to this edge interface above and to the substrate 30 below (located within the bottom of the gap) in a tight-fitting manner to implement fixation. The holding space 515 is formed on the left and right sides of the gap to hold the solid object 61 in a tight fit (the edge interface at the bottom of the gap is formed by a struts adjacent to the first porous structure 61); position-limiting protrusions 591 are formed on each side of the edge interface extending into the gap, which position-limiting the range of movement of the solid object 61 and prevent the solid object 61 from falling out through the opening above ( Assuming that the individual position-limiting protrusions 591 are wide enough, the position-limiting protrusions 591 on one side alone can also block the solid object 61 at the opening of the gap). The holding space 516 (see enlarged view) is formed as an edge interface on the upper and lower sides of the gap, with a position-limiting protrusion 593 on the lower edge interface and a position-limiting recess 594 in the corresponding location of the solid object 61 to match it; the upper and lower edge interfaces also correspondingly form a position-limiting protrusion 592 that closely contacts the solid object 61 from two directions respectively to position-limiting it, and the corresponding position-limiting protrusion 592 on the solid object 61 is not provided with a position-limiting recess. There is no position-limiting recess at the position of the position-limiting protrusion 592 on the solid object 61.

If the holding space itself has a stable solid object edge interface, the first porous structure 10 or other component nearby, etc., can secure the solid object edge interface of the holding space (not limited to carrying, connecting, fastening, or position-limiting) in a similar manner to the fixation of solid contained substance; and the solid object edge interface of the holding space can also be secured to the contained substance of the internally placed solid object or its capsule in a similar manner. The solid object edge interface of the holding space can also be secured to the solid contained substance or its enclosure bodies by similar means.

In some examples, the first porous structure 10 itself has pores that can serve as channels or openings of the holding space; alternatively, additional more regularly shaped channels or openings can be opened at the first porous structure 10.

Depending on the actual application, the various channels or openings in the holding space, as described above, may remain open or may be closed by enclosure bodies(on a short-term or long-term basis); for example, certain channels or openings are closed after placement of the contained substance into the holding space; or, certain channels or openings are pre-closed (and can be opened for subsequent use).

The solid volume fraction of the enclosure bodiesare designed according to the needs of the practical application. The closure bodies may be solid object; or, the closure bodies may be a porous structure (the solid volume fraction may be the same or different from the solid volume fraction of the first porous structure 10). The material of some of the enclosure bodiesor their solid volume fraction may be designed to allow for the channel of contained substance material through the enclosure bodies(e.g., to enter and exit the holding space).

The example closure bodies may be integrally formed with the first porous structure 10. It may also be provided later, for example, after the first porous structure 10 is formed into a composite with the intermediate, or after the composite is connected to the substrate 30, or after placement of the contained substance; for example, by injecting some molten substance (which may be injected with a polymeric material, or with a material identical or similar in nature to, but not limited to, the first porous structure 10 or the intermediate or the substrate 30), which solidifies to form the closure bodies, or, alternatively, the formed part is directly inserted or covered in a location adjacent to the first porous structure 10 within the holding space (if necessary, the inserted or covered part can be further connected to the adjacent first porous structure 10 or intermediate or substrate 30, or a plurality of the inserted or covered parts can be connected to each other). Exemplarily, the portion of the first porous structure 10 that was removed when the channel or opening was opened may also be placed back into the channel or opening as a closure bodies.

Given that the first porous structure 10 itself has numerous pores, it is also possible to use other parts of the connected structure (intermediate, substrate 30, etc.) or, alternatively, a separately provided component structure to close the edge interfaces, channels or openings of the holding space for the holding space formed therein.

In some examples, a number of formed parts and/or filling surfaces (of any shape and size as desired) are further provided at the first porous structure 10 adjacent to the holding space. In this case, the formed parts are externally made and placed to the corresponding locations within the connected structure. For example, suppose the edge interfaces of the holding spaces 514, 515, 516 shown in FIG. 14 are formed by some kind of formed wall plates, including the upper position-limiting protrusions 592, 593, etc., which can be integrally formed or pre-connected to the said wall plates, such that the wall plates are integrally inserted into the appropriate gap positions of the first porous structure 10.

By placing filler bodies, some neighboring struts of the first porous structure 10 are coupled to form a filler surface (combination of filler and struts). The left one of FIG. 15 illustrates a part of a pore 517 of the first porous structure 10 before it is filled, where the pore is open; the middle one illustrates that part of the pore 517' is filled by the filling body 91 and some struts ends still protrude from one side to the other side of the filling body 91; the right one illustrates that part of the pore 517 " is filled by another filler body 92 and within the corresponding range the ends of the struts are completely buried within the filler body 92.

Said forming component and/or filling surface may be used as a closure bodies, to close the channel or opening of the holding space. They may also be used to construct a part of the edge interface of the holding space: for example, to achieve boundary delimitation; or, to secure the contained substance (such as supporting, connecting, tight fitting or position-limiting, etc.) alone or in conjunction with other edge interfaces, or other components of the connected structure, without being limited to these specific cases. Said forming components or filling body of the filler surface may be formed as closure bodies, without being limited to such.

For example, a forming part or a filling surface may independently cover all or parts of the area corresponding to one side of the edge interface. Alternatively, a plurality of formed parts cooperating with each other cover all or part of the area corresponding to one side of the edge interface (the plurality of formed parts covering part of the area may be spaced out or close to each other without spacing). Alternatively, all or part of the area corresponding to the edge interface on one side is formed with a filling surface. For example, the forming part and the filling surface can be provided simultaneously, for example, cooperatively on the same side of the edge interface, or on edge interfaces of different directions.

If one or some directions of the formed part or filling surface, or its edge interface do not need to be completely closed, the channel or opening of the holding space can be allowed to be placed at the formed part or filling surface or at other locations of their edge interfaces; or, distributed between the forming parts (or filling surfaces) near the edge interface region; or, some of the pores of the first porous structure 10 are open at the filler surface (or in an uncovered area of the edge interface); or, materials of certain compositions (or solid volume fraction) through which the contained substances can pass may form said formed parts or filling surfaces.

The specifications of the holding space or contained substance in this Embodiment that are associated with the first porous structure 10 can all be applied in other Embodiments.

### Embodiment II

In this Embodiment, the holding space is designed and the contained substance is placed based on the cooperation of the first porous structure 10 with the interlayer of the intermediate, as shown in FIGS. 17 to 19. At least a portion of the structure of the interlayer of the intermediate, is located between the first porous structure 10 and the substrate 30.

Preferably, the connection between the interlayer of the intermediate and the substrate 30 is achieved with laser welding or resistance welding.

In some examples, the beam of laser welding or the electrode of resistance welding can directly contact the exposed surface of the interlayer of the intermediate itself from some directions; in some examples, the beam of laser welding or the electrode of resistance welding can pass through the first porous structure 10 itself or the gaps opened therein to reach the location of the interlayer and make direct contact with the interlayer (e.g., the beam of laser welding in FIGS. 71 to 73 energy can directly penetrate the first porous structure 10 to reach the position to be welded at the bottom 21 and periphery 22 of the intermediate body 20; as in FIG. 64, FIG. 66 the electrode segment 409 passes through the gap to contact the top surface of the intermediate body 20). Alternatively, in some examples of resistance welding, the electrode directly contacts the first porous structure 10 and/or the insertion of the intermediate, etc., and conducts current through the first porous structure 10 and/or the insertion to the interlayer (e.g., the first porous structure 10 of FIG. 3 contacts the electrode 401 and conducts current to the intermediate body 20 and raised structures 21; the support part 83 of FIG. 56 contacts the electrode 401 and conducts current to the intermediate body 20 and raised structures 21, as in FIG. 56).

As shown in Figure 18, the interlayer comprises raised structure 21 with bumps facing the substrate 30. Preferably, in the example where resistance welding is performed, the raised structure 21 can increase the contact resistance by reducing the contact area of the welding interface to generate more resistance heat and improve the welding efficiency and welding strength between the interlayer and the substrate 30.

As shown in FIG. 2, the interlayer can contain an intermediate body 20, which can be applied under laser welding or resistance welding or other connection methods. example is an intermediate body 20, as a layer, a sheet or a plate; intermediate body 20 is not limited to a flat surface, but can be curved or otherwise adjusted with the substrate 30 (surface shape, etc.) to make the intermediate body 20 and the substrate 30 fit better when connected. Can also be in the intermediate body 20 somewhere, such as to enhance the structural strength, adapt to the shape of the substrate 30, to facilitate the connection of the first porous structure 10 or substrate 30, to enhance the electrical conductivity, connectivity and other purposes to make local adjustments, such as increasing or decreasing the thickness of a certain place or other dimensions, such as adjusting a certain place for other shapes, such as making a certain place to form a regular or irregular hollow shapes, and so on.

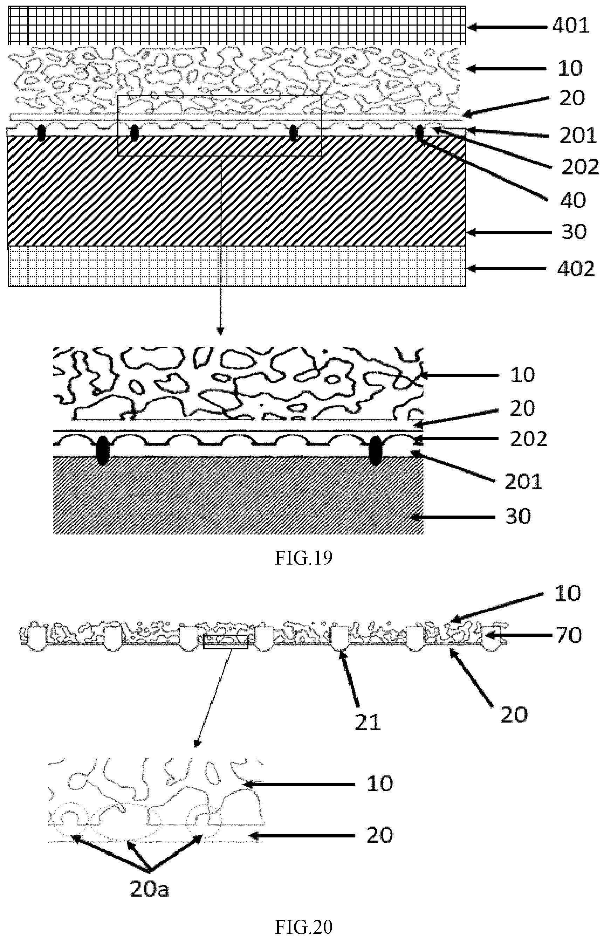

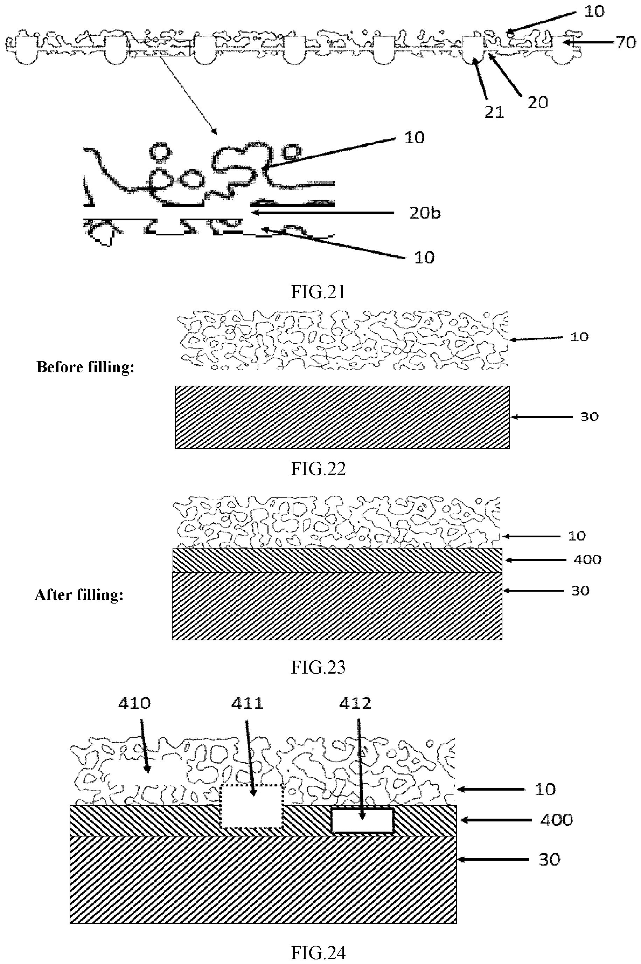

The intermediate body 20 and the first porous structure 10 shown in FIGS. 20 and 21 are partly connected to each other (symbol 20a schematically indicates the fusion of the top of the intermediate body 20 with the bottom of the first porous structure 10, and symbol 20b schematically indicates that both the top and the bottom of the intermediate body 20 are connected to the first porous structure 10, i.e., the intermediate 20 is arranged laterally near the bottom of the first porous structure Fig. 20 and Fig. 21 also contain both raised structures 21 of the intermediate (and the support part 80 of the insertion portion), and Fig. 20 and Fig. 21 can both connect the substrate 30 by raised structures 21, and Fig. 21 may also connect the substrate 30 by the part of the first porous structure 10 that is below the intermediate body 20.

The intermediate body 20 and the raised structure 21 can be provided separately or simultaneously. When the intermediate body 20 or the raised structure 21 is provided separately (FIG. 2, FIG. 17), the intermediate body 20 or the raised structure 21 is pre-bonded to the bottom surface (the surface near the side of the substrate 30) of the first porous structure 10. In the case of simultaneous setting, as shown in FIG. 3 and FIG. 5 to FIG. 14, raised structures 21 that is raised toward the substrate 30 can be formed on the bottom surface (surface near the side of the substrate 30) of the intermediate body 20; the top surface (surface away from the side of the substrate 30) of this intermediate body 20 is pre-bonded to the bottom surface of the first porous structure 10. As shown in FIG. 6, when the intermediate body is a second porous structure 20', the raised structure 21 is formed at the bottom of the second porous structure 20', and the two can have some fusion.

Without limiting the number, shape, or texture pattern formed on the surface of the first porous structure 10 or the intermediate body 20 by distributing a number of raised structures 21, etc., the present invention, FIG. 4 provides an example of the distribution of raised structures 21. For example, any one of the raised structures 21 can be formed in the form of a point, a line, or a surface. When multiple raised structures 21 are set, they can be uniformly distributed; or the location and sparsity of the distribution of raised structures 21 can be specifically designed according to the state of the welding interface or the need for welding strength.

In the direction shown in FIG. 3, FIG. 17, etc., the cross-sectional shape of the raised structure 21 in the longitudinal direction is not limited. It can be a shape with a large top and a small bottom, such as an arch, to reduce the contact area of the weld interface during resistance welding. raised structures in this example is a number of bumps raised downward.

As shown in FIG. 19, in an example where the intermediate body 20 is provided separately, the top surface of the substrate 30 is provided with a substrate raised structures 202 that is raised toward the bottom surface of the intermediate body 20 (in the direction), and in the example where resistance welding is performed, the substrate raised structures 202 can also increase the contact resistance between the intermediate body 20 and the substrate 30 by reducing the contact area of the welding interface, thereby generating more resistance heat to improve the welding efficiency and strength of the weld. The substrate raised structure 202 may be solid object or highly porous (higher solid volume fraction than the first porous structure 10); the substrate raised structure 202 may be integrally formed with the substrate 30, or may be additionally provided to the substrate 30 (either combined with the substrate 30 prior to resistance welding), or the substrate raised structure 202 may be formed on the top surface of a surface connection layer 202. top surface, the bottom of this surface connection layer 202 being preconnected (e.g., welded) to the top surface of the substrate 30. Except for the opposite cross-sectional shape in the projection direction and longitudinal direction, the structural arrangement of the substrate raised structures 202, etc., can be referred to raised structures 21 of the interlayer without going into detail.

When the holding space is provided by the first porous structure 10 in cooperation with the interlayer of the intermediate, at least a part of the holding space is formed within the first porous structure 10. Said holding space may extend to the interlayer of the intermediate in some directions: the positions on the interlayer corresponding to these extension part directions may not be formed with openings, so that the interlayer becomes the edge interface of the holding space in these directions (e.g., the intermediate body 20 of FIG. 11 has its top surface within the corresponding gap as the edge interface of the bottom of the holding space 504); or the positions on the interlayer corresponding to these extension directions may also form openings, such as a recess structure (not limited to the shape of a hole, slot, etc.) that allows the holding space to extend into the interlayer section, or a structure (not limited to the shape of a hole, slot, etc.) that extends through the interlayer section so that the holding space can extend further into the substrate 30 (such as the bottom of the holding space 509 of FIG. 12 extending through the intermediate body 20 and the raised structure 21 into the substrate 30 a recess at the bottom). Similarly, in the example where the holding space extends into the substrate 30, openings may or may not be formed in the substrate 30 at locations corresponding to these directions of extension part, allowing the substrate 30 to be the edge interface of the holding space in these directions, or allowing the holding space to extend into or even through the substrate 30 by means of a recessed or through structure formed in the substrate 30 at corresponding locations. this increases the volume of the contained substance The volume of the holding space can be increased; when necessary, the contained substance can also be fixed through the interlayer or the substrate 30.

The intermediate body 20 of the intermediate can serve as the edge interface of the holding space near the side of the substrate 30 (e.g., FIG. 11, FIG. 14). When the accommodator or its external enclosure bodies is a relatively stable solid object in structure, form, etc., the intermediate body 20 is fixed (such as carrying, connecting, tight fitting or position-limiting, etc.) to the accommodator (or its enclosure bodies) alone or with other edge interfaces, or with other parts of the connected structure. As shown in Figure 14 the edge interfaces at the bottom of the holding space 514 are constructed by the corresponding range of intermediate bodies 20 for carrying or attaching to the solid object 61.
Exemplarily, for the intermediate body 20 that is within the holding space, a recessed structure that can be extended for the holding space can be formed by reducing the thickness of the upper surface of this part of the intermediate body 20.

The intermediate body 20, which is in the range of the holding space, may be closed, or may be provided with a channel or opening. The space between adjacent raised structures 21 within the holding space can be a channel or opening that connects the holding space to the outside. Referring to the Embodiments documented above, said channels or openings may be open or closed (for a short or long period of time) by means of closure bodies. In addition to the closure bodies described in other Embodiments, the portion cut off at the intermediate body 20 to open the channel or opening can be used as an enclosing body to enforce closure bodies after placement of the contained substances.

Exemplarily, the intermediate body 20 may further form a position-limiting protrusion extending into the holding space to position-limiting it in contact with the surface of the contained substances (or its closure bodies); the surface of the contained substances (or its closure bodies) may further form a position-limiting recess to correspond to said position-limiting protrusion; or, the surface of the contained substances (or its closure bodies) may form a position-limiting protrusion extending outward into the holding space and the intermediate body 20 forms a corresponding recess. The position-limiting protrusions or position-limiting recesss of the intermediate body 20 may be provided alone or in conjunction with the position-limiting protrusions or position-limiting recesss of the first porous structure 10 to restrict the surfaces of the contained substances (or their covering bodies) in different directions, respectively.

When the interlayer alone contains the raised structures 21 (corresponding to the example without intermediate body 20), the spacing distance between adjacent raised bumps of raised structures 21 is reasonably set: in some examples, the contained substances (or their capsules) are partially set in the spaces between adjacent raised bumps of the raised structure 21 and can be cooperatively secured by the surrounding first porous structure 10, the substrate 30 between raised bumps of the raised structures 21, and other components (as in Figure 17); or, in some examples, one or more of raised bumps of the raised structures 21 support the contained substances of the solid object (or its capsule) from below (the solid object 61 on the left side of FIG. 18 is supported by raised structure 21). The middle of FIG. 18, by being connected to some extension part 520, allows raised bump 211 of this example to be within the confinements of the holding space 518. It may be that a part of the substrate 30, the intermediate (the part outside raised structures 21), the first porous structure 10 or another part provided, etc., is used as the extension part 520, a part of said extension part 520 being connected to other parts outside or adjacent to the corresponding holding space 518 (here the extension part section 520 is connected at the first porous structure 10), and said other portion of the extension part 520 is connected to raised bump 211 and extends that raised bump 211 into the confinements of the holding space 518. Alternatively, another part of the solid object edge interface at the bottom of this holding space 518 may be supported by an adjoining raised bump 212.

Considering that raised structures 21 is provided to reduce contact resistance and may itself be small in size, it is possible not to provide position-limiting protrusions or recesss on raised structures 21. In some examples, it is allowed to set the position-limiting protrusions or recesss on the surface (such as the top or side) of the raised structure 21, or on other parts arranged in extension part on the basis of said surface.

Some examples have certain edge interfaces of the holding space in the form of formed parts (see Example 1). For ease of description, a wall plate is used here as an example. Said wall plates may be supported by or connected to the interlayer of the intermediate; for example, longitudinally arranged wall plates that may be erected on the top surface of the intermediate body 20; or, for example, transversely arranged wall plates that may be stacked on the top surface of the intermediate body 20 (in contact with or connected to each other). For example, the longitudinally or transversely arranged wall plates may be supported or connected by the upper surface of the raised structure 21 (e.g., wall panel 519 on the right side of FIG. 18 is erected on the corresponding raised structure 21). In some examples, the wall plates may not have any connection or contact with the interlayer, and such a wall plates may be separated from the interlayer by a first porous structure 10 (e.g., edge interface 511, 513, etc. at FIG. 13) or by a gap (e.g., edge interface 514, etc. at FIG. 14, with additional contained substances within the gap).

In this Embodiment, for example, the specific features related to the intermediate body 20 or the raised structure 21 of the interlayer, and the holding space or the contained substance can al be applied in other Embodiments.

### Embodiment III

As shown in FIGS. 22 to 33, in this Embodiment, the design of the holding space and the placement of the contained substance when a polymer material is provided at the porous structure is depicted.

When joining two components (e.g., between substrate 30 and a porous structure, or between adjacent porous structures) by injecting a polymer material, for example, a high-temperature molten polymer material that penetrates simultaneously into the surfaces of the two components to be connected (the surfaces of the two components to be connected touch each other or are separated by a short distance); or by placing the polymer material into and between the surfaces of the two components to be connected, the two parts and the polymer material are heated up as a whole to melt the polymer material, so that it penetrates simultaneously into the two surfaces to be connected.

Exemplarily, as shown in FIGS. 22 and 23, the first porous structure 10 is connected to the substrate 30 with an injected polymeric material, which forms a polymeric interlayer 400. The first porous structure 10 in this example may be a stand-alone structure (not forming a composite with the intermediate), or a part of the first connected structure of the composite that is in direct contact with the substrate 30 (without restricting the case where the composite is connected with the substrate 30 by other means, while the composite comprises an intermediate, or a first porous structure 10 and/or the intermediate are also connected to the substrate 30 by other means), not shown in the figures.

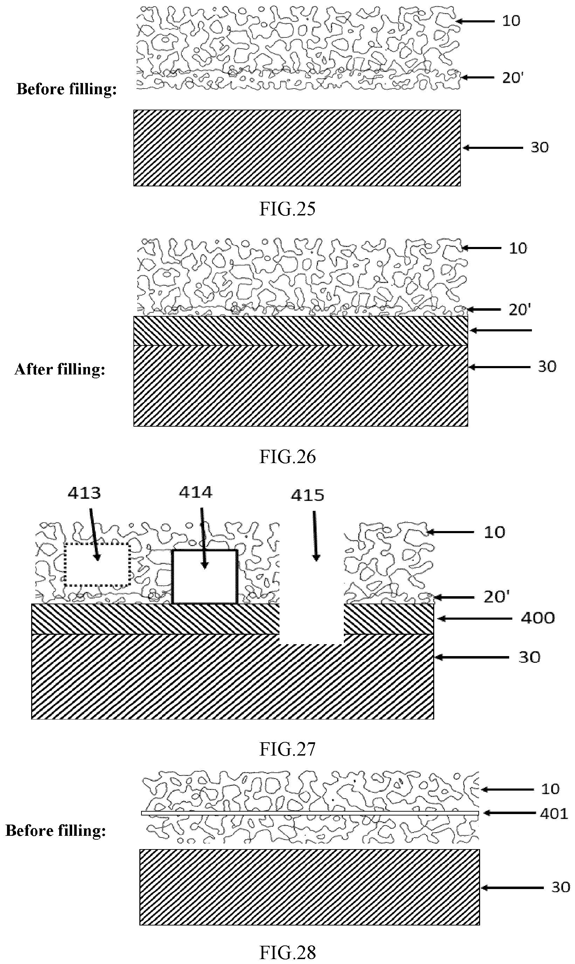

In the example where a second porous structure 20' is used for the interlayer itself pre-connected with the first porous structure 10, as shown in FIGS. 25 and 26, this second porous structure 20' is connected to the substrate 30 through an injected polymer material interlayer 400.

The substrate 30 which is connected to the first porous structure 10 or the second porous structure 20' through a polymeric material may be a solid object. Preferably, the surface of the substrate 30 is rough or formed with pores. Alternatively, the substrate 30 connected to the first porous structure 10 or the second porous structure 20' by means of a polymeric material is overall a third porous structure (higher solid volume fraction than the first porous structure 10), or at least the surface of the substrate 30 being a third porous structure. In several of the above examples, at least part of the surface of the first porous structure 10 (e.g., the top surface) is exposed to external open space.

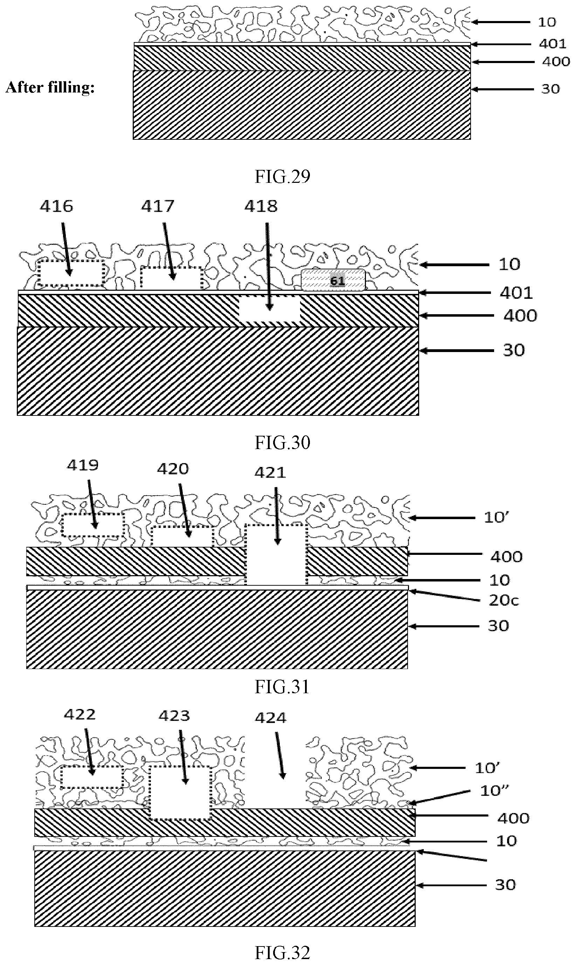

In some examples, it is assumed that the first porous structure 10 is already connected to the substrate 30, or that the composite comprising the first porous structure 10 and the intermediate is already connected to the substrate 30 (as shown in FIG. 31, the first porous structure 10 is already connected to the substrate 30 through the intermediate body 20c, connected to its bottom); such a first porous structure 10 is also connected to the fourth porous structure 10' on some other surfaces (e.g., the surface on the side away from the substrate 30, in this case the top surface) through the injected polymeric material, forming a polymeric interlayer 400.

This fourth porous structure 10' is provided independently, or, alternatively, is pre-connected with the fifth porous structure 10" to form another composite (similar to the way how to form a composite with the first porous structure 10 pre-connected with the second porous structure 20'), and at least part of the surface of said fourth porous structure 10' (e.g. on the side not connected to the first porous structure 10, in this case the top surface) is exposed to external open space. The solid volume fractions of said fourth porous structure 10' and fifth porous structure 10" are different, and the respective solid volume fractions of the fourth porous structure 10',and fifth porous structure 10" can be same as or different from that of any of the first porous structure 10, the second porous structure 20 ', and the third porous structure.

Various examples of this Embodiment are formed by injecting polymeric materials for connection of the corresponding components while forming porous structures exposed to external open sapce on at least one side of the substrate 30. In the case of an orthopedic prosthetic implant with a connected structure, the solid volume fraction of the exposed porous structure (such as the first porous structure 10 of FIG. 25 or the exposed top surface of the fourth porous structure 10' of FIG. 31) is designed to exhibit the "bone ingrowth" effect, while the respective porous structure with the polymer injected into the pores of the surface (such as the bottom surface of the first porous structure 10 connected to the substrate 30 of FIG. 25 or the bottom surface of the fourth porous structure 10' and top surface of the first porous structure 10 of FIG. 31) is designed such that the solid volume fractions of the porous structures would facilitate the injection of the polymer material into the pores of the surface and reliable connection of the corresponding parts.

In these examples of this Embodiment, the thickness of the substrate 30 can be appropriately reduced by the polymer material incorporated, which can ensure the basic strength of the whole connected structure and also improve the stress shielding phenomenon. The stress shielding phenomenon means that when two materials with different elastic moduli are loaded together, the one with the larger elastic modulus will bear more stress; the elastic modulus of the substrate 30 is much larger than that of the bone, so the bone bears less stress, which can lead to postoperative osteolysis in severe cases. Since the present invention incorporates polymer material with elastic modulus less than that of substrate 30, the stress shielding phenomenon will be improved accordingly.

As shown in FIGS. 28 and 29, a barrier layer 401 can further be provided in the parts to be connected, and it can prevent the flow of polymeric material on one side thereof to the other side, and can prevent the injected polymeric material from spreading beyond the set region. Exemplarily, the barrier layer 401 is placed at the edge region within the connected part where the polymer material is to be injected. A barrier layer 401 can be provided in a porous structure or at the boundary of two adjacent porous structures. The barrier layer 401 of FIG. 28 and FIG. 29, in the case of a barrier plate, is provided at the edge of the region of the first porous structure 10 where the polymer material is to be injected. In order to prevent the polymer material from passing through, the barrier plate 401 may be solid object, or a porous structure with a high solid volume fraction (higher than the part to be connected where the barrier plate 401 is located), or another suitable material. The barrier plate 401 may be integrally formed with the part to be connected, or may be provided separately (both before injection of the polymer material).

The injected polymer material and the regions of the two parts connected by the polymer material are called the polymer material intermediate layer 400. The connected structure of this Embodiment contains at least one polymer material intermediate layer 400.

The location of the holding space may avoid the polymeric material intermediate layer 400 and be set at other porous structures that are not connected to the polymeric material intermediate layer 400, or at a location of the same porous structure that is not in contact with the polymeric material intermediate layer 400. Alternatively, it is provided at the intermediate or substrate 30 that is not in contact with the polymeric material intermediate layer 400.

For example, the holding space 413 of FIG. 27 is located at the first porous structure 10 where the polymeric material intermediate layer 400 is not provided (this polymeric material intermediate layer 400 is located between the second porous structure 20' and the substrate 30). The holding space 410 of FIG. 24 is located at the first porous structure 10 without the polymeric material interlayer 400 (the polymeric material interlayer 400 is located between the bottom of this first porous structure 10 and the substrate 30). The holding space 416 of FIG. 30 is located in the area where the polymeric material intermediate layer 400 is not provided at the first porous structure 10 (this polymeric material intermediate layer 400 is blocked on the other side by the barrier layer 401). The holding space 421 of FIG. 31 is located in the region where the polymeric material intermediate layer 400 is not provided in the fourth porous structure 10'; The holding space 422 of FIG. 32 is located in the fourth porous structure 10' where the polymeric material intermediate layer 400 is not provided (this polymeric material intermediate layer 400 is provided between the fifth porous structure 10 " and the first porous structure 10).

In some examples, the polymeric material layer or barrier layer 401 thereof is used to provide the holding space and secure the contained substance. For example, a portion of the holding space is located within the polymeric material layer, and the holding space may extend to a component to which the polymeric material is connected (e.g., porous structure, substrate 30, etc.), or to other components of the connected structure through a notch in the connected component, or further connected to the external space; or, the holding space may be located entirely within the polymeric material layer without extension part.

For example, the holding space 411 of FIG. 24 is located partially within the polymeric material intermediate layer 400 and partially within the connected first porous structure 10; The holding space 412 is placed entirely within the polymeric material layer 400. So is the holding space 418 of FIG. 30. And the holding space 415 of FIG. 27 is placed in the polymer material intermediate layer 400 and the first porous structure 10 to which it is connected, the substrate 30, and this holding space 415 is further connected to the external open space. The holding space 419 of FIG. 31 is placed at the polymeric material intermediate layer 400 and the first porous structure 10 and the fourth porous structure 10' to which it is connected, the intermediate body 20c at the bottom of the first porous structure 10 is the edge interface of the holding space 419. The holding space 423 of FIG. 32 is placed at the polymeric material intermediate layer 400 and the fourth porous structure 10' and the fifth porous structure 10" on one side thereof. The holding spaces 425, 425' and 426 of FIG. 33 are all placed at the polymeric material intermediate layer 400 and the first porous structure 10 on one side thereof, and the holding space 425 extends through the intermediate body 20c to the substrate 30; the holding space 425' does not extend to the substrate 30, but has the intermediate body 20c as the bottom edge interface; the holding space 426 has the intermediate body 20c as the bottom edge interface and also has the barrier layer 401 as the top edge interface.

The contained substances may be in direct contact with the polymer material or barrier layer 401 if it does not interfere with the placement and/or use of the contained substances. The layer of polymer material, or barrier layer 401 thereof, may be used to form an edge interface on one side of the holding space. When using barrier plate 401, the holding space may be formed on the side of barrier plate 401 in close proximity to the polymeric material or on the other side away from the polymeric material. The barrier layer 401 can be used alone or in conjunction with other component structures to secure (load, connect, tight fit, position-limit, etc.) the contained substance or its capsule. The polymeric material layer, if suitably formed, can also be used alone or in conjunction with other component structures to provide similar fixation of the contained substances or its enclosure bodies (the polymeric material can at least carry the contained substances).

Examples of using the polymeric material intermediate layer 400 as a certain edge interface can be seen in the following figures: Figure 24 with the holding space 411 (which is partially placed within the polymeric material intermediate layer 400, with the polymeric material intermediate layer 400 forming the bottom and side edge interfaces) and the holding space 412 (with the polymeric material intermediate layer 400 as the edge interface of its periphery in multiple directions); Figure 27 with the holding space 414 (which is formed within the first porous structure 10 and the second porous structure 20' with the polymeric material intermediate layer 400 as the edge interface at the bottom thereof) and the holding space 415 (which partially passes through the polymeric material intermediate layer 400, with the polymeric material intermediate layer 400 as the edge interface on the side where 415 passes through 400); Figure 30 with the holding space 418 (placed within the polymer material intermediate layer 400 below the barrier layer 401, with the polymer material intermediate layer 400 as edge interfaces in a plurality of directions) and Figure 32 with the holding space 420 and Fig 32 with the holding space 424 (each with the corresponding polymer material intermediate layer 400 as an edge interface at the bottom thereof).

Examples of a barrier layer 401 as an edge interface on one side can be seen in the holding space 417 of FIG. 30 with the barrier layer 401 as the edge interface at the bottom thereof; the barrier layer 401 may also carry the solid object 61 placed above, and the holding space 419 of FIG. 31 with the barrier layer 401 as the edge interface at its bottom, and the holding space 426 of FIG. 33 with the barrier layer 401 as the edge interface at the top thereof.

The parts of the intermediate layer 400 or barrier layer 401 of polymeric material, corresponding to the holding space, may be closed, or may be provided with a channel or opening for the overlying holding space. Exampes are seen in FIG. 31, with arrow 71 at the holding space 419 schematically indicating a channel placed along the polymeric material intermediate layer 400 and connected to the exterior; also FIG. 32 with, the holding space 423 having an opening 72 connected to the pores of the surrounding fourth porous structure 10', thus allowing connection to the exterior or to other components, and with the holding space 424 having an opening directly connected to the external open space; and in FIG. 33 with arrows 73 schematically indicating a channel placed along the substrate 30 and connected to the holding space 425, and with arrows 74 indicating a channel connected to the pores of the fourth porous structure 10' and connected to the holding space 425' through the polymeric material interlayer 400. Other channels and openings in the holding space or closure bodies are not annotated in the figures.

Another example is a certain form of polymeric material or barrier layer 401 that can allow a contained substance to pass from that polymeric material or barrier layer 401. Alternatively, it is possible to make a formed part or filler somewhere, or to make a closure bodies somewhere, using the injection of the polymer material, or the formation of the barrier layer 401.

The polymer material injection, the polymer material intermediate layer 400 and the barrier layer 401, and the characteristics of the holding space or the contained substances in relation thereto etc, as in this Embodiment, can be applied to other Embodiments.

### Embodiment IV

As seen in Figs.34-70, in this Embodiment, the holding space is designed and the contained substances are placed by providing a support part, or by the cooperation of the support part with a component such as the first porous structure 10 (or the interlayer of the intermediate, or the substrate 30). One or more support parts are provided, each having at least a portion inserted in the first porous structure 10.

The preferred support part is inserted substantially from the side of the first porous structure 10 away from the substrate 30, to a direction towards the side close to the substrate 30. The angle of insertion can vary. In the illustrated direction, for example, it can be inserted vertically or with some inclination. When there are multiple support parts, their respective insertion angles can be the same or different, depending on the actual application.

The support part may secure (e.g., carry, connect, tightly fit, or position-limit, etc.) to the contained substance, either alone, or in conjunction with other support parts, or in conjunction with other component structures, without limitation. The capsule of the contained substance, or the edge interface of the solid object of the holding space, etc., may be similarly secured, without being described in detail.

For example, a support part may be coupled to a contained substance; at least one side surface of the contained substance may be coupled to one or more support parts. The support parts may carry the contained substance; a contained substance may be carried by one or more support parts. The plurality of support parts may carry or connect the contained substances from the same direction, or may carry, connect, tightly fit, or position-limit the contained substances from different directions. The support parts may also be provided through the load. The surface of the contained substances not carried or connected by the support parts may be suspended or may be secured by other components such as the first porous structure 10 or the intermediate interlayer or substrate 30.

In FIG. 34, for example, adjacent support parts 801 provide a tight fit to a solid object 61 provided therebetween; in FIG. 35, for example, a support part 802 carries a solid object edge interface of a holding space 427, which is a recess of a first porous structure 10 formed above the support part 802; a support part 803 cooperates with the substrate 30 and the first porous structure 10 to form an edge interface of the holding space 428 in all directions; the support part 803 also cooperates with another adjacent support part 804 so that a solid object edge interface of the holding space 429 is connected at said support parts 803, 804; FIG. 37, for example, shows a solid object edge interface of the holding space 428 in all directions. The support part 803 fits the substrate 30 and the first porous structure 10 to form the edge interface in all directions of the holding space 428, and the support part 803 also fits the other adjacent support part 804 so that the solid object edge interface of the holding space 429 is connected at said support parts 803, 804; the support part 808 of FIG. 37 is flush with the top surface of the first porous structure 10 and fits the top surface of the first porous structure 10 to carry a solid object 61. The support part 821 of FIG. 39 passes through the holding space 437. in FIG. 43, the solid object edge interface of the holding space 431 is located between adjacent support parts 812 (which may be tightly fit or connected by the support part 812); the holding space 432 of FIG. 44 is located between adjacent support parts 813, and the solid object edge interface of the holding space 432 is set inside the support part 813 (e.g., the support part 813 is notched, etc.); the support part 814 of FIG. 45 is carried substantially at the four corners of the solid object edge interface of the holding space 433.

In some examples, where the support part itself is sufficiently large compared to the contained substances to be placed, or where the support part is hollow, one or more holding spaces may be formed directly inside the support part (e.g., holding spaces are placed inside support part 810 of FIG. 38). Alternatively, the support part itself will be structured so that there will be some gaps or openings, and said gaps or openings can be used to construct all or part of the holding space, or to provide other components that can be secured to the contained substance.

For example, the support part 813 of FIG. 44 has openings that allow the edge interface on the corresponding side of the holding space 432 to extend into the interior of that support part 813. For example, the support parts 816 and 817 of FIG. 47 each have openings into which the ends of one wall plate 991 are set; in contrast, wall plate 992 between the support parts 818 and 819 is not set into the interior of these two support parts 818 and 819; wall plate 993 is connected to the outside surfaces of the support parts 816 and 818; and wall plate 994 is connected to the other wall plates already secured by the support parts 991 and 992. These wall plates 991 to 994 of FIG. 47, respectively, serve as edge interfaces in different directions of the holding space 435. The support part 814 of FIG. 46 then cooperates with one of the wall plates 99 in the opposite direction to set one edge interface of the holding space 434.

The holding space, gap or opening, etc., constructed based on the support part may be pre-designed and formed while the support part is made, or may be formed by subsequent processing (formed prior to placement of the contained substances, and not restricted to after the support part is made, or after the first porous structure 10 forms a composite with the intermediate, or after the composite is connected to the substrate 30).

Depending on the way of securing the contained substance, the support part can be an edge interface in one or more directions of the holding space. One or more corresponding support parts may be located on one side of the edge interface (other edge sides with or without support parts). The spacing between adjacent support parts may be open, and is connected to the neighboring first porous structure 10. or external open space; or, adjacent support parts are close to each other without spacing; or, the spacing between adjacent support parts is closed (e.g., by forming wall plates, filler surfaces, polymeric material layers, barrier plates, etc. as described in other Embodiments to implement closure, and not limited thereto). Alternatively, when the support parts are sufficiently large, one or more directional surfaces of one support part (which may be outer surfaces or inner surfaces formed by openings etc.) are used as edge interfaces in one or more directions of the holding space (e.g., the surface on one side of the support part 820 of FIG. 48 for forming an edge interface in a corresponding direction of the holding space 436).

The channel or opening of the holding space can be located at the edge interface close to the support part (e.g., avoiding the support part itself), or in the space between adjacent support parts, or a channel or opening can be formed directly by running through a portion of the support part (running through transverse, longitudinal, or other directions in isolation or in combination). Said channels or openings may be left open after placement of the contained substance, or closed by closure bodies (temporarily or permanently).

For example, the support part 810 of FIG. 38 is provided with an internal holding space and is schematically represented with an arrow 75 as a channel connecting it to the first porous structure 10 and to the exterior. Arrow 76 of Figure 39 schematically indicates an internal channel of the support part 821, connecting the external space and the holding space 437. The holding space 440 of FIG. 40 has the substrate 30 as the bottom edge interface and a lower side of the support 823 in close proximity thereto as part of the edge interface in the corresponding direction, which is also provided with an opening that connects to the pores of the first porous structure 10 below the support 823, which is closed by the closure body 93; the holding space 441 also has the substrate as the bottom edge interface, and the holding space 823 is not in contact with the nearby support part 824, etc., and the top of the holding space 441 having an opening connected to the outside via the opening 103 corresponding to the first porous structure 10; this opening is closed by the closure body 94. For example, the holding space 442 of FIG. 41 has an opening at one edge interface that connects to a gap 105 at the first porous structure 10, with gap 105 corresponding to the intended installation position of the support 826. Once the holding space 442 has been placed with the contained substance, the said support part 826 is installed, which closes up the gap 105 and also the opening on this side of the holding space 442.

The support part may have direct contact or connection with the first porous structure 10 around its insertion site; or, there is no such contact or connection, but the support part is inserted at a gap formed within the first porous structure 10; which may be pre-designed and formed with the first porous structure 10 or by later processing (the timing of the later processing is not limited to after the formation of the first porous structure 10, or after the formation of the composite of the first porous structure 10 with the intermediate, or after the composite is connected to the substrate 30). As in FIG. 34, there is no spacing gap between the support part 801 and the first porous structure 10; the support part 806 of FIG. 36 has the spacing gap 100 with the surrounding first porous structure 10.

The structure, number, shape, size and location of the support part are not restricted and can be determined according to actual applications. For example, the support part can be plate, strip, block, column, peg, etc., and others not limited to the above; its transverse (or longitudinal) cross-section, can be of a certain geometric shape, or irregular shaped; its cross-section shape and size at each height can be the same or different; its side surface can be flat, curved, arbitrary curved, or a combination of a variety of shapes. A support part can be a single piece, or can be a plurality of parts assembled.

Assuming that the support of each of the other examples is circular in cross-section, while Figure 47 schematically provides a different shaped support part 818 with a square cross-section; Figure 46 schematically provides a support part 815 with a semi-circular cross-section and a straight edge corresponding to the holding space interface 434; this support part 815 being made with a semi-circular cross-section or by later processing to the shape described.

The material of the support part is not limited if it is mainly for the purpose of holding the object, etc. The support part itself may be integrally formed with the first porous structure 10, etc., or may be provided separately at a later stage (or before placing the contained substance). In some examples, the support part is made of a molten substance that solidifies after injection to the first porous structure 10.

Alternatively, in some preferred examples, the composite is connected to the substrate 30 by resistance welding, wherein the support part may function as an insertion portion of an intermediate, placed into the first porous structure 10 prior to welding, to function as an electrode position-limit and/or to enhance current conduction.

A support column is used as an example structure of a support part. The features described in each example of the support column can be applied to other support parts, and vice versa. The cross-sectional shape of the support column is not limited (it can be geometric, circular or some kind of polygon, or irregularly shaped), and a cylinder is used as an example in each figure, and the directions depicted are the directions shown in the figures.

In the example of resistance welding, the support part can be used to position-limit the electrode. Since the electrode of resistance welding needs to be pressed against the workpiece to be welded while providing current, it is prone to cause damage to the surface of the workpiece due to the resistance heat generated or the pressure applied.

Considering that, in the connected structure, the first porous structure 10 itself has numerous pores and usually at least part of the surface of the first porous structure 10 is exposed and more susceptible to damage from resistive heat or pressure; for example, the pores in the compressed part of the first porous structure 10 may become smaller, the struts may break or shift, affecting the porous form of its surface as originally designed, and indentation marks may also be formed; if the resistive heat is too high, it may also leave burning marks on the surface of the first porous structure 10.

Thus, the examples below are illustrated mainly in terms of a support part to position-limit the electrode (first polar electrode 401) close to the side of the first porous structure 10. The second polar electrode 402, for example, is in contact with the bottom surface of the substrate 30, or with another composite connected to the bottom side of the substrate 30; and the substrate 30 or intermediate has a higher solid volume fraction, etc., than the first porous structure 10, and may not have a surface that would be exposed to the exterior; thus, a support part may be provided to position-limit the second polar electrode 402 in a similar manner, or may not position-limit the second polar electrodes 402.

In the first example, as shown in FIGS. 52 to 55, the top surface of the support part 81 is lower than the top surface of the first porous structure 10, i.e., the support part 81 is buried in the first porous structure 10, and then the first polar electrode 401 compresses the top surface of the first porous structure 10 downward by a certain distance (see the enlarged view in FIG. 52). This electrode, if it contacts the top surface of the support part 81, is position-limited without continuing to move downward; i.e., the top surface of the first porous structure 10 can still be substantially flush with the top surface of the support part 81 after maximum compression (the top surface of the first porous structure 10 would still be higher than the top surface of the support part 81 if it were not maximally compressed).

In the second example, as shown in FIGS. 56 to 57, the top surface of the support part 82 is flush with the top surface of the first porous structure 10, then the electrode 401 (or the electrode segment 405) of the first polarity is pressed against the stop surfaces of support part 82 and first porous structure 10, and the electrode will not continue to move downward due to the position-limiting effect of the support part 82, i.e., it will not collapse the top surface of the first porous structure 10 .

In the third example, as shown in FIGS. 58 to 61, the top surface of the support part 83 is higher than the top surface of the first porous structure 10, then the electrode 401 (or the electrode segment 405) of the first polarity mainly contacts the top surface of the support part 83 when pressed and is limited by the support part 83 and does not continue to move downward; thus this electrode 401 is not in contact with the top surface of the first porous structure 10 and does not affect the top surface form of the first porous structure 10.

In the first and second examples mentioned above, since the electrode 401 (or the electrode segment 405) is still in conductive contact with the first porous structure 10, the support part 81 or 82 can be made of either insulating or conductive material; in the third example mentioned above, the electrode 401 (or the electrode segment 405) is not in conductive contact with the first porous structure 10, the support 83 needs to be made of conductive material so that the current applied through the electrode 401 (or the electrode segment 405) can be conducted through the support 83. The support part 81 or 82 made of insulating material is utilized by the present invention for its function of position-limiting the position. The support parts 81, 82, 83 made of conductive material, on the other hand, can further enhance current conduction.

Preferably, the solid volume fraction of the support part is higher than the solid volume fraction of the first porous structure 10, and if the support part is solid object, or a porous structure with higher solid volume fraction, the structural strength is enhanced to effectively resist the force when the electrode is pressed. On the other hand, when such a support is a good conductor, the support itself has less resistance and better electrical conductivity than the first porous structure 10 with pores in its vicinity (with air and other bad conductors inside the pores), and the current is preferentially conducted through the support to the interface between the composite and the substrate 30; according to the previous section, the resistance heat Q is proportional to IR², and if the other structures remain unchanged, the use of the support part of the good conductor increases the current I in the circuit by reducing the resistance in the current conduction path, which in turn increases the value of the resistive heat and improves the efficiency and strength of the welding bond.

In considering the position-limiting function, it is necessary to design the top height of the support part (see the first example to the third example), but the other parts can be set arbitrarily as needed. And even when the entire support part of the good conductor is buried in the first porous structure 10 and there is still a first porous structure 10 above and below the support part (such as the support part 801 shown in FIG. 34; the support part 827 in FIG. 41, and the support part 830 in FIG. 42), the resistance at this support part is still smaller compared to the first porous structure 10 around it, and the current can be guided through the support part more efficiently.

Alternatively, the bottom of the support part of the good conductor, which may extend downward to the bottom of the first porous structure 10, contacts the interlayer or substrate 30, conducting the current more quickly near the interface where the composite is connected to the substrate 30. For example, the support part 811 of FIG. 38, and the support part 822 of FIG. 39, each contacting the substrate 30 at the bottom; the support part 81 of FIG. 52, FIG. 53, and FIG. 55, the support part 82 of FIG. 56 and FIG. 57, and the support part 83 of FIG. 58 and FIG. 59, each contacting the interlayer directly at the bottom (while the interlayer in each figure varies in form and contains the intermediate body 20 and/or the raised structure 21). The bottom of the support part 81 of FIG. 54 extends to the bottom of the first porous structure 10 without direct contact with the interlayer (raised structure 21), but can conduct current through the first porous structure 10.

The support part may be inserted in a gap in the first porous structure 10 without contacting the surrounding first porous structure 10 to avoid damage to the surrounding first porous structure 10 by conducting resistive heat to the surface of the surrounding first porous structure 10 (as shown in FIG. 153 with the support part 806 separated by a gap 100 around it). The support part may also be in contact with or connected to the surrounding first porous structure 10 to conduct current to the surrounding first porous structure 10 (e.g., FIG. 52 to FIG. 61, etc.).

And according to the above equation, if it is possible to increase the contact resistance R at the same time as the current I increases, it is possible to make the resistance heat even greater. The contact resistance R is more related to the contact interface state of the workpiece to be welded (composite, substrate 30). For this reason, in addition to providing the support part of the good conductor separately, it is possible in some examples to provide the support part together with the interlayer of the intermediate, for example, during resistance welding, the intermediate contains both the support part and the intermediate body 20, both the support part and the raised structure 21, both the support part, the intermediate body 20 and the raised structure 21, etc.

The support part and the intermediate body 20 may or may not be in contact with each other (the intermediate body 20 is provided with raised structures 21 below or without raised structures 21); the number of the support part and raised structures 21 may be the same or different, and the arrangement of the two may correspond to each other or be staggered (raised structures 21 may be located below the intermediate body 20 or directly below the first porous structure 10), and the support part may or may not be in direct contact with the raised structure 21. Preferably, it is made that the support part is in direct conductive contact with the intermediate part, and/or that the electrodes are in direct conductive contact with the support part, avoiding losses from current conduction through the first porous structure 10. Of course, it is allowed that in some examples the current applied by the electrode is first conducted to the support part through some parts of the first porous structure 10, or that the current obtained by the support part is conducted to the intermediate or the substrate 30 through some parts of the first porous structure 10.

For example, FIG. 34, FIG. 35, and FIG. 37 to FIG. 40 show that the support part is not provided simultaneously with the interlayer; FIG. 153, FIG. 41, and FIG. 42 show that the support part is provided simultaneously with the interlayer, and the interlayer of FIG. 153 contains the intermediate body 20 which is in direct contact with the support part 805, 806, etc.; the interlayer of FIG. 41 contains the intermediate body 20 and raised structures 21, but neither of them is in contact with the support part 827. The interlayer sectionof FIG. 42 contains the intermediate body 20, which is also not in contact with the support part 830, etc.

For example, the interlayers of FIG. 52 and FIG. 53 both contain the intermediate body 20 and the raised structure 21, and the support parts 81 are both in contact with the intermediate body 20, but the number of support parts 81 in FIG. 52 is the same and corresponds to the raised structure 21 one-by-one, and the number and position of the support parts 81 in FIG. 53, do not correspond to the raised structure 21. The interlayers of FIG. 54 and FIG. 55 both contain the raised structure 21, and the support parts 81 of FIG. 54 do not correspond to the raised structure 21 and are not in direct contact with each other, while the support parts 81 of FIG. 55 correspond to the raised structure 21 one by one and are in contact with each other. The interlayer of FIG. 56 and FIG. 57 contains the intermediate body 20, and both are in contact with the support part 82. The interlayer of FIG. 58 and FIG. 59 contains intermediate 20 and raised structure 21, and the support parts 83 are all in contact with intermediate body 20, and the number of support parts 83 is the same as and corresponds to raised structure 21 one by one. The interlayer of FIGS. 60 and 61 contains the raised structure 21, and the support parts 83 are in one-to-one correspondence and direct contact with the raised structure 21.

In one example, as shown in FIG. 62, the intermediate comprises an intermediate body 20, a plurality of support parts 80 connected to the upper surface of the intermediate body 20, and a plurality of raised structures 21 connected to the lower surface of the intermediate body 20 (raised toward the side of the substrate 30). In this example, the second polar electrode 402 contacts the lower surface of the substrate 30, and the top surface of the first porous structure 10 is provided with an indenter 490; said indenter 490 applies force with the second polar electrode 402 to compress the composite to be connected, the substrate 30, between them; the first polar electrode 406 contacts the side area of the first porous structure 10, and a current circuit is formed between the first polar electrode 406 and the second The current circuit is formed between the first polar electrode 406 and the second polar electrode 402, such that the current flowing from the first polar electrode 406 passes through the side area of the first porous structure 10, and then is conducted to other parts of the intermediate (such as the interlayer body 20 and the raised structure 21 in the intermediate area) through the support part 80 and the intermediate body 20 near the side area, etc., and then the composite body (such as near the raised structure 21) is welded to the substrate 30 is fixed by welding. Then, the support part 80 corresponding to the middle area of the first porous structure 10 is used more for position-limiting the indenter 490 (avoiding excessive underpressure) and, if needed, for conducting the current. The current entering from the side has less overall resistance on the path passed from the first polar electrode 406 through the intermediate (interlayer) to the substrate 30, improving the current conduction effect. Said first polar electrode 406 located on the side, in any form, can be a ring-shaped frame around the side, or a planar electrode, or a single unit of electrode, etc.

A variation of the above example is to make the first polar electrode directly contact at least one of the support part 80, the intermediate body 20, and raised structures 21 located at the edge from the side, and conduct current to the intermediate at other locations via the conductive parts of the edge, and thus weld the composite body to the substrate 30; the side of the first porous structure 10 of this variation example may not be exposed, and the support part 80, the at least one of the intermediate body 20, and the raised structure 21 has an exposed surface located directly at the edge of the composite body to make conductive contact with the first polar electrode; alternatively, the first polar electrode may be inserted inside the first porous structure 10 in the edge region to make conductive contact with at least one of the support part 80, the intermediate body 20, and the raised structure 21 located in the edge region. For example, the first polar electrode 407 of FIG. 63 is inserted into the interior of the first porous structure 10 from the edge to contact one of the leftmost support parts 80 and the raised structure 21; the first polar electrode 408 contacts the intermediate body 20 and the rightmost side of the first porous structure 10. The first polar electrodes 407, 408 schematically represent two electrodes of the same polarity.

Based on the above example and this variation, it can be understood that the side electrode can also be applied to the case where there is no support part or the support part is not conductive, as long as there are other intermediates (such as the interlayer) can be directly contacted with the side electrode, or the side electrode can be conducted to other intermediates (such as the interlayer) via the first porous structure 10 in the edge area, the path of current flow can be somewhat shortened and the welding efficiency can be improved..

Another variant, as shown in Figure 63, is provided with side electrodes while another first polarity electrode 401 replaces the indenter 490 (Figure 62), said other first polarity electrode 401 simultaneously providing another current and applying pressure from the top surface of the first porous structure 10, in conjunction with a second polarity electrode 402 to compress the composite with the substrate 30.

The support part itself may be integrally formed or pre-connected to the first porous structure 10 and/or the interlayer, etc., or may be provided later (at any point before welding); and in addition to securing the contained substance (or its capsule or holding space) in the state at which it is made or when it is welded, in some examples the support part may be machined after welding. For example, if the surface to be fixed is flat, a part of the side of the cylindrical support part can be cut off to form a flat surface to increase the surface to be fixed to the holding space; if the surface to be fixed of the holding space is of other shapes,, a matching surface shape can be machined on the support part. For example, after the resistance welding, an opening is made at a part of the support part to secure the contained substance or install a part securing the contained substance. If an opening is formed before resistance welding, the gap in the opening may affect the conductive effect of the support part itself. Another example is that after resistance welding, the support part may be opened, sectioned, or completely removed (e.g., Figure 41 a support part is in two segments 825a, 825b, with 825a segment removed; e.g., Figure 42 the support part 828 is removed in its entirety), thereby leaving more space in the first porous structure 10 to set up the holding space, place the contained substance, or to directly use the space left by removing the support part to place the contained substances or hold the contained substances.

In this Embodiment, the relevant characteristics of the support part, the holding space and the contained substance can be applied to other Embodiments.

### Embodiment V

In this Embodiment, some examples of variations concerning the support part, the first porous structure 10, the electrode, etc. are provided, whereby the holding space is designed and the contained substance is placed. The support column is still used as an example in the figures.

In the third example of Embodiment IV, the top surface of the support part is higher than the top surface of the first porous structure 10 (e.g., support part 83 of FIGS. 58, 59). This support part can be removed above the top surface of the first porous structure 10 after the resistance welding is completed to avoid the support part protruding on the product surface during subsequent use.

Alternatively, one or more of the support parts above the top surface of the first porous structure 10 can be retained after the resistance welding is completed (the higher portions of the other support parts can be removed), and a corresponding hoop structure (e.g., a ring structure formed by adapting the cross section of the support part) can be formed over the contained substance (or capsule, solid object edge of the holding space) so that the hoop structure is over the corresponding support part; the top surface of the support part may be higher, equal to or lower than the top surface of the hoop structure. In this example, the contained substance is located on the top surface of the first porous structure 10 (next to the support part) and further carried or connected by the top surface of the first porous structure 10 (the top surface may remain flush or form a downward recess to hold the contained substances), or, alternatively, the contained substance, etc. may be suspended without contact with the top surface of the first porous structure 10. As shown in FIG. 39, the holding space 438 has a solid object edge interface, and the hoop structure 481 is provided over the adjacent support part 822 above the top surface of the first porous structure 10, with the bottom of the holding space 438 overhanging and not in contact with the top surface of the first porous structure 10 (it is understood that if the holding space 438 is positioned further down, the bottom of it can be carried by the top surface of the first porous structure 10).

Referring to the above, based on the first or second example of Embodiment IV, the first porous structure 10 around the top of one or more of the support parts is removed so that the top of the support parts is exposed, and the hoop structure is provided (contained substance, capsule, solid edge interface of the holding space, etc.) on the top of the support parts to secure the contained substance etc..

For example, if the top of the support part 805 of FIG. 153 is lower than the top surface of the first porous structure 10, and the support part 805 itself is inserted at the gap 100 formed within the first porous structure 10, then the holding space 430 has a solid object edge interface, and the hoop structure 481 provided can use the gap 100 to set onto the top of the support part 805 and can form a recess in the corresponding area at the top of the first porous structure 10 to carry the holding space 430. The support part 811 of FIG. 38 is inserted directly into the first porous structure 10 (without a peripheral spacing gap), and the top of the support part 811 is lower than the top surface of the first porous structure 10, and the top of the first porous structure 10 is removed in the corresponding area to form a recess and to expose the top of the support part 811, and a solid object 61 set hoop structure 481 directly over the top of the support part 811, the solid object 61 itself also being carried by the first porous structure 10 within the recess.

Similarly, when the holding space matches parts of the support part at certain heights (eg the bottom or middle part), the hoop structure designed for securing the contained substance (or its capsule, or solid edge interface of the holding space, etc.) can be secured at corresponding locations on the support part (with the nearby portion of the first porous structure removed and a channel or opening provided thereto). ,If the hoop structure can be conveniently slid and snapped onto the support part from its top or bottom end, the hoop structure can be provided as a closed-hoop structure (Figure 49 providing a closed-hoop hoop structure 481); However, when it is snapped at other heights of the support part, or if it is not conveniently snapped directly from the top or bottom end, the hoop structure can be made into a non-closed-hoop structure (e.g., the hoop structures on each side form an outwardly facing arch with the cylindrical surfaces of the two side supports being in close contact to secure the fixation; FIG. 50 provides an example of an open hoop structure 482). As shown in FIG. 37, the first porous structure 10 between the lower portions of the support parts 807, 708 is removed to form a gap 101 for placing a solid object 61 that is provided with an open-hoop structure 482 that can be secured in close contact with the support parts 807, 808 on both sides.

In some examples, after compelting welding, one or some of the support parts are completely or partially removed, leaving a corresponding gap in the first porous structure 10, and a matching insertion structure (e.g., column, peg, block, plate, etc. and others not confined by the above) is formed near the contained substance (or its capsule, or solid edge interface of the holding space, etc.), and the insertion structure can be inserted in the gap left by the removal of the support parts. The first porous structure 10 on the side of the gap is used to position-limit the insertion structure and prevent it from sliding laterally. If the insertion structure fits tightly into the said gap, the contained substance etc. is thereby secured; if they do not fit tightly, they function mainly for position-limit and need to work in conjunction with other parts of the connected structure for securing the contained substance. For example, the contained substances may be located on the top surface of the first porous structure 10 (at the part next to the gap), may be further carried or connected by the top surface of the first porous structure 10 (the top surface may remain flush or form a downward recess to hold the contained substances), or may be suspended without contacting with the top surface of the first porous structure 10.

For example, one part of the support parts of FIG. 41 is 825a and 825b, where 825a section is removed to leave a gap 104 so that an insertion structure 483 provided at the bottom of the solid object 61 can be inserted into the gap 104 to allow the solid object 61 to be secured; or one part of the support part 828 of FIG. 42 is completely removed to leave a gap 106, and the insertion structure provided at the bottom of the solid object 61 is inserted into the gap 106 to allow the solid object 61 to be fixed. In both cases, the solid object 61 may be inserted deep enough to be carried by the top surface of the first porous structure 10 at the same time; alternatively, the solid object 61 alone is held in place by the combination of the first porous structure 10 with the insertion structure at the gap 104 or 106, without contacting with the top surface of the first porous structure 10.

The insertion structure of the above example is arranged substantially longitudinally; in another example, a lateral insertion structure may also be provided on the solid object, and inserted into a gap in the support part, pores in the first porous structure 10, etc., to secure the solid object. For example, the holding space 429 of FIG. 40 has an edge interface of the solid object, with the insertion structure 483 provided on each side, inserted into the adjacent support part 823; this holding space 429 is also carried by the top surface of the first porous structure 10. FIG. 51 illustrates one example of the lateral insertion structure 483. It will be appreciated that, similar to the example of FIG. 37, replacing the hoop structure 482 of the solid object 61, with a lateral insertion structure (the support part 807 needs to have a corresponding opening for insertion), also allowing the fixation of this solid object 61.

In some examples, the support part is inserted in a gap formed at the first porous structure 10, which has a gap between the support part and its surrounding first porous structure 10; a hoop structure matching said support part or an insertion structure (e.g., a hollow column) matching said gap is formed at the contained substance (or capsule, edge interface of the solid object of the holding space, etc.); the hoop structure of the contained substance, etc., can be placed over said support part or the insertion structure can be inserted into said gap after completing resistance welding. If the hoop or insertion structure achieves a tight fit, then the contained substances, etc. can be secured thereby; otherwise, they can be secured when in conjunction with other components of the connected structure (e.g., the support part, the first porous structure 10, etc.). For example, the contained substances may be located on the top surface of the first porous structure 10 (next to the support) and further carried or connected by the top surface of the first porous structure 10 (the top surface may remain flush or form a downward recess to hold the contained substances), or the contained substances, etc. may be suspended without contacting with the top surface of the first porous structure 10.

For example, the bottom of a solid object 61 of FIG. 42 is provided with an integrated hoop and insertion structure 484 inserted at a gap 107 around the top of a support part 829 while snap at the top of that support part 829, which was originally below the top surface of the first porous structure 10, and a portion of structure 10 around the top of that support part 829 is removed to form said gap 107. It is understood that, similar to Figure 153, the support part 806 itself is inserted into a gap 100 within the first porous structure 10, and that the integrated hoop and insertion structures may also be inserted into that gap 100, and the solid object is held in place by the support part 806 (or in conjunction with the first porous structure 10 around it, etc.).

In addition, although FIGS. 37 to 40 and FIGS. 49 to 51 illustrate hoop structures 481, 482 and insertion structure 483 are located on both sides of the solid object 61 or holding space, the actual number of hoop structures 481, 482 or insertion structures 483 are not specified in either number or location of settings. For example, an solid object has a greater number or more orientations of hoop structures 481, 482 and insertion structures 483 to match more support parts for fixation at the same time. Or, some times, an solid object having fewer hoops or insertion structures may also be secured by the support part alone or by matching the support part with the surrounding first porous structure 10, for example. Similarly, although FIG. 41, FIG. 42, etc. show one insertion structure, or one integrated hoop structure and insertion structure of an solid object, the actual number or location of insertion structures or integrated hoop structures and insertion structures provided per solid object or per holding space is not limited; when an solid object or holding space has a greater number or more directions of insertion structures or integrated hoop structures and insertion structures. Each of them can correspond to the support part, or at least one of the support parts can be matched with one of the insertion structures or integrated hoop structures and insertion structures.

In some examples of implementing resistance welding, a first polar electrode, proximal to the side of the first porous structure 10, contains one or more electrode segments. Said electrode segments may be inserted into the gap formed within the first porous structure 10 in direct contact with an intermediate interlayer portion (e.g., intermediate body 20, raised structure 21, intermediate body 20 with raised structure 21, etc.) exposed within the gap; alternatively, the gap is not directly connected to the interlayer portion, but a portion of the first porous structure 10 remains at the bottom of the gap, and the segments of electrodes are in direct contact with this part of the first porous structure 10 is in direct contact (not shown in the figure), so that the current is conducted through this part of the first porous structure 10 to the interlayer of the intermediate. The electrode segment may be in contact with the first porous structure 10 next to the gap into which it is inserted, or it may be separated by the gap or insulator.

As shown in FIGS. 64 and 66, a plurality of the first polar electrode,segments 409 inserted into a gap within the first porous structure 10, and thereby directly contacting the top surface of the intermediate body 20; FIG. 69 shows that the first polar electrode 401 is a large planar electrode, which simultaneously contacts a plurality of conductive media 4011, each of which inserted into a gap within the first porous structure 10 and thereby directly contacting the top surface of the intermediate body 20 (intermediate body 20 with raised structure 21 in Figures 64 and 69, and intermediate body 20 without raised structure 21 in Figure 66).

Alternatively, said electrode segment may also be inserted into a gap formed within the support part in direct conductive contact with the support part (which may have a top surface higher or lower than or equal to the top surface of the first porous structure 10, with the gap of the support part recessed downward from the top surface of the support part). The intermediate of this example may contain only the support part or may contain both the support part and the interlayer (e.g., intermediate body 20, raised structure 21, intermediate body 20 with raised structure 21). It is allowed to have a part of the first porous structure 10 between the support part and the intermediate, or between the support part and the substrate 30. More preferably, it is made to have a direct conductive contact between the support part and the intermediate to conduct the electric current.

A plurality of the electrode segments 409 are as shown in FIG. 67, each inserted into a recess at the top of the support 831, are in direct contact with that support 831; the electrode 401 of the first polarity, shown in FIG. 70, is a large planar electrode, which simultaneously contacts a plurality of conductive media 4011, each of which is inserted into a recess at the top of the support 831, in direct contact with that support 831 (FIG. 70 of the intermediate body 20 with the raised structure 21, and the intermediate body 20 of FIG. 67 without the raised structure 21).

In each of the above examples, there is no or a small amount of electrical current flowing through the first porous structure 10 so as to, improve the conductive efficiency and also avoiding thermal damage to the exposed surface of the first porous structure 10. After completion of resistance welding, the inserted electrode segment 409 or conductive medium 4011 is removed, leaving gaps 108 located within the first porous structure 10 (FIG. 65) or gaps 110 located within the support part 831 (FIG. 68); these gaps 108 or 110 can be used directly as holding spaces to place the contained substances; or, these gaps 108 or 110 are used to set the insertion structures, where the contained substance (or its capsule, the edge interface of the solid object of the holding space, etc.) is placed (e.g., the bottom of the holding space 443 of FIG. 65 and FIG. 68 is provided with insertion structures 483 inserted into the gaps 108 and 110, respectively). If the insertion is a tight fit, the contained substances, etc. can be secured thereby; otherwise, the contained substances, etc. can be secured in conjunction with a first porous structure 10 or a support part, etc., next to the gap. For example, the contained substances may be located on the top surface of the first porous structure 10 and may be further carried or connected by the top surface of the first porous structure 10 (the top surface may remain flush or form a downward recess to hold the contained substances), or the contained substances, etc. may be suspended without contacting with the top surface of the first porous structure 10.

Although the examples are described in terms of insertion of the electrode segment from the top, it is understood that if the electrode segment is inserted into the first porous structure 10 from other directions (e.g., the side) or into a gap in the support part (opened sideways), both the implementation of resistance welding and the use of the gap to place an contained substance or its insertion structure are also possible.

The variation examples in this Embodiment and the characteristics of the holding space or the contained substances associated with them can all be applied to other Embodiments.

### Embodiment VI

As shown in FIGS. 71 to 85, in this Embodiment, some examples are provided of setting other parts using the support part and designing the holding space and placing the contained substance thereby.

The support part 80 in the figure is still exemplified by the support post. The support part 80 corresponds to and contacts raised structures 21 of the interlayer, which also contains the intermediate body 20.

A coupling part 981 or extension part 982 can be provided on the support part 80 to build the edge interface of the holding space, or it can be used directly to secure (carry, connect, tightly fit or limit, etc.) the contained substance (or its capsule). Each coupling part 981 is connected to two support parts 80 at each end. Each extension part 982 is connected to the support part 80 at one end and may extend to other components (such as the first porous structure 10) at the other end or be open at the other end.

For example, the coupling part 981 of FIG. 71 is connected to the lower part of the support part 80, substantially flush with the intermediate body 21 of the interlayer, and the top surface of the coupling part 981 becomes the edge interface at the bottom of the holding space 459. For example, the coupling part 981 of FIG. 72 is connected to the upper portion of the support part 80 above the intermediate body 21, forming a pocket-like holding space 460 below the coupling part 981, with the top surface of the coupling part 981 becoming the edge interface at the top thereof. The extension part 982 as in Figure 73 is connected to the lower part of one support part 80 on the left side at one end and is open at the other end (with the first porous structure 10 left partially between the other support part 80 on the right side); the holding space 461 is formed between the two supports 80, partly above the extension part 982 and partly laterally beyond the extension part 982, opening between the extension part 982 and the right support part 80; this extension part 982 is substantially flush with the intermediate body 20.

The coupling part 981 or extension part 982 may also be in contact with or connected to other components of the connected structure, such as the first porous structure 10 supported from below; alternatively, the coupling part 981 or extension part 982 may be suspended, such as where the first porous structure 10 at the corresponding location is removed and the coupling part 981 or extension part 982 is not in contact with the first porous structure 10, for example (and instead relies on the connected support part).

As shown in Fig 74, one side of the support part 80 is connected with an extension part 982a, and the other side has an opening to connect with another extension part 982b; the extension parts 982a and 982b are not at the same height, and their top surfaces are the bottom edge interfaces of the holding spaces 462 and 463, respectively. The top surfaces of the holding spaces 462, 463 have openings connected to the external space; the holding space 463 also has the support part 80 as the edge interface on one side; the holding space 462 is not connected to the support part 80; the extension part 982a is overhanging below, its left end connected to one of the support ends of the first porous structure 10; part of the first porous structure 10 below the extension part 982b can carry it; the interlayer of this example does not have an intermediate body.

The shape, size and quantity of the coupling part 981 or extension part 982 are not limited, and can be in the form of a plate, strip, rod, etc. If it does not affect the placement or use of the contained substances, or channel or opening of the holding space so formed, then the space above coupling part 981/extension part 982 can be hollowed out, or there can be a gap between adjacent coupling part 981/extension part 982 corresponding to the same edge interface.

One coupling part 981 (or extension part 982) may correspond to the edge interface of the holding space on its one side, covering all or part of the area corresponding to that side edge interface; or, multiple coupling parts 981 (or extension parts 982) may simultaneously correspond to one side of the edge interface of the holding space, cooperatively covering all or part of the area corresponding to the edge interface on that side (multiple coupling parts 981 / extension parts 982, covering part of the area, may be spaced apart or close to each other without spacing). The coupling part 981 and the extension part 982 may be provided at the same time or at different time; may be located at the edge interfaces on the same side or at different edge interfaces; may be connected to the same support part 80 or to different support part 80.

Exemplarily, the two support parts 80 connected to each end of the coupling part 981 may be located at two adjacent or oppositely oriented edge interfaces within the holding space. And between two support parts there could be one or multiple coupling parts 981. Exemplarily, two extension parts 982 of the same edge interface of a holding space may be provided at the same or different heights, and the respective ends of the two extension parts 982 that are not connected to the support part 80 may be close to or apart from each other at a distance.

For example, the coupling part 981c shown in FIG. 82 is a sheet corresponding to an edge interface in a certain direction of the holding space, with two support parts connected to each of its left and right sides. The coupling parts 981d and 981e shown in FIG. 83 are strips, each having one end connected to the same support part, and the two coupling parts 981d and 981e are located at different heights; the extension part 982c is a strip, which is at the same height as the coupling part 981e, spaced apart from each other, and jointly located at the edge interface on the same side of the holding space.

The coupling part 981/extension part 982 may be integrally formed with the support part 80, or the coupling part 981/extension part 982 may be an additionally provided component (separately formed and connected to the support part 80, provided prior to securing the contained substance, etc.). Other Embodiments of the wall plate, filling surface, polymer material layer, barrier plate, etc. all can be used as the coupling part 981/extension part 982. If set prior to the resistance welding implementation, it is preferred to use a conductive material (e.g., solid object or high solid volume fraction porous structure) to make the coupling part 981/extension part 982.

An example holding space, as shown in FIG. 72, contains: a coupling part 981, a surrounding (on both sides or above) support part 80, the intermediate body 20 within the holding space is provided with a gap; the coupling part 981 is located above the gap. The support part 80 is inserted in the first porous structure 10 and connected to the intermediate body 20 adjacent to the holding space, and the coupling part 981 separates the first porous structure 10 above it. Thus a pocket-like structure of the holding space 460 is formed between the coupling part 981, the support part 80, and the substrate (not shown) below the notch of the intermediate body 20. as shown in Figure 76, the contained substance (solid object 61) may fit tightly with the surrounding support part 80; alternatively, the contained substance (solid object 61) may be carried by the substrate 30 below the notch of the intermediate body 20; alternatively, the contained substance is fixedly connected to at least one side of the components (e.g., the coupling part 981, the support part 80, the substrate 30), and the way of setting the contained substances is not limited to these examples. The notch opened in the intermediate body 20 may serve as a channel or opening in the holding space 460; the channel or opening may additionally be opened at (e.g., the coupling part 981, the support part 80, etc.) other locations.

An example holding space, as shown in FIG. 75, comprises: a coupling part 981, a surrounding (on both sides or above) support part 80, an intermediate body 20 within the holding space 464 provided with a gap, and an extension part 982 formed within the gap. The support part 80 is inserted in the first porous structure 10 and connected to the intermediate body 20 adjacent to the holding space, and the coupling part 981 is arranged above the gap of the intermediate body 20 and the extension part 982. The coupling part 981 is arranged above the notch of the intermediate body 20 and the extension part 982, separating the first porous structure 10 above. In this way, a pocket structure similar to a pocket with a closed bottom is formed as the holding space 464 between the coupling part 981, the support part 80, and the intermediate body 20 with the extension part 982 .

The extension part 982 in this example can be constructed from a part of the intermediate body 20; or it can be a separately provided part, of which the material, thickness, height from the substrate 30, etc. can be the same or different from the intermediate body 20. The extension part 982 shown in FIG. 75 on the left and right sides can be different parts of the same extension part (such as an annular extension part 982d shown in FIG. 84); or it can be two separate extension parts (such as the extension part 982e shown in FIG. 85), each connected to the support part 80 on the corresponding side, and the material, thickness, height from the substrate 30, etc. of the two extension parts can be the same or different.

After a suitably shaped and sized contained substance is placed in the holding space. Its edge can be carried by the extension part 982 so that it does not fall off, or it can be further fixedly connected to the extension part 982 if needed (FIG. 77 also shows an example of jamming the solid object 61 between the extension parts 982 on either side). The other parts of the contained substances may not be in further contact with the substrate 30, the coupling part 981, the support part 80, etc. below the notch in the intermediate body 20; or, the contained substances may be carried by the substrate 30 (or both the substrate 30 and the extension part 982); or, the contained substances may be further connected to one or more of the substrate 30, the coupling part 981, the support part 80, and the manner in which the contained substances are provided is not limited to these examples. The notch opened in the intermediate body 20 may serve as a channel or opening in the holding space; the channel or opening may additionally be opened in other locations (e.g., the coupling part 981, the support part 80, the extension part, etc.).

For example, Figure 78 shows that a holding space 465 is formed as pocket structure with a closed bottom, with the upper coupling part 981, support part 80, intermediate body 20 with openings and extension parts 982, ; the coupling part 981 has a number of openings 78, which can be connected to the first porous structure 10 above the coupling part 981 and thus to the external open space.

An example holding space, as shown in FIG. 79, contains: a first coupling part 981a above, a surrounding (on both sides or above) support part 80, and a second coupling part 981b below. the support part 80 is inserted in the first porous structure 10 and is connected to the intermediate body 20 next to this holding space, and the first coupling part 981a separates the first porous structure 10 above. The second coupling part 981b may be a part of the intermediate body 20 or may be a component independent of the intermediate body 20, with the ends of the second coupling part 981b connected to the support parts 80 on each side thereof. If it is an independently formed part, the material, thickness, height from the substrate 30, etc. of the second coupling part 981b can be the same or different from the intermediate body 20. The second coupling part 981b can be used to carry the contained substances placed in the holding space, and can also be fixedly connected to the second coupling part 981b when needed; other parts of the contained substances can be left out of contact with the first coupling part 981a, the support part 80, or can be further connected to the first coupling part 981a and/or the support part 80.

For example, the holding spaces 466 and 467 shown in FIGS. 79 and 80 each contain a first coupling part 981a, a support part 80, and a second coupling part 981b; the first coupling part 981a is provided with a number of through-holes 78 that can connect to the first porous structure 10 above the coupling part 981 and thus to the external open space; the second coupling part 981b is provided with a number of through-holes 79 that can The second coupling part 981b is provided with a number of through-holes 79 that connect to the gap below the second coupling part 981b and thus to the gap between raised structures 21 and the substrate 30. The through holes 79 of FIG. 79 are open, while the through holes 79 of FIG. 80 are closed by the closure bodies 95. For example, the holding space 468 shown in Figure 81 contains a first coupling part 981a, a support part 80, and a second coupling part 981b; the first coupling part 981a has a number of openings 78 that connect to the first porous structure 10 above the coupling part 981 and thus to the external open space, and the openings 78 are closed by the closure bodies 96; the second coupling part 981b does not have openings.

In each of the above examples, in addition to forming the channel or opening for the holding space in one of the coupling part 981/extension part 982 (or support part 80), it is also possible to not install one of the coupling part 981/extension part 982 (or support part 80) in its designed position, leaving the designed position as a channel or opening for placing the contained substance, and then the coupling part 981/extension part 982 ( or support part 80) can be installed in place to close the channel or opening.

For the three examples of FIG. 72, FIG. 75, and FIG. 79 above, the support parts 80 correspond to at least two sides of the holding space, as shown in the left and right directions (at least one on each side); the edge interfaces corresponding to the front/back directions of the paper may have one or more support parts 80 or no support parts 80. Depending on the holding space closure bodies requirements, the morphological use of the contained substance, or the connection requirements, etc., the adjacent support parts Depending on the holding space closure bodies requirements, the morphological use of the contained substance, or the connection requirements, etc., the adjacent supports 80 may be connected to each other (e.g., each side of the left, right, front, or rear) with other coupling parts 981 and/or extension parts 982, or without coupling parts 981 or extension parts 982 and directly facing the first porous structure 10 outside the holding space (or even, in some examples, to an external open space, or to an intermediate body 20 or substrate adjacent to one side of the holding space 30, etc.).

For the three examples in Figures 72, 75, and 79 above, the distance between the upper coupling part 981 (or first coupling part 981a) and the substrate 30 and the lower extension part 982 (or second coupling part 981b) is greater than or equal to the thickness of the contained substance (at the time of insertion). The illustration shows the substrate 30, the extension part 982, and the second coupling part 981b at the bottom of the housing, but if the entire structure is subject to orientation changes (e.g., the difference in orientation between production and use, or orientation changes during use, etc.), the other oriented parts of the structure (the upper coupling part 981/first coupling part 981a, the support part 80, the peripheral The first porous structure 10, etc.) can also be fixed (such as carrying, connecting, tightly fitting, or position-limiting, etc.) to the contained substances, etc., individually or cooperatively. Certain orientations that do not contact or connect to the contained substance may be open, allowing portions of the contained substance to extend beyond these open boundaries (i.e., beyond certain boundaries of the holding space) on the basis of ensuring that the contained substance can be secured.

In this Embodiment, the support part 80 with the coupling part 981 or extension part 982, and the characteristics of the holding space or contained substance associated with it, can be applied in other Embodiments.

### Embodiment VII

As shown in FIGS. 86 to 93, in this Embodiment, some examples of variations of the intermediate and its application in resistance welding are provided, and the holding space and placement of the contained substance are designed thereby.

When using laser welding, the welding position between the intermediate of the composite and the substrate 30 is not specifically limited, and at least one pair of contact surfaces can be selected for welding according to the application needs. As an example, intermediate body 20 contains a bottom, which can be any shape and size, a porous structure or solid object with a higher solid volume fraction than the first porous structure 10, the bottom of the intermediate body 20 in Figure 2 is in the form of a sheet or plate; the bottom of the intermediate body 20 is welded to the top surface of the substrate 30. Alternatively, in some examples, the intermediate body 20 contains a bottom and extends to at least one side above the bottom to form a periphery of the intermediate body 20; the bottom and the periphery of the intermediate body 20 may have the same or different shape, size (e.g., thickness), solid volume fraction, etc.; preferably, the solid volume fraction of both the bottom and the periphery of the intermediate body 20 are higher than the solid volume fraction of the first porous structure 10 (which is a porous structure with higher solid volume fraction or solid object). Preferably, the solid volume fraction of both the bottom and the periphery of the intermediate body 20 are higher than the solid volume fraction of the first porous structure 10 (which is a more porous structure or solid object).

FIG. 86 illustrates the case where a recess is provided on the substrate 30, where the composite of the first porous structure 10 and the intermediate body 20 is set in the recess, and where the bottom 20a and the periphery 20b of the intermediate body 20 are in contact with the top surface 30a and the side 30b of the recess of the substrate 30, and where these contact surfaces are respectively welded in place (the symbol 40 schematically indicates that the two are welded to each other). Of course, the present invention does not limit the shape of the recess, its size or its position on the substrate 30, etc.; nor does it limit, in other examples, the weld fixation only between the intermediate body 20 and the substrate 30 or one of the contact surfaces of the recess thereof (and no weld between the other contact surfaces); for example, weld only between the bottom 20a of the intermediate body 20 and the top surface 30a of the recess of the substrate 30 or welding only at the periphery 20b of the intermediate body 20 to the recess side 30b. It is also possible, for example, in some examples, to have the composite body have both portions set in the recess and portions that contact the non-recess location of the substrate 30 (e.g., portions that contact the surface 33 of the substrate 30 next to the opening of the recess), and these contact portions of the composite body may all be welded to corresponding locations of the substrate 30 or only some of them may be welded to corresponding locations of the substrate 30.

Based on the example of FIG. 86, the following structural variations can be made: the side surfaces 30b of the recess of the substrate 30 in FIG. 86 are substantially perpendicular to the top surface 30a of the recess; the periphery 20b of the intermediate body 20 is also substantially perpendicular to the bottom 20a. All side surfaces of the recess of the substrate 30 in FIG. 87 (both sides shown) are inclined bevels 321; part of the side surfaces of the recess of the substrate 30 in FIG. 5 are bevels 321 (one side of the recess is shown to be beveled and the other side is substantially perpendicular to the top surface of the recess). The inclined side surfaces at the recess of the substrate 30 in FIG. 4 or FIG. 5 indicate that there is a set angle formed between that side surface of the recess of the substrate 30 and the top surface of the recess; said angle is preferably acute; then the recess (cross-section) is a trapezoid with a narrow top and a wide bottom, and the further away from the top surface of the recess, the smaller the opening of the recess.

To this end, the periphery of the intermediate body 20 is accordingly provided with an inclined bevel 221, which also has a set angle (preferably acute) with the bottom of the intermediate body 20; thus, when the composite of the first porous structure 10 and the intermediate body 20 is set in the recess, the inclined periphery (bevel 221) of the intermediate body 20 is able to fit with the inclined sides (bevel 321) of the recess of the substrate 30; The bottom and periphery of the intermediate body 20 are in contact with the top and side surfaces of the recess of the substrate 30, and are welded and fixed at these contact surfaces respectively. The present invention does not specifically position-limit the location, welding position, shape (e.g., change to curved surface, arc, etc.), and size of the contact surface between the intermediate body 20 and the substrate 30 (or its recess), and can be designed according to the actual application.

Based on the example of FIG. 86, the following structural variations can be made: in the example of FIG. 92 or FIG. 93, a snap structure is provided correspondingly between the contact surface of the recess of the substrate 30 and the intermediate body 20. Figure 92 shows that each side of the recess of the substrate 30 and each side of the periphery of the intermediate body 20 (both sides are shown) are provided with a snap structure correspondingly; Figure 93 shows that part of the side of the recess of the substrate 30 and part of the periphery of the intermediate body 20 are provided with a snap structure correspondingly (one side is shown with a snap structure and the other side is not). The said snap structure, for example, is in the form of a position-limiting opening 322, let's say in the form of a recessed hole or groove, on the side of the recess; accordingly, a position-limiting protrusion 222, such as pin-like or strip-like, is provided on the periphery of the intermediate body 20, which can be set in the position-limiting opening 322 at the corresponding position. When the composite of the first porous structure 10 and the intermediate body 20 is set in the recess, the intermediate body 20 is positioned with the recess of the substrate 30 by a snap structure, and the bottom and periphery of the intermediate body 20 are in contact with the top and side sides of the recess of the substrate 30 correspondingly, and are welded and fixed at these contact surfaces respectively.

The position-limiting protrusion 222 in the snap structure shown in FIG. 92 or FIG. 93 is substantially laterally extending outward from the bottom of the intermediate body 20 and is located substantially at the junction of the periphery and bottom of the intermediate body 20 (with the position-limiting port 322 at a corresponding location on the substrate 30); while in other examples, the protrusion in the snap structure can be made to form at the periphery of the intermediate body 20 and/or the bottom of the intermediate body 20 at other locations (with the position-limiting port at a corresponding location on the substrate 30), not limited to the junction of the periphery and the bottom. Alternatively, the protrusions in the snap structure can be formed on the top and/or side of the substrate 30 (locating the position-limiting ports at corresponding locations on the intermediate body 20) without affecting the insertion of the composite. Thus, the present invention does not specifically position-limit the part, location, shape, size, etc. where the snap structure is located, and can be designed according to the actual application.

The bevel structure and snap structure of the contact part between the recess of the substrate 30 and the intermediate body 20 in the above multiple examples can be provided individually or cooperatively. These structures are collectively referred to as positioning structures, mainly to ensure that the two can fit tightly together without displacement when welding the substrate 30 to the intermediate body 20; and also to serve as a secondary fixation after the welding is completed.

In this Embodiment, the positioning structures such as bevels or position-limiting ports at the contact surfaces of the substrate 30 can be integrally formed with the substrate 30, or formed subsequently by machining after the substrate 30 has been formed. Preferably, the intermediate body 20 is pre-connected or integrally formed with the first porous structure 10. Preferably, the positioning structure such as a bevel or position-limiting protrusion at the contact surface of the intermediate body 20 is integrally formed with the rest of the intermediate body 20, using 3D printing or other processes (without limiting that, in some examples, the structure such as a bevel or protrusion is formed by certain machining means after the intermediate body 20 body is formed).

When the holding space is provided with the connected structure of each of the above examples, at least a portion of each of the holding spaces 444 to 449 is opened within the first porous structure 10 as shown in FIGS. 89, 90, and 91 (the holding space 449 is completely formed within the first porous structure 10 and is not in direct contact with either the intermediate body 20 or the substrate 30). In some examples, the bottom or periphery of the intermediate body 20 on the side adjacent to the first porous structure 10 may be the edge interface of the holding space, either alone or cooperatively; the bottom or periphery of the intermediate body 20 may remain in the form in which it was made or when laser welding was performed (e.g., the intermediate body 20 adjacent to the holding space 444, 448), or the bottom or periphery of the intermediate body 20 can be processed (e.g., making notched to expand the holding space, or processing the intermediate body 20 adjacent to the holding space 445, 447).

In some examples, the notch formed at the bottom or periphery of the intermediate body 20 does not extend through such that the edge interface in the corresponding direction of the holding space is located inside the intermediate body 20; or, the bottom or periphery of the intermediate body 20 extends through such that the edge interface of the holding space extends further to the top or side of the recess of the substrate 30 (e.g., the intermediate body 20 adjacent to the holding space 446).

When the bottom or periphery of the intermediate body 20 is used as an edge interface, or, when the top surface or side surface of the recess of the substrate 30 is used as an edge interface, the contained substances or its capsule can be fixed (carried, connected, tightly fitted, limited, etc.) alone or in conjunction with an edge interface in other directions. A channel or opening for the holding space can be opened at the bottom or periphery of the intermediate body 20, the top or side of the recess of the substrate 30, etc. (e.g., arrow 77 of FIG. 89 schematically indicates a channel from the side 30b of the substrate 30 through the periphery 20b of the intermediate body 20 and thereby connecting to the holding space 444).

The aforementioned holding space or the setting of the channel/opening therein may avoid the location where the positioning structure (corresponding bevels on the contact surface or matching protrusions and position-limiting openings, etc.) is located. Alternatively, the aforementioned holding space or the setting of the channel/opening therein may be allowed at a part of the positioning structure if it does not interfere with the use of other positioning structures. Alternatively, if the positioning structure mainly functions during the welding process, then if the holding space or the channel/opening therein is set after welding, the position of the positioning structure may not be avoided or the positioning structure may be further used to set the edge interface of the holding space or to open the channel or opening of the holding space.

In this Embodiment, the structure of the bottom/periphery of the intermediate body 20 mating with the top or side of the recess of the substrate 30, and the characteristics of the holding space or contained substance associated with it, can be applied in other Embodiments.

### Embodiment VIII

As shown in FIGS. 94 to 113, in this Embodiment, some examples are provided when the intermediate is in the form of an anchor point and its application in resistance welding, and the resulting design of the holding space and placement of the contained substance.

For the preceding Embodiment VII, the intermediate contains substantially the bottom (or both the bottom and the periphery), and the bottom is substantially a sheet or a plate; the shape and size of an intermediate (or multiple intermediates when combined) may be substantially the same as the shape and size of the connection area on the substrate; the connection area of the intermediate to the substrate is welded such that the first porous structure forming a composite with the intermediate covers the connection area of the substrate. The intermediate is welded to the connection area of the substrate so that the first porous structure forming a composite with the intermediate covers the connection area of the substrate and constitutes the exposed surface at the connection area.

The main difference from Embodiment VII is that in the connected structure between the first porous structure and the substrate described in Embodiment VIII, the intermediate between the first porous structure and the substrate is in the form of anchor points, and the first porous structure and a plurality of anchor points are formed into a composite body, and the composite body is connected to the substrate by welding the anchor points to the substrate. In this Embodiment, the anchor points can be solid object or porous structure with higher solid volume fraction.

### < Freestanding anchor point structure>

FIG. 94 is a top view of the freestanding anchor structure when it is set up, and the intermediates in the form of individual anchor points are independent of each other and are distributedly set in the first porous structure 10, which is called the freestanding anchor structure 200; FIGS. 95 to 97 are three examples corresponding to the side view in the A-A direction shown in FIG. 94. The examples use a substrate 30 with a recess, and the composite of the freestanding anchor point structure 200 or 210 and the first porous structure 10 is set in the recess of the substrate 30 (without position-limiting the use of other types of substrates 30 connected to the composite of this Embodiment).

Each freestanding anchor structure 200 illustrated in FIG. 95 contains a bottom, and the individual freestanding anchor structures 200 are spaced apart above the bottom, leaving a gap 113 in which the first porous structure 10 is not formed. 10. The bottom of each freestanding anchor structure 200 shown in FIG. 95 or FIG. 96 is welded to the top surface of the recess of the substrate 30 at a corresponding location (the connection of the two parts is schematically indicated as welded by the symbol 40 in the figure) to achieve a reliable connection of the composite body to the substrate 30.

Each freestanding anchor structure 210 illustrated in FIG. 97 contains a bottom and a periphery; the height of the side of the recess of the substrate 30 may or may not be equal to the height of the periphery of the freestanding anchor structure 210 (e.g., the periphery of the anchor point does not exceed the height of the side of the recess). In this example, there is a gap 114 above the bottom of the anchor point and in the space enclosed by the periphery, which does not form the first porous structure 10; the welding operation is carried out in said gap 114, and the bottom of each freestanding anchor point structure 210 is welded to the top surface of the recess of the substrate 30 at the corresponding position to achieve a reliable connection between the composite body and the substrate 30. The periphery or bottom of the freestanding anchor structure 210 corresponds to an edge interface of the holding space, allowing the opening of a channel or an opening therein.

Preferably, the examples of FIGS. 95 to 97 all use laser welding (the freestanding anchor structure 210 shown in FIG. 97 containing the periphery may also be used for resistance welding). After completion of the welding, the gaps 113, 114 reserved in FIG. 95 or FIG. 97, can be used as holding spaces to place the contained substances. For example, FIG. 99 shows a freestanding anchor point 200 on the left side carrying a solid object 61 placed in gap 113, with the middle freestanding anchor point 200 above the gap 113 further expanded to form a holding space 453. FIG. 98 shows a number of freestanding anchor points 200 carrying a solid object 61, with the first porous structure 10 above these freestanding anchor points 200 forming a recessed gap to hold the solid object 61. FIG. 100 shows an example of a holding space 455 formed entirely within the first porous structure 10. For example, the gap 114 on the left side of FIG. 101 contains a scattering of contained substance, and the middle gap 114 is set in the solid object 61 (which may protrude partially beyond the top surface of the first porous structure 10); the periphery of the middle and right freestanding anchor structures 210 may be a direct edge interface to the holding space 451 between the two.

The examples of FIG. 95 or FIG. 97 can also use these gaps 113, 114 to set the insertion structures formed at the contained substance (or its capsule, the edge interface of the solid objects of the holding space, etc.) (reference Embodiment V; insertion structure 483 at the bottom of the solid object edge interface of the holding space 454 of FIG. 99, inserted into the gap 113 on the right side; insertion structure 483 at the bottom of the solid object edge interface of the holding space 452 of FIG. 101, inserted into the gap 114 on the right side). If the insertion is a tight fit, solid objects such as the contained substance can be secured thereby; otherwise, they can be secured in conjunction with the first porous structure 10 next to the gap 113 or 114 or a component such as the anchor point periphery or the side surface of the substrate 30. For example, the contained substance may be located on the top surface of the first porous structure 10, may be further carried or connected by the top surface of the first porous structure 10 (the top surface may remain flush or form a downward recess to hold the object), or, solid objects such as contained substances may be suspended without contact with the top surface of the first porous structure 10. There is no pre-formed gap in Figure 96, but there may be a portion of the struts located above the freestanding anchor structure 200 that is cut off during laser welding to form a gap 113 similar to Figure 95, or the first porous structure 10 above the freestanding anchor structure 200 may be removed directly after welding to form a gap 113 similar to Figure 95 to hold the contained substances or secure the insertion structure.

In each of the above examples, the freestanding anchor structures 200, 210 are not provided at the edge of the composite; the first porous structure 10 at the edge is located between the periphery of the corresponding freestanding anchor structure 210 and the side of the recess of the substrate 30, and is in direct contact with the side of the recess of the substrate 30 (it is possible to keep only the contact between the two (Figure 97); or in some examples, the anchor point is still predominantly welded to the substrate 30, and a welding point is added between the first porous structure 10 and the side of the recess of the substrate 30). As a variant example of FIG. 97, the anchor point can be provided to the edge part of the composite so that the periphery closer to the edge on the freestanding anchor structure 210 directly contacts the side surface of the recess of the substrate 30; or a weld point can be added between the periphery of the freestanding anchor structure 210 and the side surface of the recess of the substrate 30; or, further, a positioning structure such as a mating bevel and/or snap can be provided between the two. Then, based on the periphery of the freestanding anchor point structure 210 and the side surface of the recess of the substrate 30, the holding space, the contained substance, and the opening of the channel/opening can also be provided, see also the structure of the bottom/periphery of the intermediate described in Embodiment VII that fits with the top or side surface of the recess of the substrate.

### < Linked anchor point structure>

For the above-mentioned freestanding anchor structure, several intermediates in the form of anchor points are independent of each other and form a composite with the first porous structure; the shape and size of a composite (or multiple composites when combined) can be substantially the same as the shape and size of the connection area on the substrate; the freestanding anchor structure is welded to the corresponding points of the connection area of the substrate so that the composite covers the connection area of the substrate, ( mainly with the first porous structure) constitutes the exposed surface of the connection area.

In this example, the first porous structure is connected to the substrate, and the intermediate between the first porous structure and the substrate uses a jointed anchor structure, i.e., a plurality of anchor points are included, and each anchor point is interconnected with at least one other anchor point by a joint body. The first porous structure is shaped into a composite with the anchor points and their coupling parts, and the composite is connected to the substrate by welding of the anchor points to the substrate.

The anchors may be of the same material as the anchors and may be of the same or different form; the anchors may be solid object or a porous object with higher solid volume fraction than the first porous structure; the coupling part between the anchors may be solid object or a porous object with higher solid volume fraction than the first porous structure; the anchors and the coupling part may be of the same or different solid volume fraction when both are porous. The anchor point and the coupling part are preferably formed in one piece by 3D printing or other means; however, without limitation, in some examples, the two are formed separately (supplemented by machining) and then combined together.

FIG. 102 is a top view of the linked anchor structure when set up, with a number of anchor points 220 and a coupling part 250 connecting some of the anchor points 220, located in the first porous structure 10. The shape (e.g., strip, or sheet, etc., regular or irregular) and size of the coupling part 250 are not limited; the anchor points 220 interconnected by the coupling part 250 may or may not be adjacent to each other.
FIGS. 103 to 107 are five examples of the linked anchor structure, corresponding to the side view in the A-A direction shown in FIG. 102. The examples use a substrate 30 with a recess, and the composite of the linked anchor structure and the first porous structure 10 is set in the recess of the substrate 30 (without limiting the use of other types of substrates 30 connected to the composite in this example).

In the linked anchor structure illustrated in Figure 103 or Figure 104, welding operations are performed at intervals, with the location of the welding points corresponding to anchor points 220. Coupling part 250 situates between anchor points 220 (the anchor points 220 and coupling part 250 in these two examples can be indistinguishable in form themselves and are extensions of the same part). That is, the bottom of each anchor point 220 is welded to the top surface of the recess of the substrate 30 at the corresponding position to achieve a reliable connection between the composite body and the substrate 30. Therein, a first porous structure 10 is formed above the coupling part 250; while a gap 115 is left above each anchor point 220 shown in Figure 13, and there is no first porous structure 10 inside the gap 115; while there is no gap above each anchor point 220 shown in Figure 104, which is filled by the first porous structure 10.

Figures 105 to 107 illustrate anchor points with a different state than the coupling part itself. Each anchor point 230 in Figure 105 contains a bottom and a periphery, and each coupling part contains a bottom 251 (in contrast, anchor point 220 and coupling part 250 in Figure 103 or Figure 104 contain bottoms, respectively); the bottom of some anchor points 230 is connected to the bottom of the corresponding coupling part 251. The height of the side of the recess of the substrate 30 may or may not be equal to the height of the periphery of the anchor point 230 (e.g., the periphery of the anchor point 230 does not exceed the height of the side of the recess). In this example, there is a gap 116 above the bottom of the anchor point 230 and in the space enclosed by the periphery, which does not form the first porous structure 10; the welding operation is carried out in said gap 116, and the bottom of each anchor point 230 is welded to the top surface of the recess of the substrate 30 at the corresponding position to achieve a reliable connection of the composite body (first porous structure 10) to the substrate 30.

Then, based on the examples of FIGS. 103 to 105, by pre- or post-forming gaps 115 or 116 above anchor point 230, said gaps 115 or 116 can be used as holding spaces to hold the contained substances after completion of welding. Alternatively, these gaps 115 or 116 are used to set the insertion structure formed at where the contained substance (or its capsule, the solid object edge interface of the holding space, etc.) is placed (see Embodiment V; and see Figure 99, Figure 101 for examples of gaps 113 or 146 to place the contained substance). If the insertion is a tight fit, the contained substances, etc. may be secured thereby; otherwise, the contained substances, etc. may be secured in conjunction with components such as the first porous structure 10 or the anchor point periphery or the side surface of the substrate 30 next to the gap 115 or 116. For example, the contained substances may be located on the top surface of the first porous structure 10 and may be further carried or connected by the top surface of the first porous structure 10 (the top surface may remain flush or form a recess downward to hold the contained substances), or the contained substances, etc. may be suspended without contact with the top surface of the first porous structure 10.

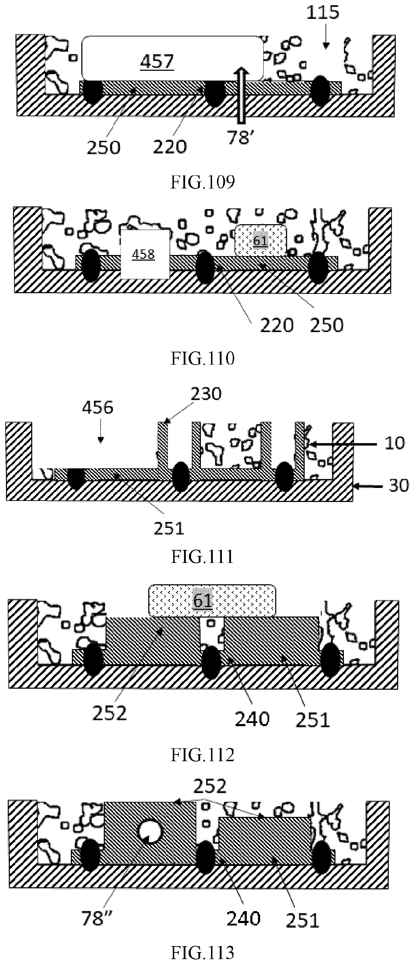

Another example is shown in Figure 109, where some anchor points 220 can be connected to the top of the coupling part 250, for example by enlarging the gap 115 to construct a holding space 457. Figure 110 shows the holding space 458 partially opened within the first porous structure 10, with the lower part running through the coupling part 250 and extending into a groove on the substrate; alternatively, the top of the coupling part 250 can carry the solid object 61 directly. Figure 111 shows that the periphery of the left anchor point 230 is removed to form a gap in the bottom 251 of the coupling part next to the anchor point 230 to form the holding space 456, and the left periphery of the middle anchor point 230 serves as the edge interface to the right of the holding space 456, which is connected to the external open space.

Each coupling part in FIG. 106 or FIG. 107 contains a bottom 251, and a sidewall 252 established on the bottom 251 thereof; each anchor point 240 contains a bottom. The bottom of some anchor point 240 is connected to the bottom of the corresponding coupling part 251 (which may be a lateral extension of a part). The height of the side surfaces of the recess of the substrate 30 may or may not be equal to the height of the sidewalls 252 of the coupling part (e.g., the height of the sidewalls 252 of the coupling part do not extend beyond the height of the side surfaces of the recess). The sidewalls 252 of different coupling parts can have the same or different heights (see Figures 106 and 107, respectively). In this example there is no gap above the bottom of the anchor point 240, which is filled by the first porous structure 10 (without limiting the use of anchor points with a gap above the bottom in other examples not shown); the bottom of each anchor point 240 is welded to the top surface of the recess of the substrate 30 at the corresponding position to achieve a reliable connection of the composite body to the substrate 30.

In turn, the sidewall 252 of the coupling part may serve as an edge interface on one side of the holding space (as in one of the sidewalls 252 of FIG. 108 for connection of the solid object 61); the bottom 251 of the coupling part may also serve as an edge interface on one side of the holding space, either alone or in conjunction with a nearby anchor point 240 with which it is coupled. Said edge interface may be used alone, or in conjunction with other edge interfaces of the holding space, or in conjunction with other components (first porous structure 10, substrate 30, other intermediates, etc.) to anchor (carry, connect, tightly fit, or position-limit) the contained substance. For example, the sidewalls 252 of the coupling part in Figure 112 are of the same height while both are below the top surface of the first porous structure 10, thus using the gap here to place the solid object 61, which is carried by the top of the sidewalls 252.

Preferably, each example uses laser welding (with the adjacent anchor points shown in Figure 105, coupling parts with sidewalls shown in Figure 106 or Figure 107, may also be used for resistance welding). The periphery or bottom of the anchor point, or the bottom or sidewall of the coupling part, corresponding to one of the edge interfaces of the holding space, allows for a channel or opening therethrough (as shown schematically in Figure 109 with arrow 78' indicating a channel from the outside through the substrate and coupling part 250 to the holding space 457; as shown in Figure 113 with an opening in the sidewall 252 of the coupling part 78 ").

In the examples of FIGS. 103 to 107, the linked anchor structure is not provided at the edge of the composite; the first porous structure 10 at the edge is located between the side surface of the recess of the substrate 30 and some corresponding linked anchor structure (e.g., around the anchor 230 or the sidewall 252 of the coupled part); the first porous structure 10 at the edge is in direct contact with the side surface of the recess of the substrate 30 (which may only maintain contact between the two; or in some examples the anchor point is still predominantly welded to the substrate 30, while additional weld points are added between the first porous structure 10 and the sidewall of the recess of the substrate 30).

As a variation, it is possible, in some other examples, to have at least part of the anchor point and/or at least part of the coupling part of the linkeded anchor point structure set to the edge part of the composite; when the anchor point with periphery/the coupling part with sidewall are at the edge part, the periphery of the anchor point/the sidewall of the coupling part, may directly contact the sidewall of the recess of the substrate; or, welding points may be added between them, or, further, a positioning structure such as a matching bevel and/or snap can be provided between them (see Embodiment I). In addition, the linked anchor point structures in the illustrations are all located at the bottom of the composite body, while in some examples, a number of linked anchor point structures may be provided individually or cooperatively at the periphery of the composite body (e.g., touching the side surface of the recess of the substrate).

Then, based on the linked anchor point structure with peripheral anchor point/coupling part with sidewall, the side surface of the recess of the substrate, the methods of forming the holding space, placing contained substance and setting the channel/opening can refer to the mating structure of the bottom/periphery of the intermediate with the top or side surface of the recess of the substrate as described in Embodiment VII.

In this Embodiment, the freestanding anchor structure or the linked anchor structure, and the characteristics of the holding space or the contained substance associated with it, can be applied in other Embodiments.

### Embodiment IX

The contained substances in this example is a sensor, and the holding space can use any of the structures and arrangements described in the Embodiments above. The type of sensor is not limited and the applicability is not limited; for example, it may be a mechanical sensor, a molecular sensor, a chemical sensor, a biochemical sensor, etc. The descriptions of the types of sensors and the selection of their modules, the structural arrangement of the holding space and the design of the channels in this Embodiment are schematic and are not meant to be limiting.

In conjunction with FIG. 75 and FIG. 114, a pocket structure similar to a pocket with a closed bottom is formed between the coupling part 981, the support part 80, the intermediate body 20 with a notch and the extension part 982 as the holding space 464; the sensor 500 is placed in this holding space 464.

In the case of the connected structure that connects a substrate to a composite containing a first porous structure and an intermediate for use in forming a prosthetic implant for implantation in the human body, as described in each Embodiment, the sensor may preferably be a temperature sensor to obtain the temperature of the human body near the implantation site. The temperature around the prosthetic implant will increase when it is infected, and the temperature sensor can detect temperature change and give an warning.

The sensor may be with an electrical energy storage elements (e.g., some kind of battery) that maintains its supply of electrical energy during standby and/or use. Alternatively, the sensor can be with a receiving coil and the necessary auxiliary components (for conversion, transmission, etc.) that work with an external feeding coil to obtain electrical energy via wireless charging technology and transmit it via lines to the electrical energy storage elements or to the core sensing element of the sensor.

Alternatively, the sensor is connected to a conductive wire, which can be threaded through the pores of the first porous structure;,or, between raised structures under the intermediate body; or, through a channel or opening in one or more of the parts including intermediate body, support part, coupling part; and, substrate. The channels on the intermediate body and/or the substrate can be in the form of unenclosed grooves or can be pipes with openings only at the input and output ends. Depending on the actual arrangement of the conductive wires, a combination of the above can be used.

The conductive wire input terminal can be connected to an energy storage and power supply device, which can be located at the substrate, at another holding space, or a separate device implanted in the body, etc., or a device located outside the body; or, the conductive wire input terminal is formed with an interface, which is located on the body surface and connected to an external power source when charging or power is required. In addition to power supply, the above wire arrangement can also be analogous to the arrangement of the signal transmission wire, and the two wires and their channels can be independent of each other or shared. This Embodiment can be further extended to the case where the contained substances are various other power-consuming devices.

The holding space is opened with channels or openings connected to the outside, and the probe of the sensor can be directed to some of these channels or openings. Alternatively, in conjunction with FIG. 78 and FIG. 115, a pocket structure similar to a pocket with a closed bottom is formed between the coupling part 981, the support part 80, and the intermediate body 20 with notches and extension part 982., which is used as the holding space 465; the sensor 500 itself can remain within the holding space 465 such that its probe 553 extends through the channels or openings beyond the holding space 465 to facilitate access to the part to be measured to collect detection information. The sensor 500 in this example can be placed into the holding space 465 through the notch at the intermediate body 20, carried by the extension part 982 on the inside of the notch of the intermediate body 20, and fixed by a number of support parts 80 at the holding space 465, the coupling part 981 between the support parts 80, and the extension part 982 at the notch (possibly in other directions, such as in front of and behind the paper surface, etc., and also utilizing the first porous (the first porous structure 10 is also utilized in other directions such as front and back of the paper). The coupling part 981 is provided with a number of external holes 78, and the probe 553 of the sensor 500 protrudes from the holes 78 and is inserted into the first porous structure 10 above the holding space 456. The sensor 500 in this example transmits signals and electrical energy through a wire 555, said wire 555 arranged through the gap between the raised structure 21 under the intermediate body 20 and the substrate 30, and connected to the sensor 500 after passing through the gap at the intermediate body 20. As shown in Figure 116, the wire 555 can also be arranged through the groove 301 opened on the surface of the substrate 30 and connected to the sensor 500 after passing through the gap at the intermediate body 20, which can also be applicable in the example where the intermediate body 20 is not provided with raised structures 21 or in the example where the raised structure 21 is spaced very little from the substrate 30 after welding.

In another example, in conjunction with FIG. 78 and FIG. 117, a pocket structure similar to a pocket with a closed bottom is formed between the coupling part 981, the support part 80, the intermediate body 20 with a notch and the extension part 982, which forms the holding space 465; the sensor is provided with a wireless charging module 551 connected to its core sensing detection module 500', placed together or separately into the holding space 465 through a notch at the intermediate body 20, carried by an extension part 982 on the inside of the notch of the intermediate body 20, and secured by a support part 80 at the holding space 465, a coupling part 981 between the support part 80, and an extension part 982 at the notch (possibly also secured in other directions using the first porous structure 10 in conjunction); the sensor is provided with an antenna 552 which connected to the wireless charging module 551 and/or the sensing detection module 500' for transmitting electrical energy and/or signals. The antenna 552 and the probe 553 of the sensor, respectively, protrude from a number of through-holes 78 provided in the coupling part 981. The example wireless charging module 551 is capable of wireless charging and information transmission based on NFC (Near Field Communication) technology. As an example, as shown in Figure 118, it is possible to further configure the electromagnetic signal trigger switch 554 (e.g. Hall switch, etc.) in the holding space, capable of converting the obtained magnetic induction signal into a corresponding electrical signal, thereby controlling the operation of the sensor by receiving external commands. Said sensing and detection module 500', wireless charging module 551, electromagnetic signal trigger switch 554, etc., may be separate devices or may be packaged as one.

In this Embodiment, the sensor and the holding space in which it is housed, as well as the features associated with it such as the substrate, the intermediate, and the first porous structure in the connected structure, can be applied in other Embodiments.

### Embodiment X

The contained substance in this example is a drug. There is no limit to the type of drug and no limit to its applicability. In each of the above examples, the structure and arrangement of the holding space having a channel or opening connected to the outside can be applied in the present Embodiment. The description of the drug form, the structural arrangement of the holding space and the design of the channel in this Embodiment are schematic and are not intended as limitations.

An example of the connected structure is a prosthetic implant implanted in the body that can be used to prevent or treat infection around the prosthetic implant by means of a drug placed in the holding space. Channels or openings are provided at the holding space to insert the drug into the space and/or to release the drug; the drug input and output channels (output and input ports) may be independent of each other or may be shared. The direction, location and size of each channel/opening can be determined according to the actual application without limitation.

Exemplarily, the holding space contains the support part, the coupling part between the support parts; as shown in FIG. 79, said coupling part comprises a first coupling part 981a located above, a second coupling part 981b below (which second coupling part 981b is independent or a part of the intermediate body), and a number of other coupling parts connecting the support part 80 in other directions (e.g., front and back of the paper, etc.).

Exemplarily, the delivery channel/output port of the drug may be opened on the first coupling part 981a, such as being a number of through holes 78 on the first coupling part 981a, whereby the released drug is diffused through the first porous structure 10 above the first coupling part 981a and delivered to a body part closely connected to the first porous structure 10. Of course, depending on the application, the delivery channel/output port of the drug can also be opened on the second coupling part 981b, such as a number of through-holes 79 on the second coupling part 981b (released via the direction where the intermediate body 20, the substrate 30 is located), or on the support part 80 or other coupling part (released drug via other directions).

Exemplarily, the input channels/input ports for the drug may be provided on the first coupling part 981a or the second coupling part 981b, for example, a number of through holes 78 or 79 on the corresponding coupling part; the input channels/input ports may also be provided on either the support part 80 or other coupling part as desired. In some examples, the through-hole 78 on the first coupling part 981a serves as both an input channel/input port and an output channel/output port (see Figure 81). In some examples, a delivery channel (e.g., in the form of a groove or pipe) is further provided on the substrate 30, and the outlet of said delivery channel is connected to the input channel/input port on the second coupling part 981b so that the drug enters the holding space via the delivery channel of the substrate 30, and the input channel/input port of the second coupling part 981b.

As shown in FIGS. 119 and 120, a number of through-holes formed in the second coupling part 981b are drug infusion holes 793, and a number of through-holes in the first coupling part 981a are drug release holes 784; the substrate 30 is formed with grooves 301, which are connected to the external, infusion holes 793, respectively, after the prosthetic implant is implanted in the human body, the drug 565 is delivered through the grooves 301 of the substrate 30, as appropriate, and through the infusion holes 793 of the second coupling part 981b into the holding space, and the drug 565 is then output through the release hole 784 of the first coupling part 981a to the first porous structure 10 above the first coupling part 981a and diffuse to the body part to which it is bonded.

Therein, the raised structure 21 of FIG. 119 is also formed with the gap between the raised structure 21 and the substrate 30, and in some cases, the said spacing gap corresponding to the second coupling part 981b can be used as an intermediate channel connecting the channel 301 and the filling hole 793 (then the channel 301 can be closed on the top side of the left half until the spacing gap corresponding to the second coupling part 981b is provided for the channel). Figure 120 applies to the example where the intermediate body 20 is not provided with raised structures 21 or where the raised structure 21 is spaced very little from the substrate 30 after welding, then the drug 565 delivered through the groove 301 of the substrate 30 is directly connected to the filling hole 793 of the second coupling part 981b (then the groove 301 may be closed on the top side of the left half until the spacing gap corresponding to the second coupling part). 981b spacing gap before the top output opening for channel 301 is provided; or, after welding the intermediate body 20 and the substrate 30 are closely conformed to each other, the top opening of the left half of the channel 301 of the substrate 20 is closed using the intermediate body 20 until the channel 301 extends to the spacing gap corresponding to the second coupling part 981b, and the drug 565 is then passed through the top opening port of the channel 301).

If the drug is required to be released continuously for a short period of time after insertion, the holding space may be not closed, with the output channel/port for release always open. Alternatively, if the drug is not released immediately after insertion, the holding space needs to be relatively closed and the input channel/port and output channel/port can be closed. For example, the openings of the input channels are closed after drug has been delivered (the closure bodies can be temporary or permanent), and the openings of the output channels can be temporarily closed and will open when needed to release the drug.

The closure bodies for closure include a variety of structures such as corks, plugs, films, etc. The closure bodies can be made of the same material (thickness, etc., can also be similar) as the components (coupling part, support part, intermediate body, etc.) next to the opening; the closure bodies can be part of these components, divided to form the opening, and then closed up after the drug placement. Alternatively, the material, thickness, etc. of the closure bodies can be unrelated to the components next to the opening, and can be chosen as needed.

For example, FIGS. 121 and 122 show that the second coupling part 981b is formed with an injection hole 791, closed by an injection hole plug 792; before implantation of the prosthetic implant into the body, use the needle of the syringe to pierce through the injection hole plug 792 into the holding space and inject the drug 565, as appropriate, and a number of through holes on the first coupling part 981a are slow release holes 781, which allow the drug 565 to pass through the slow release holes 781 for continuous transport to the first porous structure 10 above the first coupling part 981a, and diffuse to the body part.

In some preferred examples, the closure bodies may be made of a material that self-triggers to open after certain conditions are met, such as a change in temperature, pressure, humidity, for example, due to an infection around the prosthetic implant, or an artificial adjustment of the condition around the prosthetic implant by a physician, etc.); or, the material of the closure bodies may degrade after a certain period of time; or, the material of the closure bodies may react with a substance to open up (e.g., the substance that triggers the reaction is self-generated by a peri-prosthetic lesion, or may be injected by a physician when a drug release is required), so that the drug release can be controlled according to the different needs of the actual application.

As shown in FIGS. 123 and 124, the first coupling part 981a is formed with an injection hole 782, which serves as both an input and output port and may be closed by a slow release plug 783; no channel or opening is opened in the second coupling part 981b. Before implantation of the prosthetic implant into the human body, the needle of the syringe is passed through the injection hole 782, as the case may be, into the holding space and injected with the drug 565; the slow release plug 783 may be applied after the drug 565 has been injected, or it may be applied immediately before the injection, being passed through by the needle. The slow release plug 783 itself is made, for example, of a material that triggers itself to open when certain conditions are met, allowing the drug 565 to be released as needed.

Exemplarily, the drug may be inserted after the composite and the holding space therein are formed (assuming that the drug properties are not affected by the connection of the composite to the substrate). Alternatively, the drug may be inserted after the composite is connected to the substrate. Alternatively, the drug may be inserted during the surgical procedure for prosthetic implantation, as needed. Assuming that the position of the opening of the input channel to the holding space or the entrance to the transmission channel on the substrate is fixed or can be located by auxiliary detection means, the physician can inject the drug into the holding space by using a syringe or the like to inject the drug into the opening of the input channel or the entrance to the transmission channel, or, for example, after the prosthetic implant implantation procedure, to determine whether to place the drug or to replenish the released drug as needed. The drug can be replenished as needed. The release of the drug is used to treat lesions such as infections around the prosthetic implant. The rate of drug release can be controlled by designing the shape, number, and diameter of the output channels/ports.

The drug can be liquid; the need for closing the holding space can be determined based on release requirements. Assuming that the size of the drug at the time of insertion is larger than the opening of the output channel, it will not be released immediately after insertion. When certain conditions are met (e.g., dissolution by heat, liquid, etc., degradation over time, etc.), the solid object form of the drug will change (particle size becomes smaller or turns to liquid) and can be released from the output port to act on the surrounding area of the prosthetic implant. etc.) and can be exported from the output port to act on the area around the prosthetic implant. Alternatively, the drug (liquid, powder, etc.) can be externally covered by a coating (e.g., capsule, shell, etc.) to form a relatively stable solid object form, and the space or channel/opening can be set with reference to the structure described in the preceding Embodiments. The coating body itself is opened with through release channels and then the drug is released through the release channels or open boundaries of the holding space.

In combination with the contents of Embodiment IX and Embodiment X, in some examples, the sensor and the drug can be provided in the same holding space of the prosthetic implant, and can be provided in different holding spaces or in the same holding space. Then, when it is determined that the drug needs to be released based on a certain status information detected by the sensor, the drug is released using the various means described above.

Wherein, the device used to judge the status information, for example, is some kind of processor, which can be integrated with the sensor or can be set up separately, and the power supply can be referred to the power supply of the sensor; the processor can be set in the same holding space where the sensor or the drug is located, or in a different holding space, and can also be set outside the body; the processor and the sensor can be connected by wire; the processor and the sensor can be connected by a wire, or the information can be transmitted by wireless transmission, etc.

After determining the need for release, it can be a manual operation to perform the drug release. Alternatively, some trigger device may be further provided at the prosthetic implant for releasing the drug. The trigger device may be located within the holding space where the drug is located, or within another adjacent holding space, or provided somewhere outside the holding space where the drug is located. The trigger device action can act on the closure bodies itself to open the channel or opening of the holding space, or, alternatively, can change some state around the holding space, the closure bodies or the drug so that the trigger condition for the closure bodies to trigger itself to open and the drug to release itself is satisfied. For example, after determining the need to release the drug, a processor, sensor or in vitro device, etc., can give instructions to the trigger device (by wire or wireless means) to drive the trigger device to act.

Alternatively, the release of the drug may be timed. For example, a timer is used to time (or count down) the drug release, and when a specified time is reached, a command is sent to the trigger device to actuate the trigger device to enable the drug to be released. Said timing, including a defined point in time, and/or, a defined time interval, is arbitrarily set according to the needs of the specific application. For example, the drug is released at several specific points in time each day; for example, the time interval between each two releases of the drug can be set to be the same or different; there is no limitation on the unit of the time interval, it can be a number of minutes, hours, days, months, years, and so on. The timer, or timing device, may be stand-alone (a device located inside or outside the body) or may be implemented by a module built into the processor. Similarly, it can be timed to control the sensor to initiate detection and, based on the results of the detection, to determine whether a drug release needs to be triggered.

For example, the trigger device can puncture the film used for the closure bodies, the capsule wrapped around the drug, etc.; or, the trigger device can remove the closure bodies (and can reliably fix the removed closure bodies), or push the closure bodies into the holding space (the closure bodies to be pushed in does not easily leave the holding space, or does not interfere with the release of the drug or the subsequent use of the prosthetic implant), and the channel/opening is opened; or, the trigger device can change the temperature, pressure, humidity and other states around the closure bodies or drug, or can spray a liquid or deliver a substance that can react with the closure bodies or drug to meet the conditions of the closure bodies or drug capsule trigger to open, or make the drug itself change its form and suitable for release.

For example, the drugs to be released at different times can be located in different holding spaces, or in different locations in the same holding space, separated or wrapped with the corresponding covering body respectively; each time the specified time arrived, the trigger device will open up the holding space or covering body. Each time when the trigger device is used, it can be the same trigger device, or a different one. Trigger device can be for single-use, or is re-usable.

In this Embodiment, the drug and its placement and release in the holding space, the sensor, and the features associated with it such as the substrate, the intermediate, and the first porous structure in the connected structure, can be applied in other Embodiments.

### Embodiment XI

This Embodiment provides a prosthetic implants, preferably an orthopedic prosthetic implant; any one or more of the connected structures of Examples I through VIII and their respective variant examples described above may be used, and the sensors or drugs described in Embodiment IX or Embodiment X are provided in the holding spaces of these connected structures.

In the preferred example, the body of the prosthetic implant corresponds to the substrate in the connected structure, and at least a portion of the surface of the body of the prosthetic implant serves as the connection area to the composite containing the intermediate and the first porous structure, and at least a portion of the surface of the first porous structure becomes the exposed surface of the prosthetic implant by the connection (preferably welding) of the composite to the substrate.

A connected structure can be provided with one connection area to the substrate, or a plurality of connection regions connected or separated; the structure of the intermediate at each connection area can be the same or different, the connected structure of the composite to the substrate can be the same or different, and the structure for providing the holding space or the contained substance can be the same or different.

### < Artificial hip joint>

The artificial hip joint is used as an example. The artificial hip joint contains a femoral stem, a femoral ball head (not shown), an acetabular cup, and a liner (not shown), all of which are prostheses made of medical materials that can be implanted in the human body, for example, metallic materials such as titanium, cobalt-chromium-molybdenum alloy, ceramics, polymers such as ultra-high molecular weight polyethylene, and are not limited to these.

As shown in FIGS. 125 to 126, and FIGS. 133 to 136, said femoral stem 600 comprises a head, a neck, and a stem body, either in one piece or formed by assembly, and is commonly made of metal. The head of the femoral stem 600 is a tapered structure, connected at one end to the stem through the neck, and the head and neck are arranged with a certain deflection angle with respect to the stem, in the form of an inclination with respect to the side (inner side) of the stem. The lower part of the stem is inserted into the medullary cavity of the femur. A number of longitudinal grooves may be provided in the lower part of the stem body. On the surface of the stem body, exemplarily, a porous structure is formed on the upper surface of the stem body; the lower part of the stem body may have a smooth surface.

The other end of the head of the femoral stem 600 is inserted into an inner taper mounting structure to the femoral ball head; as shown in FIGS. 137 to 147, the acetabular cup 700 in some examples is partially spherical (e.g., hemispherical) in dome shape; the acetabular cup 700 is snapped onto the outer side of the liner with which it fits; the femoral ball head forms contact with the inner concave surface of the liner so that the femoral ball head can rotate there. Said acetabular cup 700 may be provided with a through-hole 701 for providing a connection (screw, etc.) for attaching the acetabular cup 700 to the acetabular notch; the liner may be provided with a corresponding through-hole or without a through-hole. The liner may be made of metal or of a non-metallic material (e.g., polyethylene or ceramic, etc.) to reduce the wear of the artificial joint. The acetabular cup 700 is typically made of a metallic material. The outer peripheral surface of the acetabular cup 700, preferably, uses a porous structure.

In some examples, the inner side of the acetabular cup 700 has a number of recessed structures 702 (see FIG. 147), the outer surface of the liner matches the inner side of the acetabular cup 700, and the outer surface of the liner is formed with a number of raised structures that can be correspondingly set in the recessed structures 702 on the inner side of the acetabular cup 700, enhancing the reliability of the fit between the two. Other examples may have no liner, and the inner concave surface of the acetabular cup is in direct contact with the femoral ball head.

In this regard, the use of porous structures on the upper (proximal) surface of the stem 600 and the peripheral surface of the acetabular cup 700 can, on the one hand, increase the roughness and help the prosthetic implant achieve effective initial stability in the skeletal host site; on the other hand, it can induce osteoblastic bone to grow in, which in turn effectively connects the femoral stem 600 to the femur and the acetabular cup 700 to the acetabular notch in a fixed manner, forming a good long-term biological fixation. Enhance the stability of the interface between the artificial hip joint and the host bone tissue.

Based on the structure and method of Embodiments I to VIII or variations thereof described above, a partially enlarged side sectional view at box line E of Figure 136 is shown in Figure 141, wherein said stem body 630 of the femoral stem corresponds to the substrate in the connected structure; a composite body comprising an intermediate 620 and a first porous structure 610 is formed in this example as a stem body housing 650 (see Figures 127 to 132) that covers the connection area of the stem body 630 (proximal end), and by welding the composite body to the substrate (stem body 630), the coverage of the connection area on the substrate by the first porous structure 610 is achieved, thereby forming a porous structure on the surface of the femoral stem body. The symbol 640 in the figure schematically indicates that the parts are welded to each other.

The stem body 630 is adapted to the stem housing 650 (or intermediate 620 contained therein) at the site of contact and connection. Exemplarily, depending on the manner of welding, the intermediate 620 of the stem housing, contains an interlayer (e.g., intermediate body and/or raised structure), and/or a support part (e.g., support post); alternatively, the intermediate 620 contains a bottom (or contains a bottom and periphery), and may also use a freestanding or coupled anchor point structure. The connection area of the stem body 630 may be provided with a recessed portion set in the stem housing 650 or a portion of the housing; or the stem body 630 may have no recessed portion and have the stem housing 650 wrapped directly outside the connection area of the stem body 630 (Figure 132); the area where the stem body 630 contacts the intermediate 620 of the stem housing 650 may be further provided with positioning structures such as a bevel and/or snap.

The stem body 630 is made by forging, casting, powder metallurgy or machining, preferably of solid structure for easy processing and high strength; or stem body 630 can be a high solid volume fraction porous structure; intermediate 620 can be solid object or a porous structure with higher solid volume fraction than the first porous structure 610; when both stem body 630 and intermediate 620 use porous structures, the solid volume fraction of the intermediate 620 is between that of the stem body 630 and that of the first porous structure 610. The intermediate 620 and the first porous structure 610 of the stem body 650 are preferably realized by using 3D printing additive manufacturing process, which can well form the pores according to the design requirements, etc. The composite body with intermediate 620 at stem body 630 and stem body shell 650 is effectively connected by welding, avoiding the current problem of significant reduction in overall strength when the porous structure is connected on the surface of the femoral stem by a hot pressing process (e.g., diffusion welding process), etc.

In a specific example, the upper (proximal) part of the stem body 630 of the femoral stem 600 is provided with an connection area; the side of the head and neck of the femoral stem 600 arranged at an angle is the medial side of this femoral stem 600, and the posterior, lateral, and anterior sides of the stem body 630 are arranged in a counterclockwise direction as illustrated in FIG. 125, with the medial side opposite the lateral side and the posterior side opposite the anterior side. FIG. 125 illustrates the anterior side of the femoral stem 600, and FIG. 126 illustrates the lateral side of the femoral stem 600.

In this example, the connection region of the femoral stem 600, corresponding to the stem body 630 comprises a portion of the surface of the upper (proximal) portion of the stem body 630 corresponding to the medial, posterior, lateral, and anterior surfaces. As shown in FIGS. 127 to 132, the stem housing 650 contains two housing pieces 650-1 and 650-2, one of which corresponds to a part of the medial surface, a posterior surface, and a part of the lateral surface of the upper part of the stem body 630; the other housing piece 650-2 corresponds to the remaining part of the medial surface, the anterior surface, and the lateral surface of the upper part of the stem body 630 The remaining part of the front side surface and the remaining part of the outer side surface. After the two housing pieces 650-1 and 650-2 are brought together, they are contacted and welded to the corresponding positions in the said connection area of the upper part of the stem body 630.

The two shell pieces may be symmetrical (or they may be staggered and crossed, not shown). Exemplarily, the adjacent edges of both shell pieces may be separated from each other without being connected after forming and assembling. Alternatively, the adjacent edges of one side of the two shell pieces (e.g., the adjacent edge corresponding to the outer side) may be connected at the time of forming and may remain connected while there is some bending near the adjacent edge (to bring the two shell pieces together). Alternatively, the two shell pieces may be formed with the adjacent edges separated from each other and the adjacent edges on each side are connected (e.g., by welding or using connectors or other means of connection) after they are brought together. By adjacent edges is meant the edges adjacent to each other after the two shell pieces are brought together. The interconnection of the adjacent edges may be to the intermediate 620 and/or the first porous structure 610 of the outer layer within each shell sheet.

The porous structure of the peripheral surface of the acetabular cup can be similarly achieved using the structures and methods of Embodiments I to VIII above or variant examples thereof. As shown in FIGS. 137 to 141, 142 to 147, with a partially enlarged side sectional view at box line E of FIG. 139 illustrated in FIG. 141, at said acetabular cup 700, the cup body 730 corresponds to the substrate in the connected structure; a composite comprising the intermediate 720 and the first porous structure 710, formed on the outer side of the cup body 730 and covering the connection of the cup body 730. The composite body is welded to the substrate (cup body 730) to achieve coverage of the connection area on the cup body 730 by the first porous structure 710 to obtain a porous structure on the outer periphery of the acetabular cup 700. The symbol 740 in the figure schematically indicates that the components are welded to each other.

The cup body 730 of the acetabular cup 700 is adapted to the composite (or intermediate 720 contained therein) at the site of contact and connection. The cup body 730 may be provided with a recessed portion of the composite body or part thereof set in the connection area of the cup body 730, or the cup body 730 may be provided without the recessed portion, with the composite body wrapped directly outside the connection area of the cup body 730. ; the part of the cup body 730 in contact with the intermediate 720 may be further provided with a positioning structure such as a bevel and/or a snap.

The cup body 730 of the acetabular cup 700 is made by forging, casting, powder metallurgy, or machining, and is preferably a solid structure for ease of machining and high strength; or the cup body 730 may be a high-solid volume fraction porous structure; the intermediate 720 may be solid object or a porous structure with higher solid volume fraction than the first porous structure 710; the cup body 730 and the intermediate 720 use a porous structure, and the solid volume fraction of the intermediate 720 is between that of the cup body 730 and that of the first porous structure 710. When both the cup body 730 and the intermediate 720 use porous structures, the solid volume fraction of the intermediate 720 are between the solid volume fraction of the cup body 730 and the first porous structure 710. The intermediate 720 and the first porous structure 710, preferably achieved using a 3D printing additive manufacturing process, can well control the pores, etc., to meet the design requirements. The cup body 730 and the composite body with intermediate 720 are effectively connected by welding, avoiding the current problem of significant reduction in overall strength through hot pressing processes (e.g., diffusion welding process), etc.

In a specific example, as shown in FIGS. 137 to 140, the entire outer surface of the cup body 730 can be used as a connection area with an integral composite body set to contact and weld with it in correspondence. As shown in FIGS. 142 to 145, it is also possible to divide the entire outer surface of the cup body 730 into a plurality of separate connection regions (in this example, into three regions); a plurality of composite bodies (each of which may be a sheet or other shape adapted to the dome-shaped acetabular cup 700; intermediates 720 may each be provided), which are each in contact with and welded to the corresponding connection region on the cup body 730 . Wherein, each composite body has an exposed outer layer, all or most of which is a first porous structure 710.

### < fusion cage>

The fusion cage device is used as an example for illustration. In the presence of spinal injuries, such as degenerative disc disease with symptoms such as lumbar disc herniation and cervical spondylosis, in order to strengthen the spine between the two vertebrae involved in the injury, a fusion cage is typically placed between two directly overlapping vertebrae after removing the injured disc from the intervertebral cavity or disc space in order to maintain a predetermined distance between the vertebrae and to allow the fusion to eventually integrate into the intervertebral space and Improves intervertebral stability.

As shown in FIG. 148 or FIG. 149, in the example fusion cage device 8000, the fusion cage contains an upper end face, a lower end face (as in FIG. 148 and FIG. 149 corresponding to the inner and outer orientation of the paper face), which will contact each of the two vertebral bodies after implantation; a sidewall that connects the upper and lower end faces and is formed around the periphery; one or more through-holes 8010 running longitudinally through the upper and lower end faces are provided, which can be used for implanting autologous bone or bone substitute materials. The lateral wall can also have transversely penetrating holes (not shown) to connect to the surrounding bone tissue..

The fusion cage body 8300 acts primarily as a mechanical support; and in order to promote bone ingrowth and accelerate the bony fusion of the upper and lower vertebrae with the fusion cage, the first porous structure 8100 can preferably cover the upper, lower end faces of said fusion cage body 8300; or, further, at least part of the outer surface of the sidewall of the fusion cage body 8300, and/or at least part of the inner surface at the sidewall (around the longitudinal/transverse through-holes) are also covered with the first porous structure 8100. With the connected structure and method of the present invention, it is possible to achieve a larger coverage area of the porous structure 8100 on the surface of the fusion cage 8000 without affecting the structure or strength of the fusion cage body itself.

To this end, the structures and methods of Embodiments I to VIII above or variant examples thereof can be used similarly in said fusion cage device 8000. A partially enlarged side sectional view at box line E of Figure 149 being illustrated in Figure 141, with the fusion cage body 8300 corresponding to the substrate in the connected structure; a composite comprising an intermediate and a first porous structure 8100, covering the connection area (such as the aforementioned upper/lower end surfaces, etc.) of the fusion cage 8300. Through the connection of the composite body with the substrate, the coverage of the connection area on the fusion cage body 8300 by the porous structure is achieved, with the porous structure as the exposed surface of the fusion cage body 8300. The symbol 8400 in the figure schematically indicates that the components are welded to each other.

The fusion cage body 8300 mates with the composite (or its intermediate) at the site of contact and connection. Exemplarily, the intermediate may contain an interlayer and/or a support part, or may contain a bottom (or contain a bottom and a periphery), and may also use a freestanding or linked anchor structure; the connection area of the fusion cage body 8300 may be provided with a recessed portion in which the composite or a portion thereof is placed; or the fusion cage body 8300 may have no recessed portion, with the composite directly overlying the connection area of the fusion cage body 8300 The fusion cage body 8300 may be further provided with a positioning structure such as a bevel and/or a snap where a part of the fusion cage body 8300 is in contact with the intermediate.

The fusion cage body 8300 is made by forging, casting, powder metallurgy or machining, preferably as a solid structure, which is easy to machine and has high strength; or the fusion cage body 8300 can also be a porous structure with a high solid volume fraction; the intermediate can be a solid object or a porous structure with a higher solid volume fraction than the first porous structure 8100; When both the fusion cage body 8300 and the intermediate use porous structures, the solid volume fraction of the intermediate is between that of the fusion cage body 8300 and the first porous structure 8100. The intermediate and the first porous structure 8100 are manufactured, preferably using a 3D printing additive manufacturing process to effectively control the pores, etc., to meet the design requirements. The fusion cage body 8300 is effectively connected to the composite body containing the intermediate with the welding process, avoiding the current problem of significant reduction in overall strength by hot pressing processes (e.g., diffusion welding process), etc.

### < Artificial knee joint>

The artificial knee joint is used for illustration. The knee prosthetic implant consists of femoral condyle and tibial tray (both often metallic), an insert set between the two (which can be made of polyethylene etc), and the patellar component. The femoral condyle is connected to the distal femur and the tibial tray to the proximal tibia. The lower part of the insert is in contact with the upper surface of the tibial tray, and the convex surface of the femoral condyle is in contact with the upper part of the insert and the articular surface of the patellar component, allowing for flexion, extension, sliding, rotation and other activities within a defined range.

Referring to femoral condyle 900 shown in FIG. 150 or FIG. 151, femoral condyle body 930 is curved and substantially saddle-shaped, with the anterior and posterior ends upwardly curved, respectively; the superior frontal surface of femoral condyle 900 is a non-divided area with the middle, inferior and posterior end surfaces forming two halves on the left and the right, corresponding to the medial and lateral condyles, which are located on either side of intercondylar notch 906, respectively. In some examples, an upwardly extending restrictive flange (post) may be formed on the insert to mate within the intercondylar notch 906. In some examples, the middle of the femoral condylar body 930 may further form an upwardly extending post 907 that mates with the femoral condyle to improve the stability of the prosthetic implant.

The outer convex surface of the femoral condylar body 930 is usually smooth to reduce wear with the insert; while the inner concave surface of the femoral condylar body 930, preferably a porous structure, will match and contact with the cut surfaces of the distal femur, with host bone growing into the pores to achieve a strong bond between the implant and host bone, thereby improving the mechanical stability and biological compatibility of the implant.

Exemplarily, at the femoral condyles 900, in view of the presence of the condylar surface and the intercondylar notch 907, the inner concave surface of the femoral condylar body 930 can be divided into a plurality of regions, such as: the superior end region and anterior region, corresponding to regions 901, 902, respectively, where there is no left/right division; the central, posterior and posterior end regions 903, 904, 905,, each having left and right halves. Wiithin each of these regions, there provides the first porous structure 910. Regions 901-905 may be independent of each other or inter-connected; the first porous structures 910 located in these regions 901-905 may be independent of each other or inter-connected.

To this end, for the said femoral condyle 900, the structure and method of Embodiments I to VIII above or variant examples thereof can all apply. As shown in Figure 152, the femoral condyle body 930 corresponds to the substrate of the connected structure; including a composite containing the intermediate 920 and the first porous structure 910, which covers a plurality of connection regions on the inner concave surface of the femoral condyle body 930, by welding of the composite to the substrate. The first porous structure 910 covers the connection regions on the femoral condylar body 930 with a porous surface on the concave surface of the femoral condylar body 930.

Shown in FIGS. 153 and 154 is a tibial component 1000 of a knee prosthetic implant, comprising a tibial tray and a connection portion 1001, which forms a substantially T-shaped structure when connected; said upper surface of the tibial tray is in contact with the insert (which can be fixed or rotated relative to each other); said connection portion 1001 extends downward from the inferior surface of the tibial tray and is inserted into the tibia bone to achieve reliable fixation of the tibial tray. Wherein, the inferior surface of the tibial tray (e.g., in the areas surrounding the fixation structure) is provided with a porous structure to facilitate bone ingrowth for biological fixation for improved stability and longevity of the prosthetic implant.

To this end, for the said tibial tray 1000, the structure and method of Embodiments I to VIII above or variant examples thereof can be similarly used. In Figure 155, the tibial tray body 1030 corresponds to the substrate of the connected structure; a composite comprising an intermediate 1020 and a first porous structure 1010, covering a number of connection regions on the inferior surface of the tibial tray body 1030. Through the welding of the composite body to the substrate, the coverage of the first porous structure 1010 over the connection regions of the inferior surface of the tibial tray body 1030 is achieved, providing a porous surface of the tibial tray body 1030.

The femoral condylar body or tibial tray body, as described above, mates with the respective composite body (or its contained intermediate) at the site of contact and connection. Exemplarily, the intermediate may contain an interlayer and/or support part, or the intermediate may contain a bottom (or contain a bottom and periphery), and may use a freestanding or linked anchor structure; the connection area of the femoral condylar body/tibial tray body may be provided with a recess in which the composite body or a portion thereof is placed; or the femoral condylar body/tibial tray body may have no recess, with the composite body directly overlying the femoral condylar body/tibial tray body. The femoral condyle body/tibial tray body may be provided with a bevel and/or a positioning structure such as a snap at the location where the femoral condyle body/tibial tray body is in contact with the intermediate.

The femoral condylar body/tibial tray body is made by forging, casting, powder metallurgy or machining, preferably as a solid structure for ease of processing and with high strength, or a porous structure with high solid volume fraction; the intermediate may be a solid object or a porous structure with a higher solid volume fraction than the first porous structure; When both the femoral condylar body/tibial tray body and the intermediate use porous structures, the solid volume fraction of the intermediate is between that of the femoral condyle body/tibial tray body and that of the first porous structure. The intermediate and the first porous structure are preferably made by using 3D printing additive manufacturing process, which can effectively control the pores and curvatures, etc. to meet the design requirements. The femoral condyle body/tibial tray body and the intermediate are effectively connected by welding, avoiding the current problem of significant reduction in overall strength through hot pressing processes (e.g., diffusion welding process), etc. The patellar prosthetic implant can likewise use the structures and methods of Embodiments I through VIII above or variations thereof to add porous structures to its bone-contacting surface.

Based on the description of the present Embodiment, the structures and methods of Embodiments I to VIII, or variations thereof, can be widely used in any other types of orthopedic prostheses, including artificial joints and other prosthetic implants. Examples are spinal prostheses, ankle joints, shoulder joints, elbow joints, finger joints, toe joints, facet joints, temporo-madibular joints, wrist joints, and so on; the present invention allows the first porous structure, which is pre-connected or integrally formed to the intermediate, to cover the surface of the connection area on the prosthetic implant body by making contact with the intermediate and welding it to the connection area. (The basic structure and working principle of the various prosthetic bodies can be achieved by referring to the prior art in the field and will not be discussed here).

In addition, for any of the prostheses, after completion of the connection of the composite to the substrate, on the bone-contacting surface of the prosthetic implant, including but not limited to the first porous structure of the prosthetic implant, a coating or a carrier may be provided: an osteoconductive or osteoinductive coating (e.g., hydroxyapatite (HA)) formed by spraying or other means; a carrier containing cells/growth factors (e.g., using materials such as gel/collagen as carriers), or containing antimicrobial agents (e.g. antimicrobial agents/silver ions), etc.

Optionally, certain edges of the composite body described herein may form a seal to the edge of the intermediate, to the edge of the first porous structure, or to the edge at the junction of the two (not shown). Said edges may be solid or porous structures with a higher solid volume fraction than the first porous structure. Each edge may be a continuous section or a plurality of sections spaced apart at the edge where it is located.

Optionally, a number of skirt lines may be provided on the surface of said substrate facing the connecting composite; said skirt lines may be a porous structure with a higher solid volume fraction than the first porous structure or a solid structure. It is possible to set said skirt lines at some edges of the connection area on the substrate. Each skirt line may be a continuous section or a plurality of sections arranged at intervals at the edge of the connection area where it is located.

In the case of the prosthetic implant documented in the above Embodiment, the skirt line 650 on the femoral stem 600, for example, can be located at the upper edge and at the lower edge of the connection area of the stem body 630 (upper) (FIG. 135, FIG. 136). The skirt line 750 at acetabular cup 700, for example, is located at the edge of each separate connection area (Figures 139, 140; 142, 143). The skirt line 850 of the fusion cage 800, for example, can be located at the respective outer and inner edges of the superior/inferior end surfaces, the edges of the longitudinal/transverse through-holes, etc. (Fig. 149). The skirt line 950 of the femoral condyle 900, for example, is located at the edge of each connection area of the inner concave surface of the femoral condyle body 930 (Figure 150). For example, skirt line 1050 of tibial component 1000, located at the edge of each connection area on the inferior surface of the main body of the tibial tray 1030 (Figure 153).

In different examples, the skirt line may be a strip structure outlining the edge where it is located, the structure itself having a certain width and raised on the substrate surface. One side of the structure touches (or further connects) the opposite edge of the composite, the opposite other side may be openly arranged (not touching or connecting with other components), or the opposite other side may touch (or further connected). Alternatively, the side of the skirt line in contact with (or further connected to) the opposite edge of the composite may be similarly implemented as a recess side surface of the substrate, and the opposite side of the skirt line may extend as a portion of the substrate next to the recess opening without protruding from the surface of the substrate. On the side of the skirt line in contact with (or further connected to) the opposite edge of the composite, a positioning structure such as a bevel and/or a snap may be provided.

The present invention is not limited to the number and location of the welding points of the substrate and the composite body; for example, the welding points may be distributed in the non-edge area of the composite body. And when the substrate is provided with a skirt line, some of the welding points can preferably be distributed and set on the skirt line of the substrate and the corresponding edge of the composite body in contact with the skirt line; there can be no other welding points, or some other welding points can be arranged in the non-edge area of the composite body. The welding at the corresponding skirt line position is relatively easy to implement and the reliability of the connection is relatively high, and the design selection process for the location of the welding points is simplified and the efficiency is improved.

Although the contents of the present invention have been described in detail by the above preferred Embodiments, it should be recognized that the above description should not be considered a limitation of the present invention. A variety of modifications and alternatives to the present invention will be apparent to those skilled in the art after reading the foregoing. Accordingly, the scope of protection of the present invention shall be limited by the appended Claims.

## Claims

1. A connected structure of a porous structure and a substrate, **characterized in that** the said connected structure comprises: a composite, comprising of a first porous structure and an intermediate pre-connected or integrally formed with the said intermediate having a higher solid volume fraction solid volume fraction than the first porous structure; and a substrate connected to the first porous structure and/or the intermediate of the composite; and the said connected structure is provided with at least one holding space in which a drug and/or sensor is placed.

2. The connected structure as claimed in Claim 1, **characterized in that** said intermediate comprises an insertion portion and/or an interlayer; said insertion portion having at least a portion of a structure disposed within the first porous structure; said interlayer having at least a portion of a structure disposed between the first porous structure and the substrate.

3. The connected structure as claimed in Claim 2, **characterized in that** said intermediate is a solid structure, or a second porous structure; said second porous structure having a higher solid volume fraction than said first porous structure.

4. The connected structure as claimed in Claim 3, **characterized in that** said substrate is a solid structure, or a third porous structure; said third porous structure having a higher solid volume fraction than said first porous structure.

5. The connected structure as claimed in Claim 4, **characterized in that** the solid volume fraction of said second porous structure is between that of said first porous structure and that of said third porous structure.

6. The connected structure as claimed in Claim 4, **characterized in that** said substrate is made by forging or casting or machining or powder metallurgy or metal powder injection molding.

7. The connected structure as claimed in Claim 3, **characterized in that** said first porous structure of the composite, together with the intermediate, is integrally formed by a 3D printing additive manufacturing process, or a vapor phase precipitation process, or a sintering process.

8. The connected structure as claimed in Claim 3, **characterized in that** said substrate is metallic; said first porous structure is metallic; and said intermediate is metallic.

9. The connected structure as claimed in Claim 3, **characterized in that** the channel or opening of the holding space, is directly or indirectly connected to an open space outside said connected structure or to other holding spaces of the connected structure.

10. The connected structure as claimed in Claim 9, **characterized in that** the channel or opening of the holding spaceis connected to the external open space through channels or openings of single or multiple components comprising a first porous structure, an intermediate, a substrate, an additional substructure of the connected structure.

11. The connected structure as claimed in Claim 9, **characterized in that** the channel or opening of the holding space is connected to other holding spaces throughchannels or openings of single or multiple components comprising a first porous structure, an intermediate, a substrate, an additional substructure of the connected structure.

12. The connected structure as claimed in Claim 9, **characterized in that** said channel or opening of said holding space, directly or indirectly connected to an exposed surface of said connected structure, said exposed surface being exposed to an external open space; with one or more of the following components having said exposed surface; and said components comprising a first porous structure, an intermediate, a substrate, an additional component of the connected structure.

13. The connected structure as claimed in Claim 9, **characterized in that** one or more of the following components contain channels or openings serving as or connecting with those of the said holding spaces; the said components comprising: a first porous structure, an insertion portion, an intermediate, a substrate, an additional component of the connected structure.

14. The connected structure as claimed in Claim 9, **characterized in that** said channel or opening of said holding space is open, or closed and to be subsequently opened, or, closed and then no longer to be opened.

15. The connected structure as claimed in Claim 9, **characterized in that** the holding space for holding the drug, is the same or a different holding space as that for holding the sensors..

16. The connected structure as claimed in Claim 14, **characterized in that** the channels or openings of the holding space for placing the drug are provided for the delivery of the drug.

17. The connected structure as claimed in Claim 16, **characterized in that** when the holding space for the drug is provided with openings/channels, the said openings/channels comprise an input port/channel and an output port/channel for the drug; said input and output ports/channels being different openings/channels or the same opening/channel;

18. The connected structure as claimed in Claim 17, **characterized in that** the said output port of the drug is an open orifice for controlled release, or an orifice closed by a controlled-release plug; and the said input port of the drug is an injection orifice closed by a plug for injection, or an open injection orifice.

19. The connected structure as claimed in Claim 17, **characterized in that** an external detection device can be used to detect and locate the input channel or input port for the drug, or the channel or opening connected with the input channel or input port.

20. The connected structure as claimed in Claim 19, **characterized in that** the external detection device uses radiation or ultrasound for detection.

21. The connected structure as claimed in Claim 16, **characterized in that** the plug for the channel or port is made of any of the following materials; a material that can self-trigger to open up in response to a change in the surrounding environment; a material that can trigger to open up in reaction with a set substance; a material that can degrade over a set period; a material that can dissolve; a material that can allow drugs to pass through; a material that can slowly release drugs.

22. The connected structure as claimed in Claim 9, **characterized in that** the drug form when placed into the holding space and that when released from the holding space are the same, or different.

23. The connected structure as claimed in Claim 22, **characterized in that** the said drug is liquid, or solid object, or gas when placed into the holding space; and the said drug is liquid, or solid object, or gas when output from the holding space.

24. The connected structure as claimed in Claim 9, **characterized in that** said connected structure is subjected to a change in the state of the environment to which the drug is subjected, causing a transformation in the form of the drug.

25. The connected structure as claimed in Claim 9, **characterized in that** said drug reacts with the set substance to transform the form of the drug.

26. The connected structure as claimed in Claim 24 or 25, **characterized in that** the transformed drug is released from the holding space.

27. The connected structure as claimed in Claim 9, **characterized in that** said drug is encapsulated with any of the following materials; a material that can self-trigger to open up in response to a change in the surrounding environment; a material that can trigger to open up in reaction with a set substance; a material that can degrade over a set period; a material that can dissolve; a material that can allow drugs to pass through; a material that can slowly release drugs.

28. The connected structure as claimed in Claim 9, **characterized in that** said sensor detects at least one state inside or outside of the said connected structure; or, the said sensor detects a set substance.

29. The connected structure as claimed in Claim 28, **characterized in that** said sensor detects the temperature, or the pressure, or the humidity of the environment in which the connected structure is located.

30. The connected structure as claimed in Claim 28, **characterized in that** said sensor detects a set substance, including bacterial strains.

31. The connected structure as claimed in Claim 21 or 25 or 27 or 30, **characterized in that** the said set substance is purposefully placed into the connected structure; or, spontaneously formed within the surrounding environment of the connected structure.

32. The connected structure as claimed in Claim 31, **characterized in that** said set substance is input from an open space outside the connected structure or from other holding spaces of the connected structure, directly or indirectly, to the holding space for placing the drug and/or to the sensor holding space.

33. The connected structure as claimed in Claim 31, **characterized in that** said set substance is input through an exposed surface of the connected structure and thus into a holding space directly or indirectly connected to said exposed surface.

34. The connected structure as claimed in Claim 31, **characterized in that** said set substance is input into the holding space for placing drugs and/or sensors, via channels or ports provided by one or more of the following components, comprising a first porous structure, an insertion section, an intermediate structure, a substrate, or an additional sub-structure of the connected structure.

35. The connected structure as claimed in Claim 31, **characterized in that** said set substance is input into the holding space prior to, or subsequent to, or simultaneously with the input of the drug.

36. The connected structure as claimed in Claim 35, **characterized in that** said set substance is water, or another drug.

37. The connected structure as claimed in Claim 28, **characterized in that** the sensor holding space is provided with a channel or opening for placing the sensor into the holding space.

38. The connected structure as claimed in Claim 28, **characterized in that** the sensor holding space is provided with a channel or opening, the sensor being provided with an antenna transmitting information or electrical energy in a wireless manner, said antenna being within the holding space and facing said channel or opening, or extending into the outside of the holding space through said channel or opening.

39. The connected structure as claimed in Claim 28, **characterized in that** the sensor holding space is provided with a channel or opening, the sensor is provided with a probe for detection, said probe being within the holding space and facing said channel or opening, or extending to the outside of the holding space through said channel or opening.

40. The connected structure as claimed in Claim 28, **characterized in that** said sensor is provided with detection probes or detection surfaces, which are exposed to the outside of the connected structure or are facing channels or openings connected to the exposed surface of the connected structure.

41. The connected structure as claimed in Claim 28, **characterized in that** devices charging the sensors are provided in the sensor holding space or other holding spaces within the connected structure or provided outside of the connected structure; said charging devices supplying power to the sensors by wired or wireless means.

42. The connected structure as claimed in Claim 28, **characterized in that** said sensor transmits electrical energy and/or information with other devices via wires; said other connected devices being located in the sensor holding space or other holding spaces of the connected structure,, or outside of the connected structure.

43. The connected structure as claimed in Claim 42, **characterized in that** the channels or openings of the sensor holding space are used for wiring; and the channels or openings of the following components are used for wiring, which are connected to the sensor holding space; the said components including a first porous structure, an intermediate, a substrate, and additional components of the connected structures.

44. The connected structure as claimed in Claim 41, **characterized in that** the receiving coil of the wireless charging module cooperates with the external feeding coil to obtain electrical energy wirelessly, and the electrical energy is converted, processed and supplied to the sensors or other electrical components or electrical energy storage elements.

45. The connected structure as claimed in Claim 41, **characterized in that** the device for charging the sensors or other electrical components of the connected structure, including electrical energy storage elements; said electrical energy storage elements obtaining electrical energy from an external source by wired or wireless means; said electrical energy storage elements provide electrical power by wired or wireless means.

46. The connected structure as claimed in Claim 28, **characterized in that** the electromagnetic signal trigger switch receives a magnetically induced signal corresponding to an external command and converts it to a corresponding electrical signal to control the sensor or other controlled components of the connected structure; the said electromagnetic signal trigger switch being provided in the holding space where the sensors or other controlled devices are located or in another holding space where there is no sensor or other controlled device; said electromagnetic signal trigger switch providing the converted electrical signal to the sensors or other controlled devices by wired or wireless means.

47. The connected structure as claimed in Claim 28, **characterized in that** said sensor is paired with a processor for signal transmission and analyses, with the said processor being used to analyze the detection data; said processor and sensor are integrated or both are independent and achieve signal transmission by wireless or wired means; said processor is provided in the sensor holding space or other holding spaces or outside the connected structure .

48. The connected structure as claimed in Claim 28, **characterized in that** said connected structure is provided with a trigger device, which acts upon the closure bodies plug, or the drug capsule, or opens the drug input or output channel/port by applying a set substance, or by changing the surrounding environment of the connected structure; said trigger device is engaged upon receiving, by wired or wireless means, instructions from sensors, processor, electromagnetic signal trigger switch, external devices, timer, or timing devices.

49. The connected structure as claimed in Claim 1 or 48, **characterized in that** said drug is timed for controlled release at scheduled time points or time intervals.

50. The connected structure as claimed in Claim 48, **characterized in that** said triggering device is for single-use or multiple-uses.

51. The connected structure as claimed in Claim 28, **characterized in that** said sensor is driven to detect at set time schedule according to instructions from a timer or a timing device; said sensor performs detection at defined time points or time intervals.

52. The connected structure as claimed in Claim 3, **characterized in that** said holding space has no edge interface along external boundary of the connected structure; or, has edge interfaces in at least some directions.

53. The connected structure as claimed in Claim 49, **characterized in that** said edge interface of said holding space is either closed or not closed.

54. The connected structure as claimed in Claim 49, **characterized in that**, that said edge interface of the holding space is formed by a first porous structure or an intermediate or a part of the substrate adjacent to the holding space, or an additional sub-structure of the connected structure.

55. The connected structure as claimed in Claim 3, **characterized in that** the holding space contains one or more, or none of the following components: a first porous structure, or an intermediate, or a substrate, or an additional sub-structure of the connected structure.

56. The connected structure as claimed in Claim 3, **characterized in that** components on one or multiple sides around the holding space, individually or cooperatively, secure the solid object; the said components of the connected structure comprise of one or more of the following: a first porous structure, an intermediate, a substrate, an additional component of the connected structure; and said solid object comprises of one or more of the following: a solid object of the contained substance, an encapsulated object of the contained substance, an object formed by edge interfaces of the holding space.

57. The connected structure as claimed in Claim 53, **characterized in that** a solid object formed directly by the drug itself is directly secured with corresponding components of the connected structure; or, the encapsulated drug is placed into the holding space or secured by corresponding components of the connected structure; or, the solid object or non-solid object formed by the drug itself is placed within a holding space with solid object edge interfaces on at least one side.

58. The connected structure as claimed in Claim 53, **characterized in that** the sensors are directly secured by corresponding components of the connected structure; or, the sensors are enclosed in a solid object enclosure bodies, which is then placed into the holding space or secured by corresponding components of the connected structure; or, the sensors are placed in the holding space with a solid object edge interface on at least one side.

59. The connected structure as claimed in Claim 53, **characterized in that** securing the solid object includes loading the solid object: said components support the solid object from one or multiple directions, either individually or cooperatively; said components carry the load through structural parts adjacent to the holding space or those extended into the confinement of the holding space.

60. The connected structure as claimed in Claim 53, **characterized in that** securing the solid object includes a tight fit to the solid object: said components are in close contact with the solid object from at least two opposite directions; said components achieve tight fit through structural parts adjacent to the holding space or those extended into the confinement of the holding space; through structural parts adjacent to the holding space or those extended into the confinement of the holding space; and the tight fit to the solid object is achieved by different parts of the same component, or different components.

61. The connected structure as claimed in Claim 53, **characterized in that** securing the solid object includes position-limiting the solid object: said components individually or cooperatively form a position-limiting structure located in at least one direction of the solid object, preventing the solid object located on one side of the position-limiting structure from moving to the other side; said position-limiting structure includes position-limiting protrusions.

62. The connected structure as claimed in Claim 53, **characterized in that** securing the solid object includes position-limiting the solid object: said components individually or cooperatively form a position-limiting structure in close contact with the solid object from at least one direction; said position-limiting structure includes at least one position-limiting protrusion, formed at a location on the said component adjacent to the holding space and extending into the confinements of the holding space.

63. The connected structure as claimed in Claim 59, **characterized in that** the part of said solid object corresponding to the position-limiting protrusion is provided with a position-limiting recess matching the position-limiting protrusion so that said position-limiting protrusion can be set into the position-limiting recess; alternatively, the part of said solid object has no position-limiting recess corresponding to the position-limiting protrusion..

64. The connected structure as claimed in Claim 58 or 59, **characterized in that**, when the component position-limiting the solid object comprises a first porous structure, said position-limiting protrusion comprises the ends of a portion of the struts of the first porous structure; which are adjacent to the holding space and their ends may extend into the confinements of the holding space.

65. The connected structure as claimed in Claim 53, **characterized in that** securing the solid object comprises position-limiting solid object: said component individually or cooperatively forming a position-limiting structure in close contact with the solid object from at least one direction; said position-limiting structure comprising matching position-limiting protrusions and position-limiting recesss, said position-limiting recesss being formed at said parts adjacent to the holding space, said position-limiting protrusions being set on the surface of the solid object and extending into the confinements of the holding space, with the said position-limiting protrusions being set in the said position-limiting recesss.

66. The connected structure as claimed in Claim 62, **characterized in that**, when the component position-limiting the solid object comprises a first porous structure, said position-limiting recess comprises a portion of the pores of the first porous structure adjacent to the holding space; this portion of the pores retains the form it had when the first porous structure was made, or by machining this portion of the pores of the first porous structure to form said position-limiting recess.

67. The connected structure as claimed in Claim 9, **characterized in that**, after the composite is connected to the substrate, a part of the connected structure is removed to form at least a part of the holding space, or to form a channel or opening in the holding space; the removed part comprises one or more of the following: a part of the first porous structure, at least a part of the insertion, a part of the intermediate, a part of the substrate, at least a part of the additional components of the connected structure.

68. The connected structure as claimed in Claim 64, **characterized in that** the removed part is used as a closure bodies plug to subsequently close said channel or opening.

69. The connected structure as claimed in Claim 9, **characterized in that** the closure bodies that closes the channel or opening of the holding space is formed by the first porous structure or intermediate or part of the substrate adjacent to the holding space, or by an additional component of the connected structure.

70. The connected structure as claimed in Claim 9, **characterized in that** at least a portion of the intermediate, at a predetermined position of the connected structure, is adjacent to the holding space and serves as a channel or opening of the holding space; after the contained substance is placed into the holding space, that portion of the intermediate is mounted to the predetermined position to achieve closure bodies of the channel or opening.

71. The connected structure as claimed in Claim 9, **characterized in that** the additional components of the connected structure, includes a component which is used to form the holding space by acting as at least a part of the edge interface of the holding space or forms a closure bodies for closing the channel or opening of the holding space; said component being used to form the holding space by connecting with parts of the first porous structure or intermediate or substrate at locations adjacent to the holding space, or connected with other components of the connected structure.

72. The connected structure as claimed in Claim 9, **characterized in that** the additional component of the connected structure comprises a filling body which connects a portion of the struts of the first porous structure adjacent to the holding space to form a filling surface; said filling surface acting as at least a portion of the edge interface of the holding space, or forming a closure bodies for closing a channel or opening in the holding space; said filling surface being separately provided at the first porous structure or coupled to an intermediate or substrate or other components of the connected structure.

73. The connected structure as claimed in Claim 9, **characterized in that** a substance in molten form, after solidification at a designated part of the connected structure, serves as at least a portion of the edge interface of the holding space, or forms a closure bodies for closing a channel or opening in the holding space, or for coupling part a portion of the struts of the first porous structure; the substance is a polymeric material, or a material identical or similar in nature to the first porous structure or intermediate or substrate.

74. The connected structure as claimed in Claim 70, **characterized in that** the molten substance is injected into the designated part of the first porous structure and then waits for it to solidify; or, the raw material of the substance is placed in the designated position, at least the vicinity of the designated position is heated so that the raw material of the substance becomes molten and fills the pores of the first porous structure around the designated position.

75. The connected structure as claimed in Claim 9, **characterized in that** the closure bodies for closing the channel or opening is made of any of the following materials: a material identical to the first porous structure or intermediate or substrate; a material having properties similar to the first porous structure or intermediate or substrate.

76. The connected structure as claimed in Claim 72, **characterized in that** similar properties mean similar electrical conductivities, or similar solid volume fractions.

77. The connected structure as claimed in Claim 9, **characterized in that** the closure bodies for closing the channel or opening is a solid structure or a porous structure with the same or different solid volume fraction as the first porous structure.

78. The connected structure as claimed in Claim 3 or 9 or 53, **characterized in that** all or part of said holding space is formed within the first porous structure; at least part of the surface of said first porous structure is an exposed external surface of said connected structure.

79. The connected structure as claimed in Claim 3 or 9 or 53, **characterized in that** a portion of said holding space is formed at or within the first porous structure and other portions of the holding space are formed at or within one or more of the following components; said components comprising an intermediate, a substrate, an additional component of the connected structure.

80. The connected structure as claimed in Claim 3 or 9 or 53, **characterized in that** the recess formed at the first porous structure serves as at least a part of the holding space.

81. The connected structure as claimed in Claim 77, **characterized in that** said recess is formed on an exposed surface of the first porous structure.

82. The connected structure as claimed in Claim 3 or 9 or 53, **characterized in that** a specific pore or a plurality of interconnected pores within the first porous structure serves as at least a portion of the holding space.

83. The connected structure as claimed in Claim 3 or 9 or 53, **characterized in that** the first porous structure or intermediate or the substrate is formed with recessed or through structures at locations adjacent to the holding space such that a portion of the holding space extends into these structures.

84. The connected structure as claimed in Claim 9, **characterized in that** a portion of the poress of said first porous structure, serve as channels or openings for holding spaces, or are connected to channels or openings for holding spaces, or are connected to external open spaces, or are connected to channels or openings provided by one or more of the following components: an intermediate, a substrate, an additional component of the connected structure.

85. The connected structure as claimed in Claim 9, **characterized in that** said channel provided at the substrate, is connected to a channel or opening in the holding space, or to an external open space, or to a channel or opening provided by one or more of the following components: an intermediate, a substrate, an additional component of the connected structure; said channel provided at the substrate, being a duct formed inside the substrate; or, said channel provided at the substrate is a groove which is formed on the surface of the substrate near the side of the composite, and which is open at least partially in the direction in which the composite is located.

86. The connected structure as claimed in Claim 9, **characterized in that** the interlayer portion between the composite and the substrate, comprises raised structures and/or an intermediate body; said interlayer portion has the following positional relationship with the holding space (a1 and/or a2) :
a1, wherein there is no interlayer portion within the confinements of said holding space, or, wherein said confinements of said holding space contain raised structures and/or an intermediate body;
a2, wherein a part of the said interlayer is adjacent to the holding space and contains raised structures and/or an intermediate body; or, said interlayer is not adjacent to the holding space;
wherein the interlayer between the composite and the substrate contains raised structures without an intermediate body, said raised structures being provided on the side of the first porous structure proximal to the substrate and raised bumps facing toward the substrate;
said interlayer being in contact and connected with the substrate at said raised structures;
or, the interlayer between the composite and the substrate comprises an intermediate body without raised structures, said intermediate body being in contact with and connected to the substrate;
or, the interlayer between the composite and the substrate comprises an intermediate body and raised structures, said raised structure being provided on the side of said intermediate body proximal to the substrate and raised bumps facing towards the substrate; said interlayer being in contact with and connected with the substrate.

87. The connected structure as claimed in Claim 83, **characterized in that** when said part within or adjacent to the holding space contains raised structures without the intermediate body, said raised structures alone, or in conjunction with one or more of the following components located within or adjacent to the holding space to achieve fixation: a first porous structure, other raised structures, an insertion into the intermediate, an additional component of the connected structure, and a substrate.

88. The connected structure as claimed in Claim 84, **characterized in that**, when said part within the holding space contains raised structures without an intermediate body, said composite body further comprises an extension part, a part of which is connected to raised structures and extends into the holding space; another part of the extension part is connected to one or more of the following parts adjacent to the holding space or outside the holding space: a first porous structure, other raised structures, an insertion portion of an intermediate, an additional component of the connected structure, a substrate.

89. The connected structure as claimed in Claim 83, **characterized in that** the gaps between adjacent raised structures serve as channels or openings for holding spaces, or are connected to channels or openings for holding spaces, or are connected to external open spaces, or are connected to channels or openings provided by one or more of the following components: the first porous structure, other interlayers of the intermediate, the insertion portions of the intermediate, the substrate, the additional component of the connected structure.

90. The connected structure as claimed in Claim 83, **characterized in that** said gaps between the raised structures and the substrate serve as channels or openings for the holding space, or are connected to channels or openings for the holding space, or connected to an external open space, or connected to channels or openings provided by one or more of the following components: a first porous structure, other interlayer portions of the intermediate, an inserted portion of the intermediate, the substrate, the additional component of the connected structure.

91. The connected structure as claimed in Claim 83, **characterized in that** said intermediate body is in the form of a layer, or a sheet, or a plate.

92. The connected structure as claimed in Claim 83, **characterized in that** the part of said intermediate body located within the holding space, alone, or in conjunction with one or more of the following components to secure the solid object within the holding space: the first porous structure, other parts of the intermediate body, other interlayer parts other than the intermediate body, the insertion portion of the intermediate, an additional component of the connected structure, the substrate.

93. The connected structure as claimed in Claim 83, **characterized in that** the part of said intermediate body located within the holding space acts as at least a part of the edge interface of the holding space.

94. The connected structure as claimed in Claim 83, **characterized in that** the part of said intermediate body located within the holding space contains openings to secure the solid object set in the openings.

95. A connected structure as claimed in Claim 83, **characterized in that** said the part of said intermediate body located within the holding space contains openings, acting as channels or openings of the holding space, or are connected to channels or openings of the holding space, or to an external open space, or to channels or openings provided by one or more of the following components: a first porous structure, other parts of the intermediate body, the interlayer other than the intermediate body, the insertion portion of the intermediate, the additional component of the connected structure, the substrate.

96. The connected structure as claimed in Claim 83, **characterized in that** the part of said intermediate body located within the holding space contains openings and an extension part is provided on the inside of the openings; the solid object being secured by the extension part.

97. The connected structure as claimed in Claim 83 or 92, **characterized in that** said intermediate body is closed; or, said intermediate body is provided with channels or openings.

98. The connected structure as claimed in Claim 94, **characterized in that** said channels provided to the intermediate body are formed in the interior of the intermediate body; or, said channel provided to the intermediate body are grooves formed on the surface of the intermediate body near the side of the substrate, and at least a portion of the groove are facing towards the substrate.

99. The connected structure as claimed in Claim 83 or 92, **characterized in that** said intermediate body is provided with a first groove as a channel for the intermediate body, and/or, said substrate is provided with a second groove at said substrate as a channel for the substrate;
wherein the first groove is formed on a surface of the intermediate body near the side of the substrate, and at least a portion of the first groove facing towards the substrate; the second groove is formed on the surface of the substrate near the side of the composite body, and at least part of the second groove facing towards the composite body;
when the first groove and the second groove are provided at the same time, they are staggered, or they are interlocked to form a channel.

100. The connected structure as claimed in Claim 83, **characterized in that** when the composite is resistance welded to the substrate, the weld interface of the composite comprises a first porous structure and/or an intermediate body, without raised structures that projects toward the substrate; or, the weld interface of the composite comprises one or more of the first porous structure, the intermediate body, and raised structures that projects toward the substrate.

101. The connected structure as claimed in Claim 83, **characterized in that** when the composite body is resistance welded to the substrate, said weld interface of the substrate is provided with a substrate raised structure, which project towards the weld interface of the composite body; said substrate is connected to the weld interface of the composite body at least through the substrate raised structures; and, connected to said substrate raised structures, is a weld interface of the composite body on the first porous structure of the composite body, or an interlayer not containing raised structures, or a part of the interlayer at which no raised structure is provided.

102. The connected structure as claimed in Claim 9, **characterized in that** said connected structure comprises a support part, at least a portion of which is inserted into the first porous structure; a first end of said support part is proximal to the first side of the first porous structure and a second end of said support part is proximal to the second side of the first porous structure; the second side of the first porous structure is the side proximal to the welding interface of the composite, the first side being the side opposite to the second side;
said support part alone or in conjunction with one or more of the following components to form the holding space, or, said support part alone or in conjunction with one or more of the following components to secure the solid object; said components, comprising: other support parts, the first porous structure, the insertion portion of the intermediate, the interlayer of the intermediate, the substrate, the additional component of the connected structure.

103. The connected structure as claimed in Claim 99, **characterized in that** said support part passes through the solid object in order to secure it;
the part of the solid object that is not secured by the support part is secured by one or more of the following components, or is not in contact with said components; said components, comprising: other support parts, a first porous structure, an insertion of the intermediate, an interlayer of the intermediate, a substrate, additional components of the connected structure.

104. The connected structure as claimed in Claim 99, **characterized in that** said holding space is formed, in whole or in part, within the support part.

105. The connected structure as claimed in Claim 99, **characterized in that** all or part of the surfaces of the support part serve as at least part of the edge interface of the holding space.

106. The connected structure as claimed in Claim 99, **characterized in that** gaps or openings existing or created in the support part serve as at least part of the holding space; or,is used for securing the solid object; or, is used to place the parts to secure the solid object.

107. The connected structure as claimed in Claim 99, **characterized in that** the gaps between adjacent support parts, and/or the channels or openings placed at the support parts, serve as channels or openings of the holding spaces, or are connected to channels or openings of the holding spaces, or are connected to external open spaces, or are connected to channels or openings of one or more of the following components: a first porous structure, an interlayer of intermediates, the insertion portion of the intermediate, the substrate, and additional component of the connected structure.

108. The connected structure as claimed in Claim 99, **characterized in that**, after the composite body has been connected with the substrate, the gaps formed subsequent to the removal of all or parts of the support parts becomes at least a part of the holding space.

109. The connected structure as claimed in Claim 53, **characterized in that** the solid object is provided with an insertion structure, which is inserted to the gaps of the said porous structure to secure the fixation of the solid object.

110. The connected structure as claimed in Claim 99, **characterized in that** the solid object is provided with an insertion structure, said insertion structure being inserted to a gap formed within the first porous structure at a location corresponding to the insertion site of the support part; the gap is formed by removing all or part of the support part after the composite is connected to the substrate; alternatively, the gap is located between the support part and the first porous structure surrounding the insertion site thereof.

111. The connected structure as claimed in Claim 99, **characterized in that** the solid object is secured at the support part by means of a hoop structure provided.

112. The connected structure as claimed in Claim 107, **characterized in that** the solid object is provided with an integrated insertion and hoop structure; the insertion structure of the solid object is inserted at a gap formed within the first porous structure corresponding to the insertion site of the support part, and the hoop structure is fixed to the support part within the said gap.

113. The connected structure as claimed in Claim 108, **characterized in that** said hoop structure is a closed-hoop structure over the support part; alternatively, said hoop structure is an open-hoop structure in close contact with the support part.

114. The connected structure as claimed in Claim 99, **characterized in that** the solid object is provided with an insertion structure, said insertion structure being inserted at a gap set in the support part; or, the solid object is provided with an insertion structure, said insertion structure being inserted at a recess set in the support part.

115. The connected structure as claimed in Claim 99, **characterized in that** a recess is formed at said support part, at which the electrode segment is inserted for connecting the composite body with the substrate; after the composite body is connected to the substrate, the insertion structure of the solid object is inserted into said recess; or, said recess is used as at least part of the holding space.

116. The connected structure as claimed in Claim 107 or 109 or 111 or 112, **characterized in that** the position into which the insertion structure of the solid object is inserted corresponds to the first end of the insertion site of the support part; the solid object is in contact or not in contact with the surface of the first side of the first porous structure;
or, the position into which the insertion structure of the solid object is inserted corresponds to locations other than the first end of the insertion site of the support part, with such locations being adjacent to the holding space; wherein, when the insertion structure is inserted, the first end or other parts of the support part are still present at the insertion area , or have been removed.

117. The connected structure as claimed in Claim 108 or 109, **characterized in that** the position at which the hoop structure of the solid object is fixed corresponds to the first end of the support part insertion site; the solid object is in contact or not in contact with the first side surface of the first porous structure; or, the position at which the hoop structure is fixed corresponds to a part of the support part insertion site other than the first end, which is adjacent to the holding space.

118. A connected structure as claimed in Claim 99, **characterized in that** the gap corresponding to the insertion site of the support part within the first porous structure, serves as a holding space, or as a channel or opening, or is used to provide other components for securing the solid object;
wherein the gap corresponding to the insertion site, is formed before the composite is connected to the substrate, said support part being within said gap;
or a gap corresponding to the insertion site, formed after the composite body is connected to the substrate, said support part being within said gap, or not located within said gap.

119. The connected structure as claimed in Claim 115, **characterized in that**, prior to the connection of the composite body to the substrate, the support part is in direct contact with the first porous structure around its insertion site; after connecting the composite body with the substrate, all or parts of the support part are removed to form a gap corresponding to the insertion site.

120. The connected structure as claimed in Claim 115, **characterized in that**, prior to the connection of the composite body to the substrate, the support part is not in direct contact with the first porous structure around its insertion site, and, said support part being inserted at the first gap formed within the first porous structure; the support part is separated from the surrounding first porous structure by the said first gap; after the connecting the composite body with the substrate, the space created by removing all or part of the support part is linked to the first gap, forming a gap corresponding to the insertion site.

121. The connected structure as claimed in Claim 115, **characterized in that**, prior to the
connection of the composite body to the substrate, the support part is not in direct contact with the first porous structure around its insertion site, and, said support part being inserted at the first gap formed within the first porous structure; the support part is separated from the surrounding first porous structure by the said first gap; after the connecting the composite body with the substrate, keep the support part, and use the said first gap as the gap corresponding to the insertion site.

122. The connected structure as claimed in Claim 117 or 118, **characterized in that**, at the time of connection of the composite body to the substrate, an insulator is provided in the interval of the first gap, separating the support part from the surrounding first porous structure; after connection of the composite body to the substrate, the insulator is removed in whole or in part to form a gap corresponding to the insertion site, or to form an interval connected to said gap.

123. The connected structure as claimed in Claim 116 or 117 or 118, **characterized in that**, after the composite body is connected to the substrate, the first porous structure around the insertion site remains the same as before the connection of the composite body to the substrate, or is partially removed to form a gap corresponding to the insertion site, or is partially removed to form a space linked to said gap.

124. The connected structure as claimed in any one of Claims 115 to 118, **characterized in that**, after the composite body is connected to the substrate, the first end of the support part does not extend beyond the first side surface of the first porous structure, the gap corresponding to the insertion site comprises the gap located between the first end of the support part and the first side surface of the first porous structure;
after the composite body is connected to the substrate, the first end of the support part extends beyond or is flush with the first side surface of the first porous structure, then the gap corresponding to the insertion site comprises the gap between the insertion site of the support part and the surrounding first porous structure.

125. The connected structure as claimed in any one of Claims 115 to 118, **characterized in that** the component provided for securing the solid object at the gap corresponding to the insertion site comprises an insertion structure and/or a hoop structure of the solid object; said insertion structure is inserted in the gap and said hoop structure is secured to the support part.

126. The connected structure as claimed in Claim 122, **characterized in that**, after the composite body is connected to the substrate, the first end of the support part extends beyond the first side surface of the first porous structure as an extended portion of the support part; the solid object insertion structure and/or the hoop structure are provided at the extended portion of the support part.

127. The connected structure as claimed in Claim 99, **characterized in that**, the second end of the support part does not extend beyond or is flush with the second side surface of the first porous structure; when the second end of the support part is flush with said second side surface, the intermediate contains an interlayer portion that is or is not in contact with the second end of the support part; When the second end of the support part is flush with said second side surface and the intermediate does not contain an interlayer portion, the second end of the support part is or is not connected to the substrate.

128. The connected structure as claimed in Claim 124, **characterized in that** the support part is in contact with the side of the interlayer's intermediate body distal to the substrate, the side of the intermediate body proximal to the substrate being provided with or without raised structures; or, the support part is in contact with the side of the interlayer's raised structures distal to the substrate, said raised structures being provided on the second side surface of the first porous structure, facing towards the substrate.

129. The connected structure as claimed in Claim 124, **characterized in that** the support part is in direct contact with the raised structures of the interlaye arranged in corresponding locations; or, alternatively, the support part is staggered with the raised structures of the interlayer and is not in direct contact with each other;
wherein said raised structures project towards the substrate and are provided on the second side surface of the first porous structure or on the side of the interlayer's intermediate body, facing the substrate.

130. The connected structure as claimed in Claim 99, **characterized in that** said support part is made of an insulating material; alternatively, said support part is made of a conductive material and is the insertion portion of the intermediate.

131. The connected structure as claimed in Claim 99, **characterized in that** said support part is made of a molten substance solidified after injection into the first porous structure; the substance being a polymeric material or a material of the same or similar nature as the first porous structure or intermediate or substrate.

132. The connected structure as claimed in Claim 99, **characterized in that** said support part is a solid structure; or, said support part is a porous structure with its solid volume fraction higher than that of the first porous structure.

133. A connected structure as claimed in Claim 99, **characterized in that** said support part is provided with a coupling part and/or an extension part, and serves as at least part of an edge interface, or as a closure bodies to close the channel or opening of the holding space, or for securing the solid object, individually or in conjunction with one or more of the following components: said components, comprising: a support part connected to the coupling part or extension part, other support parts, a first porous structure, an insertion portion of an intermediate, an interlayer of an intermediate, a substrate, other coupling part or extension parts, additional components of the connected structure; wherein two ends of the said coupling part are respectively connected to two support parts;
one end of said extension part is connected to the support part and the other end extends into the open space, or into the first porous structure, or the insertion portion of an intermediate, or the interlayer of the intermediate, or other parts provided to the component of the coupling part structure adjacent to the holding space.

134. The connected structure as claimed in Claim 130, **characterized in that** a coupling part connected to a pair of support parts at sites proximal to the first end of each of the paired support parts; openings are provided between the said pair of support parts at sites proximal to the second end of each of the support parts; a space is formed between said coupling part and the substrate surface exposed by the openings, and becomes at least a part of the holding space.

135. The connected structure as claimed in Claim 130, **characterized in that** a coupling part connected to a pair of support parts at sites proximal to the first end of each of the paired support parts; openings are provided between the said pair of support parts at sites proximal to the second end of each of the support parts; where extension parts connected to the support parts are provided; a space is formed between said coupling part and the extension parts and becomes at least a part of the holding space.

136. The connected structure as claimed in Claim 130, **characterized in that** a first coupling part connected to a pair of support parts at sites proximal to the first end of each of the paired support parts; a second coupling part connected to the same pair of support parts at sites proximal to the second end of each of the support parts; a space is formed between said first and second coupling parts and becomes at least a part of the holding space.

137. The connected structure as claimed in any one of Claims 131 to 133, **characterized in that** said space is connected to an opening in a channel or pipeline located on the surface of the substrate; said channel or pipeline is used for placing wires to connect sensors or for delivering drugs.

138. The connected structure as claimed in Claim 134, **characterized in that** the portion of the said channel on the surface of the substrate not connected with said space is covered by the intermediate body of the interlayer which is connected to the substrate.

139. The connected structure as claimed in Claim 130, **characterized in that**, the drug holding space is provided with a drug output channel or port in the first or second coupling part and a drug input channel or port in the first or second coupling part.

140. The connected structure as claimed in any one of Claims 131 to 133, **characterized in that**, the sensor holding space is provided with openings for the sensor tips and/or antenna to pass through the coupling part close to the first ends of the support parts.

141. The connected structure as claimed in Claim 130, **characterized in that** said coupling part or extension part bodies are secured by the support parts alone, or by the support parts in cooperation with one or more of the following components: the first porous structure, the insertion portion of the intermediate, the interlayer of the intermediate, the substrate, the additional component of the connected structure; the components that cooperate in securing the extension part are located at one end of the extension part or at locations of the extension part other than its ends; the components that cooperate in securing the coupling part are located at locations of the coupling part other than its ends.

142. The connected structure as claimed in Claim 130, **characterized in that** the two support parts connected to the ends of the said coupling part are located at the same edge interface of the holding space or at different edge interfaces adjacent or opposite to each other.

143. The connected structure as claimed in Claim 130, **characterized in that** said coupling part or extension part is made together with the support parts or subsequently provided for connection to the support parts.

144. The connected structure as claimed in Claim 130, **characterized in that** said gap or notch or recess at the support part is provided for connection with the coupling part or extension part.

145. The connected structure as claimed in Claim 130, **characterized in that** said coupling part or extension part is a formed part or filling surface provided to the support part; said filling surface comprises a filling body which fills up the pores of the struts of the first porous structure at locations adjacent to the support parts and the holding space; said filling body is formed after solidification of a molten substance at a designated site of the connected structure; the filling body substance is a polymeric material or a material identical or similar in nature to the first porous structure or intermediate or substrate.

146. The connected structure as claimed in Claim 130, **characterized in that** said coupling part or extension part is made of an electrical-conductive material or an electrical-insulating material.

147. The connected structure as claimed in Claim 130, **characterized in that** said coupling part or extension part is a solid structure or a porous structure having the same or different solid volume fraction as that of the first porous structure.

148. A connected structure as claimed in Claim 9, **characterized in that** said composite body is provided with anchor points; each anchor point comprises an anchor point body disposed on the second side of the first porous structure proximal to the substrate; said second side of said anchor point body is in contact with and connected to the substrate; a space formed above the first side of the anchor point body is used as a holding space, or for inserting the insertion structure of a solid object to secure the solid object, or as a channel or opening of the holding space, or connected to a channel or opening of the holding space, or connected to an external open space, or connected to a channel or opening provided by one or more of the following components: a first porous structure, a substrate, an additional component of the connected structure.

149. The connected structure as claimed in Claim 145, **characterized in that** said anchor point further comprises an anchor point periphery, with at least one anchor point periphery provided on the first side of each anchor point body, or, alternatively, with encircled anchor point periphery provided on the first side of the anchor point body; the anchor point periphery is at least partially inserted within the first porous structure, with the first end of the anchor point periphery proximal to the first side of the first porous structure and the second end of the anchor point periphery proximal to the second side of the first porous structure; wherein the anchor point periphery serves as at least a portion of the edge interface; or, the anchor point periphery is provided with a channel or opening for insertion of the insertion structure of the solid object to hold the solid object, or as a channel or opening for holding the space, or is connected to a channel or opening for holding the space, or is connected to an external open space, or is connected to a channel or opening provided by one or more of the following components: the first porous structure, an intermediate, a substrate, an additional component of the connected structure.

150. The connected structure as claimed in Claim 146, **characterized in that** said space enclosed by the peripherys of the encircled anchor points corresponds to the space on the first side of the anchor point body.

151. The connected structure as claimed in Claim 145 or 146, **characterized in that** said anchor point is connected to at least one other anchor point, by a coupling part; said coupling part comprises a coupling part body, connected to the anchor point body; said coupling part body is located on a second side of the first porous structure proximal to the substrate; said second side of said anchor point body is or is not in contact with the substrate; or, said coupling part comprises said coupling part body, further comprising at least one coupling part sidewall; said coupling part sidewall being at least partially inserted within the first porous structure, the first and second end of the coupling part sidewall being near respectively the first and second side of the first porous structure; said anchor point connected to the coupling part having no anchor point periphery, or said anchor point connected to the coupling part having an anchor point periphery connected to the coupling part sidewall; said coupling part serves as at least part of the edge interface, or, said coupling part is provided with a channel or opening, or for insertion of an insertion structure of the solid object to secure the solid object, or as a channel or opening of the holding space, or is connected to a channel or opening of the holding space, or is connected to an external open space, or is connected to a channel or opening provided by one or more of the following components: a first porous structure, an intermediate, a substrate, an additional component of the connected structure.

152. The connected structure as claimed in Claim 148, **characterized in that** said anchor point is a solid structure; or, said anchor point is a porous structure with higher solid volume fraction than that of said first porous structure; said coupling part is a solid structure; or, said anchor point is a porous structure with higher solid volume fraction than that of said first porous structure; the solid volume fraction of said coupling part is or is not consistent with that of said anchor point.

153. The connected structure as claimed in Claim 148, **characterized in that** said anchor point body belongs to the interlayer of said intermediate; said anchor point periphery belongs to the support part; said coupling part body belongs to the interlayer of said intermediate and said coupling part sidewall belongs to the support part.

154. The connected structure as claimed in Claim 9, **characterized in that** there is a pair of contact surfaces between the composite body and the substrate, and they are connected; or, there is a plurality of pairs of contact surfaces between the composite body and the substrate, and at least one pair of contact surfaces are connected to each other; the holding space is provided at locations on the composite body away from the contact surfaces, or the holding space extends to the contact surfaces of the composite body, or the holding space extends through the contact surface of the composite body to the substrate.

155. The connected structure as claimed in Claim 151, **characterized in that** the contact surface of the substrate comprises a contact area that contacts and connects to one or a plurality of composite bodies, respectively; or that the contact surface of the substrate comprises a plurality of contact areas that contact and connect to the same composite body; or that the contact surface of the substrate comprises a plurality of contact areas, each of which contacts and connects to one or a plurality of corresponding composite bodies, respectively; where there is a plurality of composite bodies, the holding spaces of adjacent composite bodies are independent from or connected to each other.

156. The connected structure as claimed in Claim 152, **characterized in that** one contact area of said substrate, corresponding to a surface at that substrate in one same direction or in different directions; and a plurality of contact areas of said substrate, corresponding to a surface at that substrate in one same direction or in different directions.

157. The connected structure as claimed in Claim 151, **characterized in that** at least one pair of contact surfaces between the composite and the substrate form matching position-limiting openings and position-limiting protrusions; the position-limiting openings being formed on one of the paired contact surfaces, and the position-limiting protrusions being formed on the other paired contact surface while insertable into the position-limiting openings; the contact surface having the position-limiting openings and that having the position-limiting protrusions form an interconnected pair of contact surfaces, or a pair of non-connected contact surfaces; wherein the holding space or the channel or opening of the holding space is placed at locations away from the contact surfaces with position-limiting opening and protrusion;
or, the holding space or the channel or opening of the holding space is placed on the contact surfaces with position-limiting opening and protrusion at locations avoiding these opening and protrusion; or the holding space or the channel or opening of the holding space is placed at locations of the position-limiting opening and/or position-limiting protrusion or such locations after further modifications, once the composite body has been connected to the substrate,

158. The connected structure as claimed in Claim 151, **characterized in that** at least one pair of contact surfaces of the composite and the substrate form matching surfaces thereby achieving positioning; this pair of contact surfaces are or are not connected to each other; wherein the holding space, or the channel or opening of the holding space, is provided away from or at the positioning contact surfaces.

159. The connected structure as claimed in Claim 151, **characterized in that** said substrate is provided with a recess, said composite body comprising a part inserted into the recess, between which and the recess of the substrate there are a pair of contact surfaces, where the composite and the substrate are connected; or, between the composite body insertion part and the recess of the substrate, there are multiple pairs of contact surfaces, where the composite and the substrate are connected at least at one pair of the contact surfaces.

160. The connected structure as claimed in Claim 156, **characterized in that** the part of the composite body at which it is inserted into the recess of the substrate comprises an interlayer portion of the intermediate; said recess of the substrate comprises an opening, a first surface opposite the opening, and a side surface disposed between the opening and the first surface; said interlayer portion of the composite body comprises a second surface in contact with the first surface of the recess, said side surface of the recess being in contact or not in contact with a first porous structure along the corresponding direction of the composite body; or, said interlayer comprises said second surface, and a third surface in contact with the side surfaces of the recess; wherein the second surface and/or the third surface of the interlayer is adjacent to a part of the holding space as at least a portion of the edge interface of the holding space; or, the second surface of the interlayer and/or the third surface adjacent to the holding space is removed such that the holding space extends to the first surface and/or side surfaces of the recess of the substrate; or, the second surface of the interlayer and/or the third surface adjacent to
the holding space is used to secure the solid object, either alone or in conjunction with one or more of the following components; or, the second surface of the interlayer and/or the third surface adjacent to the holding space is provided with a channel or opening to be used as the channel or opening of the holding space, or the channel or opening connected to the holding space, or the channel or opening connected to an external open space, or the channel or opening connected to one or more of the following components; said components, comprising: a recess of the substrate, a first porous structure, other parts of the interlayer, other intermediates, additional components of the connected structure.

161. The connected structure as claimed in Claim 157, **characterized in that** at least one of the sides of said recess of the substrate is beveled, which forms a set angle with the first surface; a third surface in contact with this side is beveled, which forms a set angle with the second surface.

162. The connected structure as claimed in Claim 158, **characterized in that** said angle is acute.

163. The connected structure as claimed in Claim 3 or 9 or 53 or 83 or 99 or 145 or 151 or 156, **characterized in that** the composite body is connected to the substrate by laser welding or resistance welding; said holding space is formed before, or during, or after connecting the composite body to the substrate.

164. The connected structure as claimed in Claim 160, **characterized in that** the interlayer is welded to the substrate by laser welding, and after completion of the welding, the space formed in the first porous structure is used as a holding space, or for insertion of the insertion structure of the solid object to hold the solid object, or as a channel or opening of the holding space, or is connected to a channel or opening in the holding space, or is connected to an external open space, or is connected to a channel or opening provided by one or more of the following components: a first porous structure, an intermediate, a substrate, an additional component of the connected structure.

165. The connected structure as claimed in Claim 4 or 9 or, **characterized in that** said connected structure comprises an intermediate layer of polymeric material; said holding space is provided with or without using a polymeric material layer;
The polymeric material layer is formed at chosen locations when a polymeric material is injected into any one of the porous structures of the connected structure, or between any one of the porous structures and the substrate, or between two adjacent porous structures, with the molten polymer material penetrating into the connected part.

166. The connected structure as claimed in Claim 162, **characterized in that** at least part of the surface of said intermediate layer of polymeric material is used as at least part of the edge interface of the holding space; or, all or part of the holding space is formed within the intermediate layer of polymeric material; or, at least part of the surface of said layer of polymeric material is used alone or in conjunction with one or more of the following components, for securing the solid object; a first porous structure, a substrate, a support part, an interlayer of the intermediate, an additional component of the connected structure.

167. The connected structure as claimed in Claim 162, **characterized in that** said holding space is provided in a component within the connected structure that does not have a polymeric material interlayer; or, the holding space is provided in an area away from the polymeric material interlayer within a component having a polymeric material interlayer.

168. The connected structure as claimed in Claim 162, **characterized in that** the intermediate layer of polymeric material is formed within the first porous structure and/or formed within the second porous structure as an interlayer.

169. The connected structure as claimed in Claim 162, **characterized in that** a polymeric material interlayer is formed between the first porous structure and the substrate; or, a polymeric material interlayer is formed between the second porous structure as the interlayer and the substrate.

170. The connected structure as claimed in Claim 162, **characterized in that** an intermediate layer of polymeric material is formed between the first side of the first porous structure and a fourth porous structure; the fourth porous structure is arranged separately and forms another composite body with pre-connected or integrally formed fifth porous structure, the fourth and fifth porous structures having different solid volume fraction; the first side of the first porous structure being its side distal from the substrate.

171. The connected structure as claimed in Claim 162, **characterized in that** a barrier layer is provided within the porous structure where the intermediate layer of polymeric material is located or adjacent thereto, and this barrier is provided for position-limiting the location where the polymeric material is injected and it penetrates; said barrier layer is integrally formed with the porous structure in which it is located or is separately provided to that porous structure.

172. The connected structure as claimed in Claim 168, **characterized in that** said barrier layer serves as at least part of the edge interface of the holding space, or is used to secure the solid object, either alone or in conjunction with one or more of the following components; a first porous structure, a substrate, a support part, an interlayer of the intermediate, an additional component of the connected structure, a polymeric material layer.

173. The connected structure as claimed in Claim 168, **characterized in that** said barrier layer is a solid structure or a porous structure, its solid volume fraction being higher than that of the porous structure into which the polymeric material is injected.

174. The connected structure as claimed in Claim 168, **characterized in that** said polymeric material layer or barrier layer is provided with channels or openings, as channels or openings of holding spaces, or channels or openings connecting to holding spaces, or channels or openings connecting to external open spaces, or channels or openings provided by one or more of the following components; said components, comprising: a first porous structure, a support part, an intermediate interlayer, a substrate, an additional component of the connected structure.

175. The connected structure as claimed in Claim 168, **characterized in that** said polymeric material layer or barrier layer acts as a closure bodies for the channel or opening.

176. a prosthetic implant, **characterized in that** said prosthetic implant is provided with the connected structure of any one of Claims 1 to 172; said prosthetic implant, comprising: a composite, comprising a first porous structure pre-connected or integrally formed with an intermediate; said intermediate having a higher solid volume fraction than the first porous structure; a prosthetic body, serving as a substrate; at least part of the surface of said prosthetic body is provided as a connection area for connecting to the first porous structure and/or intermediate of the composite; wherein said connected structure is provided with at least one holding space for placing the drug and/or sensor.

177. The prosthetic implant as claimed in Claim 173, **characterized in that** said prosthetic implant is an orthopedic prosthetic implant.

178. The prosthetic implant as claimed in Claim 174, **characterized in that** said prosthetic implant is an joint prosthesis.

179. The prosthetic implant as claimed in Claim 173, **characterized in that** said composite body is formed as a shell, wrapped around connection regions of said prosthetic body, in contact with and tapercted to the connection regions. The surfaces of the body of the prosthetic implant on which the connection regions are located correspond to the same orientation of the body of the prosthetic implant or different orientations.

180. A prosthetic implant as claimed in Claim 176, **characterized in that** said composite shell is a single unit; or, said composite shell comprises a plurality of shell pieces; wherein the plurality of shell pieces are independent of each other or are connected to each other along at least one adjacent side between the adjacent shell pieces.

181. A prosthetic implant as claimed in Claim 173, **characterized in that** said prosthetic implant is any of the following: femoral stem, acetabular cup, fusion cage, femoral condyle, tibial tray, spinal prosthetic implant, ankle joint, shoulder joint, elbow joint, finger joint, toe joint, facet joint, mandibular joint, wrist joint, tooth implant.

182. The prosthetic implant as claimed in Claim 173, **characterized in that** said prosthetic implant is a femoral stem of the hip joint, said stem body of said femoral stem serving as a substrate; at least part of the surface of the proximal end of said stem body being provided with connection regions.

183. The prosthetic implant as claimed in Claim 173, **characterized in that** said prosthetic implant is an acetabular cup of the hip joint, said cup body serving as a substrate; at least part of the outer surface of the cup body is provided with connection regions.

184. The prosthetic implant as claimed in Claim 173, **characterized in that** said prosthetic implant is a fusion cage, the fusion cage of said fusion cage serving as a substrate; said connection regions are located at least on the upper and lower surfaces of said fusion cage.

185. a prosthetic implant as claimed in Claim 173, **characterized in that** said prosthetic implant is a femoral condyle, the body of the femoral condyle serving as a substrate; said femoral condyle body having connection regions on at least a portion of its inner recess surface.

186. a prosthetic implant as claimed in Claim 173, **characterized in that** said prosthetic implant is a tibial tray, the body of the tibial tray serving as a substrate, said connection regions being located on the inferior surface of the tibial tray.

187. The prosthetic implant as claimed in Claim 173, **characterized in that** said porous surface structure is further added on its surface with any one or more of the following: an osteoconductive coating, an osteoinductive coating, an antimicrobial coating, a carrier of cells or growth factors.

188. The prosthetic implant as claimed in Claim 173, **characterized in that** the open space outside the connected structure includes the body structure where the prosthetic implant is implanted.

189. The prosthetic implant as claimed in Claim 173, **characterized in that** the drug is input to the holding space before, or during, or after implantation of the prosthetic implant.

190. The prosthetic implant as claimed in Claim 173, **characterized in that** the set substance is input to the holding space before, or during, or after implantation of the prosthetic implant, wherein the set substance reacts with the closure bodies which closes the channel or opening of the holding space, thereby opening up the closure bodies; or, the set substance reacts with a drug in the holding space, causing a change in the form of the drug; or, the set substance reacts with the capsule of the drug, thereby opening up the capsule.

191. A prosthetic implant as claimed in Claim 173, **characterized in that** said sensor is connected to one end of a wire and the other end of the wire is connected to an interface located on the body surface.

192. A method of using the connected structure of a porous structure and a substrate, **characterized in that** the connected structure as claimed in any one of Claims 1 to 172, comprising: a composite comprising a pre-connected or integrally formed first porous structure with an intermediate, said intermediate having a higher solid volume fraction than that of the first porous structure; a substrate, which is connected to the first porous structure and/or intermediate of the composite; said connected structure provided with at least one holding space for placing drugs; said connected structure provided with sensors for detecting at least one state inside or outside the connected structure, or for detecting a set substance; wherein said set substance is manually input from external sources to the connected structure or is self-generated within the internal environment of the connected structure; the result of the sensor detection being used to determine whether to open the input or output channel or port of the drug holding space.
